(19) Europäisches Patentamt
European Patent Office
Office européen des brevets

(11) EP 2 248 818 B1

(12) EUROPEAN PATENT SPECIFICATION

(45) Date of publication and mention of the grant of the patent:
**24.09.2014 Bulletin 2014/39**

(21) Application number: **09708663.1**

(22) Date of filing: **03.02.2009**

(51) Int Cl.:
***C07D 495/04*** (2006.01) ***C07D 333/24*** (2006.01)
***H01L 29/786*** (2006.01) ***H01L 51/05*** (2006.01)
***H01L 51/30*** (2006.01) ***H01L 51/50*** (2006.01)

(86) International application number:
**PCT/JP2009/051801**

(87) International publication number:
**WO 2009/099070 (13.08.2009 Gazette 2009/33)**

# (54) ORGANIC SEMICONDUCTOR MATERIAL

ORGANISCHES HALBLEITERMATERIAL

MATÉRIAU SEMI-CONDUCTEUR ORGANIQUE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **06.02.2008 JP 2008026152**

(43) Date of publication of application:
**10.11.2010 Bulletin 2010/45**

(73) Proprietor: **Sumitomo Chemical Company, Limited**
**Tokyo 104-8260 (JP)**

(72) Inventors:
- **MIYATA, Yasuo**
  **Osaka 561-0802 (JP)**
- **HANAOKA, Hidenori**
  **Suita-shi**
  **Osaka 565-0874 (JP)**

(74) Representative: **Duckett, Anthony Joseph et al**
**Mathys & Squire LLP**
**The Shard**
**32 London Bridge Street**
**London SE1 9SG (GB)**

(56) References cited:
**JP-A- 2008 255 097**

- **WONG,K. ET AL.: 'Syntheses and structures of novel heteroarene-fused coplanar n-conjugated chromophores' ORGANIC LETTERS vol. 8, no. 22, 2006, pages 5033 - 5036**
- **ZHAO,C. ET AL.: 'Derivatives of 4,9-Dihydro-s-indaceno[1,2-b:5,6-b']dithiophene-4,9-dione : Synthesis and Properties' JOURNAL OF ORGANIC CHEMISTRY vol. 72, no. 17, 2007, pages 6364 - 6371**
- **HONG,X.M. ET AL.: 'Synthesis and polymerization of methylene bridged bis(2-thienyl)benzene' POLYMER PREPRINTS (AMERICAN CHEMICAL SOCIETY, DIVISION OF POLYMER CHEMISTRY) vol. 41, no. 1, 2000, page 189**
- **HADIZAD,T. ET AL.: 'A general synthetic route to indenofluorene derivatives as new organic semiconductors' ORGANIC LETTERS vol. 7, no. 5, 2005, pages 795 - 797**
- **MERLET,S. ET AL.: 'Synthesis and Characterization of Highly Fluorescent Indenofluorenes' ORGANIC LETTERS vol. 4, no. 13, 2002, pages 2157 - 2159**
- **EBEL,F. ET AL.: 'trans-Fluorenacenedione, a new diketone' CHEMISCHE BERICHTE vol. 89, 1956, pages 2794 - 2799**
- **CHAN,S. ET AL.: 'Synthesis, Characterization and Photovoltaic Properties of Novel Semiconducting Polymers with Thiophene-Phenylene-Thiophene (TPT) as Coplanar Units' MACROMOLECULES (WASHINGTON, DC, UNITED STATES) vol. 41, no. 15, 2008, pages 5519 - 5526, XP002550804**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to polycyclic condensed ring compounds which are dihydroindacene compounds, methods for producing the polycyclic condensed ring compounds, and organic semiconductor materials containing the polycyclic condensed ring compounds.

### Background Art

Organic semiconductor devices represented by organic thin film transistors have features, which none but organic molecules have, such as exhibiting energy saving, cost reduction and flexibility, and are expected as elements applicable to the next-generation technology such as electronic papers and large screen flat panel displays. The organic thin film transistor is constituted of several kinds of members including an organic semiconductor active layer, a substrate, an insulating layer and electrodes, but particularly the organic semiconductor active layer taking on the carrier transport has an important function in a device. Characteristics of a transistor depend largely on the carrier transport ability of an organic semiconductor material constituting the organic semiconductor active layer.

As organic semiconductor materials used for organic thin film transistors, various types of organic compounds are proposed. For example, low molecular weight materials such as copper phthalocyanine and pentacene, oligomer materials obtained by linking aromatic 5-membered rings or 6-membered rings such as a thiophene hexamer, and polymer materials such as polyalkylthiophene are reported.

Transistor characteristics of organic thin films are being studied aiming at levels of characteristics that amorphous silicon exhibits. Other required characteristics include the stable drivability, long service life and coatability. However, until now, no organic material satisfying all the conditions has come to be developed.

It is reported, for example, that pentacene has as high a carrier mobility as amorphous silicon has, and develops excellent semiconductor device characteristics (see Non-patent Document 1). However, pentacene has strong cohesiveness and poor solubility, and has also problems with the stable drivability and service life. On the other hand, although oligomer or polymer materials have relatively high coatability, it is the present situation that these have an approximately one-digit lower carrier mobility (see Non-patent Documents 2 and 3).

Although a molecular design is known in which a polycyclic condensed ring compound such as pentacene can be made into an active layer material exhibiting a device with high characteristics, there are few examples of reports because of a problem on the synthesis of a polycyclic condensed ring compound having 5 or more rings. Moreover, polycyclic condensed ring compounds overcoming problematic points (low solubility and air oxidizability) that pentacene has are limited.

On the other hand, the incorporation of a heteroelement in a $\pi$-conjugated skeleton of a polycyclic condensed ring compound becomes one of means to control structural, electronic, optical and physical properties of an organic semiconductor material. It is reported, for example, that dinaphthothienothiophene having a sulfur atom incorporated in a $\pi$-conjugated skeleton is a transistor having a high carrier mobility and high stability (Non-patent Document 4). It is also reported that indolo[3,2-b]carbazole composed of 5 condensed rings having nitrogen atoms incorporated is an organic semiconductor material having coatability and stability (Non-patent Document 5). As seen in these examples, the incorporation of heteroelements in a polycyclic condensed ring compound can be said to be effective means in the molecular design of an organic semiconductor material.

Non-patent Document 1: "Journal of Applied Physics", (the U.S.), 2002, vol. 92, pp. 5259-5263
Non-patent Document 2: "Journal of the American Chemical Society", (the U.S.), 2004, vol. 126, pp. 13859-13874
Non-patent Document 3: "Science", (the U.S.) 1998, vol. 280, pp. 1741-1744
Non-patent Document 4: "Journal of the American Chemical Society", (the U.S.), 2007, vol. 129, pp. 2224-2225
Non-patent Document 5: "Journal of the American Chemical Society", (the U.S.), 2005, vol. 127, pp. 614-618

### Disclosure of the Invention

### Problems to be Solved by the Invention

Under such situations, various types of organic semiconductor materials are desired to be developed. The present invention has an object to provide a polycyclic condensed ring compound composed of 5 condensed rings as a basic skeleton having a heteroelement(s) incorporated, a method for producing the compound, and an organic semiconductor material containing the compound.

### Means for Solving the Problems

As a result of exhaustive studies to solve the above-mentioned problems, the present inventors have found novel heteroelement-containing dihydroindacenes, and methods for producing these. Additionally, the present inventors have found organic semiconductor materials containing the dihydroindacenes, and thin films thereof. These findings have led to the present invention.

That is, the present invention provides a dihydroindacene compound represented by the following formula (3), and applications of the dihydroindacene compound, as defined in the accompanying claims.

[Formula 3]

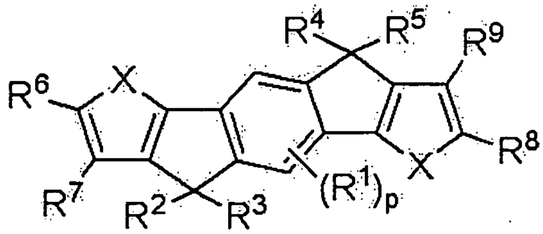

(3)

In the formula (3), $R^1$ is a hydrogen atom, alkyl that has 1 to 30 carbon atoms and may be substituted, alkenyl that has 2 to 30 carbon atoms and may be substituted, alkynyl that has 2 to 30 carbon atoms and may be substituted, or alkoxy that has 1 to 30 carbon atoms and may be substituted;

p is 0, 1, or 2;

$R^2$ to $R^5$ are identical or different from each other, and are each a hydrogen atom, or alkyl that has 1 to 30 carbon atoms and may be substituted;

$R^6$ to $R^9$ are identical or different from each other, and are each a hydrogen atom, alkyl that has 1 to 30 carbon atoms and may be substituted, alkenyl that has 2 to 30 carbon atoms and may be substituted, alkynyl that has 2 to 30 carbon atoms and may be substituted, alkoxy that has 1 to 30 carbon atoms and may be substituted, aryl that has 6 to 30 carbon atoms and may be substituted, silyl that may be substituted, a heteroaryl that may be substituted, or a halogen; and

X is identical or different from each other, and is each sulfur, oxygen, selenium, or $SO_2$,

provided that a compound represented by the following formula (2) is excluded:

[Formula 2]

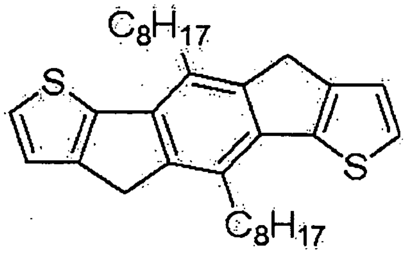

(2)

### Effect of the Invention

The present invention can provide a polycyclic condensed ring compound composed of 5 condensed rings as a basic skeleton having a heteroelement(s) incorporated, a method for producing the compound, and an organic semiconductor material containing the compound. The production method according to the present invention can produce a polycyclic condensed ring compound having a substituent(s) introduced.

### Brief Description of the Drawings

Figure 1 is a diagram of an end surface illustrating an embodiment of an organic transistor in the present invention.

Figure 2 is a diagram illustrating an electric characteristic of an organic transistor having an organic semiconductor

layer composed of 2,7-dihexyl-4,9-dihydrothiopheno[2',3'-6,5]s-indaceno[1,2-b]thiophene in Example 4.

**Explanation of Symbols**

11 SUBSTRATE
12 GATE ELECTRODE
13 GATE INSULATING FILM
14 SOURCE ELECTRODE
15 DRAIN ELECTRODE
16 ORGANIC SEMICONDUCTOR LAYER

**Best Modes for Carrying Out the Invention**

Hereinafter, modes for carrying out the present invention will be described in detail. However, the present invention is not limited to the following embodiments.

In the dihydroindacene compound (hereinafter, referred to as a dihydroindacene compound (3)) represented by the formula (3) according to the present invention, "alkyl" of "alkyl that has 1 to 30 carbon atoms and may be substituted" in $R^1$ to $R^9$ may be any of a straight chain one, a branched chain one and a cyclic one, and examples thereof include straight chain or branched chain alkyls having 1 to 30 carbon atoms, or cyclic alkyls. Specific examples of alkyl groups having 1 to 30 carbon atoms include a methyl group, an ethyl group, a n-propyl group, an isopropyl group, a n-butyl group, an s-butyl group, a t-butyl group, a n-pentyl group, a neopentyl group, a n-hexyl group, a 2-ethylhexyl group, a cyclohexyl group, a n-heptyl group, a n-octyl group, a 2-hexyloctyl group, a n-nonyl group, a n-decyl group, a n-undecyl group, a n-dodecyl group, a n-tridecyl group, a n-tetradecyl group, a n-pentadecyl group, a n-hexadecyl group, a n-heptadecyl group, a n-octadecyl group, a n-nonadecyl group, a n-icosyl group, a n-henicosyl group, a n-docosyl group, a n-tricosyl group, a n-tetracosyl group, a n-pentacosyl group, a n-hexacosyl group, a n-heptacosyl group, a n-octacosyl group, a n-nonacosyl group and a n-triacontyl group, and preferably include a methyl group, an ethyl group, a n-propyl group, an isopropyl group, a n-butyl group, an s-butyl group, a t-butyl group, a n-pentyl group, a neopentyl group, a n-hexyl group, a 2-ethylhexyl group, a n-heptyl group, a n-octyl group, a 2-hexyloctyl group, a n-nonyl group, a n-decyl group, a n-undecyl group, a n-dodecyl group, a n-tridecyl group, a n-tetradecyl group, a n-pentadecyl group, a n-hexadecyl group, a n-heptadecyl group, a n-octadecyl group, a n-nonadecyl group and a n-icosyl group, and more preferably include a methyl group, an ethyl group, a n-propyl group, a n-butyl group, a n-pentyl group, a n-hexyl group, a 2-ethylhexyl group, a n-heptyl group, a n-octyl group, a n-nonyl group, a n-decyl group, a n-undecyl group, a n-dodecyl group, a n-tridecyl group, a 2-hexyloctyl group, a n-tetradecyl group, a n-pentadecyl group and a n-hexadecyl group.

In $R^1$ to $R^9$, specific examples of alkyl groups substituted with a halogen and having 1 to 30 carbon atoms include these alkyl groups substituted with a halogen atom such as a fluorine atom, a chlorine atom, a bromine atom or an iodine atom.

"Alkenyl" of "alkenyl that has 2 to 30 carbon atoms and may be substituted" in $R^1$ and $R^6$ to $R^9$ may be a straight chain one, a branched chain one or a cyclic one, and examples thereof include straight chain or branched chain alkenyls having 2 to 30 carbon atoms. Specific examples of alkenyl groups having 2 to 30 carbon atoms include an ethenyl group, a 1-propenyl group, a 1-butenyl group, a 1-pentenyl group, a 1-hexenyl group, a 1-cyclohexenyl group, a 1-heptenyl group, a 1-octenyl group, a 1-nonenyl group, a 1-decenyl group, a 1-undecenyl group, a 1-dodecenyl group, a 1-tridecenyl group, a 1-tetradecenyl group, a 1-pentadecenyl group, a 1-hexadecenyl group, a 1-heptadecenyl group, a 1-octadecenyl group, a 1-nonadecenyl group, a 1-icosenyl group, a 1-henicosenyl group, a 1-docosenyl group, a 1-tricosenyl group, a 1-tetracosenyl group, a 1-pentacosenyl group, a 1-hexacosenyl group, a 1-heptacosenyl group, a 1-octacosenyl group, a 1-nonacosenyl group and a 1-triacontenyl group, and preferably include an ethenyl group, a 1-propenyl group, a 1-butenyl group, a 1-pentenyl group, a 1-hexenyl group, a 1-heptenyl group, a 1-octenyl group, a 1-nonenyl group, a 1-decenyl group, a 1-undecenyl group, a 1-dodecenyl group, a 1-tridecenyl group, a 1-tetradecenyl group, a 1-pentadecenyl group, a 1-hexadecenyl group, a 1-heptadecenyl group, a 1-octadecenyl group, a 1-nonadecenyl group and a 1-icosenyl group, and more preferably include an ethenyl group, a 1-propenyl group, a 1-butenyl group, a 1-pentenyl group, a 1-hexenyl group, a 1-heptenyl group, a 1-octenyl group, a 1-nonenyl group, a 1-decenyl group, a 1-undecenyl group, a 1-dodecenyl group, a 1-tridecenyl group, a 1-tetradecenyl group, a 1-pentadecenyl group and a 1-hexadecenyl group.

Specific examples of alkenyl groups substituted with a halogen and having 2 to 30 carbon atoms in $R^1$ and $R^6$ to $R^9$ include these alkenyl groups substituted with a halogen atom such as a fluorine atom, a chlorine atom, a bromine atom or an iodine atom.

"Alkynyl" of "alkynyl that has 2 to 30 carbon atoms and may be substituted" in $R^1$ and $R^6$ to $R^9$ may be a straight chain one, a branched chain one or a cyclic one, and examples thereof include straight chain or branched chain alkynyls

having 2 to 30 carbon atoms. Specific examples of alkynyl groups having 2 to 30 carbon atoms include an ethynyl group, a 1-propynyl group, a 1-butynyl group, a 1-pentynyl group, a 1-hexynyl group, a 1-heptynyl group, a 1-octynyl group, a 1-nonynyl group, a 1-decynyl group, a 1-undecynyl group, a 1-dodecynyl group, a 1-tridecynyl group, a 1-tetradecynyl group, a 1-pentadecynyl group, a 1-hexadecynyl group, a 1-heptadecynyl group, a 1-octadecynyl group, a 1-nonadecynyl group, a 1-icosynyl group, a 1-henicosynyl group, a 1-docosynyl group, a 1-tricosynyl group, a 1-tetracosynyl group, a 1-pentacosynyl group, a 1-hexacosynyl group, a 1-heptacosynyl group, a 1-octacosynyl group, a 1-nonacosynyl group and a 1-triacontynyl group, and preferably include an ethynyl group, a 1-propynyl group, a 1-butynyl group, a 1-pentynyl group, a 1-hexynyl group, a 1-heptynyl group, a 1-octynyl group, a 1-nonynyl group, a 1-decynyl group, a 1-undecynyl group, a 1-dodecynyl group, a 1-tridecynyl group, a 1-tetradecynyl group, a 1-pentadecynyl group, a 1-hexadecynyl group, a 1-heptadecynyl group, a 1-octadecynyl group, a 1-nonadecynyl group and a 1-icosynyl group, and more preferably include an ethynyl group, a 1-propynyl group, a 1-butynyl group, a 1-pentynyl group, a 1-hexynyl group, a 1-heptynyl group, a 1-octynyl group, a 1-nonynyl group, a 1-decynyl group, a 1-undecynyl group, a 1-dodecynyl group, a 1-tridecynyl group, a 1-tetradecynyl group, a 1-pentadecynyl group and a 1-hexadecynyl group.

Specific examples of alkynyl groups substituted with a halogen and having 2 to 30 carbon atoms in $R^1$ and $R^6$ to $R^9$ include these alkynyl groups substituted with a halogen atom such as a fluorine atom, a chlorine atom, a bromine atom or an iodine atom.

"Alkoxy" of "alkoxy that has 1 to 30 carbon atoms and may be substituted" in $R^1$ and $R^6$ to $R^9$ may be any of a straight chain one, a branched chain one and a cyclic one, and examples thereof include straight chain or branched chain alkoxys having usually 1 to 30 carbon atoms. Specific examples thereof include a methoxy group, an ethoxy group, a n-propoxy group, an isopropoxy group, a n-butoxy group, an isobutoxy group, a t-butoxy group, a n-pentyloxy group, a neopentyloxy group, a n-hexyloxy group, a n-heptyloxy group, a n-octyloxy group, a 2-ethylhexyloxy group, a nonyloxy group, a decyloxy group, a 3,7-dimethyloctyloxy group, a n-undecyloxy group, a n-dodecyloxy group, a n-tridecyloxy group, a n-tetradecyloxy group, a 2-n-hexyl-n-octyloxy group, a n-pentadecyloxy group, a n-hexadecyloxy group, a n-heptadecyloxy group, a n-octadecyloxy group, a n-nonadecyloxy group, a n-icosyloxy group, a n-henicosyloxy group, a n-docosyloxy group, a n-tricosyloxy group, a n-tetracosyloxy group, a n-pentacosyloxy group, a n-hexacosyloxy group, a n-heptacosyloxy group, a n-octacosyloxy group, a n-nonacosyloxy group and a n-triacontyloxy group, and preferably include an ethoxy group, a n-propoxy group, an isopropoxy group, a n-butoxy group, an isobutoxy group, a t-butoxy group, a n-pentyloxy group, a n-hexyloxy group, a n-heptyloxy group, a n-octyloxy group, a 2-ethylhexyloxy group, a nonyloxy group, a decyloxy group, a 3,7-dimethyloctyloxy group, a n-undecyloxy group, a n-dodecyloxy group, a n-tridecyloxy group, a n-tetradecyloxy group, a n-pentadecyloxy group, a n-hexadecyloxy group, a n-heptadecyloxy group, a n-octadecyloxy group, a n-nonadecyloxy group and a n-icosyloxy group, and more preferably include an ethoxy group, a n-propoxy group, a n-butoxy group, a n-pentyloxy group, a n-hexyloxy group, a n-heptyloxy group, a n-octyloxy group, a 2-ethylhexyloxy group, a nonyloxy group, a decyloxy group, a 3,7-dimethyloctyloxy group, a n-undecyloxy group, a n-dodecyloxy group, a n-tridecyloxy group, a n-tetradecyloxy group, a n-pentadecyloxy group and a n-hexadecyloxy group.

Specific examples of alkoxy groups substituted with a halogen and having 1 to 30 carbon atoms include these alkoxy groups substituted with a halogen atom such as a fluorine atom, a chlorine atom, a bromine atom or an iodine atom.

Specific examples of aryls that have 6 to 30 carbon atoms and may be substituted in $R^6$ to $R^9$ include a phenyl group, a 2-tolyl group, a 3-tolyl group, a 4-tolyl group, a 2,3-xylyl group, a 2,4-xylyl group, a 2,5-xylyl group, a 2,6-xylyl group, a 3,4-xylyl group, a 3,5-xylyl group, a 2,3,4-trimethylphenyl group, a 2,3,5-trimethylphenyl group, a 2,3,6-trimethylphenyl group, a 2,4,6-trimethylphenyl group, a 3,4,5-trimethylphenyl group, a 2,3,4,5-tetramethylphenyl group, a 2,3,4,6-tetramethylphenyl group, a 2,3,5,6-tetramethylphenyl group, a pentamethylphenyl group, a 4-ethylphenyl group, a 4-n-propylphenyl group, a 4-isopropylphenyl group, a 4-n-butylphenyl group, a 4-s-butylphenyl group, a 4-t-butylphenyl group, a 4-n-pentylphenyl group, a 4-neopentylphenyl group, a 4-n-hexylphenyl group, a 4-n-heptylphenyl group, a 4-n-octylphenyl group, a 4-(2'-ethylhexyl)phenyl group, a 4-n-decylphenyl group, a 4-n-undecylphenyl group, a 4-n-dodecylphenyl group, a 4-n-tridecylphenyl, a 4-n-tetradecylphenyl group, a 4-(2'-hexyloctyl)phenyl group, a 4-n-pentadecylphenyl group, a 4-n-hexadecylphenyl group, a 4-n-heptadecylphenyl group, a 4-n-octadecylphenyl group, a 4-n-nonadecylphenyl group, a 4-n-icosylphenyl group, a 4-methoxypheny group, a 4-ethoxyphenyl group, a 4-n-propoxyphenyl group, a 4-n-butoxyphenyl group, a 4-n-pentyloxyphenyl group, a 4-n-hexyloxyphenyl group, a 4-n-heptyloxyphenyl group, a 4-n-octyloxyphenyl group, a 4-(2'-ethylhexyl)oxyphenyl group, a 4-nonyloxyphenyl group, a 4-decyloxyphenyl group, a 4-n-undecyloxyphenyl group, a 4-n-dodecyloxyphenyl group, a 4-n-tridecyloxyphenyl group, a 4-n-tetradecyloxyphenyl group, a 4-(2'-n-hexyl-n-octyloxy)phenyl group, a 4-n-pentadecyloxyphenyl group, a 4-n-hexadecyloxyphenyl group, a 4-n-heptadecyloxyphenyl group, a 4-n-octadecyloxyphenyl group, a 4-n-nonadecyloxyphenyl group, a 4-n-icosyloxyphenyl group, a 1-naphthyl group, a 2-naphthyl group, a 1-anthracenyl group, a 2-anthracenyl group, a 9-anthracenyl group, a 1-phenanthryl group, a 2-phenanthryl group, a 3-phenanthryl group, a 4-phenanthryl group, a 9-phenanthryl group and a 2-fluorenyl group, and preferably include a phenyl group, a 4-n-hexylphenyl group, a 4-n-heptylphenyl group, a 4-n-octylphenyl group, a 4-(2'-ethylhexyl)phenyl group, a 4-n-decylphenyl group, a 4-n-undecylphenyl group, a 4-n-dodecylphenyl group, a 4-n-tetradecylphenyl group, a 4-(2'-hexyloctyl)phenyl group, a 4-n-pentadecylphenyl group, a 4-n-hexadecylphenyl group, a 4-n-hexyloxyphenyl group, a 4-n-heptyloxyphenyl group, a 4-n-octyloxyphenyl group, a 4-(2'-ethylhexyloxy)phenyl group, a 4-nonyloxyphenyl group, a 4-decyloxyphenyl group, a 4-n-undecyloxyphenyl group, a 4-n-dodecyloxyphenyl group, a 4-n-tridecyloxyphenyl group, a 4-n-tetradecyloxyphenyl group, a 4-(2'-n-hexyl-n-octyloxy)phenyl group, a 4-n-pentadecyloxyphenyl group, a 4-n-hexadecyloxyphenyl group, a 2-naphthyl group, 2-anthracenyl group and 2-fluorenyl group.

Specific examples of aryl groups substituted with a halogen and having 6 to 30 carbon atoms in $R^6$ to $R^9$ include these aryl groups substituted with a halogen atom such as a fluorine atom, a chlorine atom, a bromine atom or an iodine atom.

Examples of hydrocarbon groups of a silyl group substituted with the hydrocarbon groups in "silyl that may be substituted" in $R^6$ to $R^9$ include alkyl groups having 1 to 30 carbon atoms such as a methyl group, an ethyl group, a n-propyl group, an isopropyl group, a n-butyl group, an s-butyl group, a t-butyl group, an isobutyl group, a n-pentyl group, a n-hexyl group, a cyclohexyl group, a n-heptyl group, a n-octyl group, a n-nonyl group and a n-decyl group, and aryl group, a n-octyl group, a n-nonyl group and a n-decyl group, and aryl groups such as a phenyl group. Specific examples of silyl groups substituted with such hydrocarbon groups having 1 to 30 carbon atoms include monosubstituted silyl groups substituted with hydrocarbon groups having 1 to 30 carbon atoms, such as a methylsilyl group, an ethylsilyl group and a phenylsilyl group, disubstituted silyl groups substituted with hydrocarbon groups having 1 to 30 carbon atoms, such as a dimethylsilyl group, a diethylsilyl group and a diphenylsilyl group, and trisubstituted silyl groups substituted with hydrocarbon groups having 1 to 30 carbon atoms, such as a trimethylsilyl group, a triethylsilyl group, a tri-n-propylsilyl group, a triisopropylsilyl group, a tri-n-butylsilyl group, a tri-s-butylsilyl group, a tri-t-butylsilyl group, a tri-isobutylsilyl group, a t-butyl-dimethylsilyl group, a tri-n-pentylsilyl group, a tri-n-hexylsilyl group, a tricyclohexylsilyl group and a triphenylsilyl group, and preferably include a trimethylsilyl group, a t-butyldimethylsilyl group and a triphenylsilyl group. Other examples of hydrocarbon groups constituting these substituted silyl groups include, in addition to the hydrocarbon groups as described above, hydrocarbon groups substituted with a halogen atom such as a fluorine atom, a chlorine atom, a bromine atom or an iodine atom.

Examples of "heteroaryl" of "heteroaryl that may be substituted" in $R^6$ to $R^9$ include heterocyclic groups containing as ring-constituting atoms 1 to 5 heteroatoms selected from an oxygen atom, a sulfur atom, a nitrogen atom, a selenium atom and a tellurium atom, except carbon atoms. Specific examples thereof include furyl, benzo[b]furyl, furano[3,2-b]furyl, furano[3,2-b]furano[2',3'-d]furyl, dibenzofuryl, thienyl, benzo[b]thienyl, thieno[3,2-b]thienyl, thieno[3,2-b]thieno[2',3'-d]thienyl, dibenzothienyl, pyrrolyl, selenenyl, tellurenyl, oxazolyl, isoxazolyl, thiazolyl, isothiazolyl, imidazolyl, pyrazolyl, oxadiazolyl, furazanyl, thiadiazolyl, triazolyl, tetrazolyl, pyridyl, pyrimidinyl, pyridazinyl, pyrazinyl, triazinyl, benzofuranyl, isobenzofuranyl, indolyl, isoindolyl, 1H-imidazolyl, benzimidazolyl, benzoxazolyl, benzothiazolyl, 1H-benzotriazolyl, quinolyl, isoquinolyl, cinnolyl, quinazolyl, quinoxalinyl, phthalazinyl, naphthyridinyl, purinyl, pteridinyl, carbazolyl, acridinyl, phenoxazinyl, phenothiazinyl, phenazinyl, phenoxathiinyl, thianthrenyl, indolizinyl and fluorenyl, and preferably include furyl, benzo[b]furyl, furano[3,2-b]furyl, furano[3,2-b]furano[2',3'-d]furyl, dibenzofuryl, thienyl, benzo[b]thienyl, thieno[3,2-b]thienyl, thieno[3,2-b]thieno[2',3'-d]thienyl, dibenzothienyl, pyrrolyl, thiazolyl and carbazolyl.

"Halogen" in $R^6$ to $R^9$ includes a fluorine atom, a chlorine atom, a bromine atom and an iodine atom.

The group of preferable compounds in the dihydroindacene compound (3) includes the following.

- A dihydroindacene compound represented by the formula (3) in which $R^6$ and $R^8$ are identical or different from each other, and are each a hydrogen atom, alkyl which has 1 to 30 carbon atoms and may be substituted, aryl which has 6 to 30 carbon atoms and may be substituted, heteroaryl which may be substituted, or a halogen.
- A dihydroindacene compound represented by the formula (3) in which $R^1$ to $R^5$, $R^7$ and $R^9$ are identical or different from each other, and are each a hydrogen atom, methyl or ethyl.

In a dihydroindacene compound represented by the formula (3), X is preferably a sulfur atom. The dihydroindacene compound in which X is a sulfur atom includes the following.

- A dihydroindacene compound represented by the formula (3) in which X is a sulfur atom; $R^1$ to $R^5$, $R^7$ and $R^9$ are each a hydrogen atom; p = 2; and $R^6$ and $R^8$ are each an alkyl group having 1 to 20 carbon atoms, or an alkoxy group having 1 to 20 carbon atoms. Among the group of this compound, a compound in which the alkyl group having 1 to 20 carbon atoms is n-hexyl is more preferable.
- A dihydroindacene compound represented by the formula (3) in which X is a sulfur atom; $R^1$ to $R^5$, $R^7$ and $R^9$ are each a hydrogen atom; p = 2; and $R^6$ and $R^8$ are each 5-(C1-20 alkyl)thiophen-2-yl. Among the group of this compound, a compound in which the 5-(C1-20 alkyl)thiophen-2-yl group is 5-n-hexylthiophen-2-yl is more preferable.
- A dihydroindacene compound represented by the formula (3) in which X is a sulfur atom; $R^1$ to $R^5$, $R^7$ and $R^9$ are each a hydrogen atom; p = 2; and $R^6$ and $R^8$ are each 4-(C1-20 alkyl)phenyl-1-yl, or 4-(C1-20 alkoxy)phenyl-1-yl. Among the group of this compound, a compound in which the 4-(C1-20 alkyl)phenyl-1-yl is 4-n-hexylphenyl, or a compound in which the 4-(C1-20 alkoxy)phenyl-1-yl is 4-n-hexyloxyphenyl is more preferable.

Specific examples of the dihydroindacene compound (3) according to the present invention include compounds represented by the following formulae (3-1) to (3-144), but the compound is not limited thereto. In the formulae, n is identical or different from each other, and each denotes an integer of 0 to 30.

## [Formula 4]

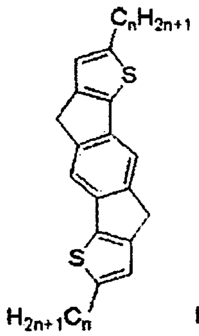
(3-1)

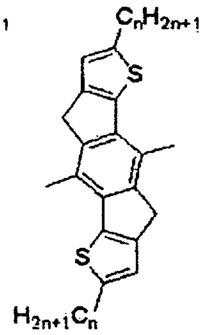
(3-2)

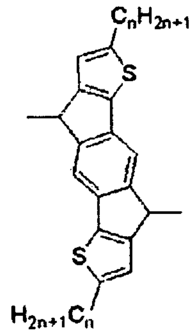
(3-3)

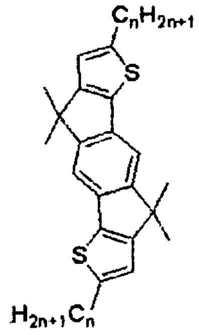
(3-4)

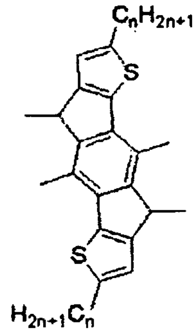
(3-5)

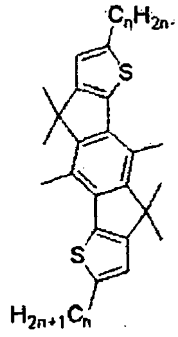
(3-6)

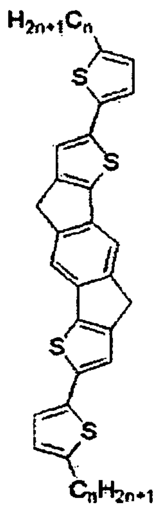
(3-7)

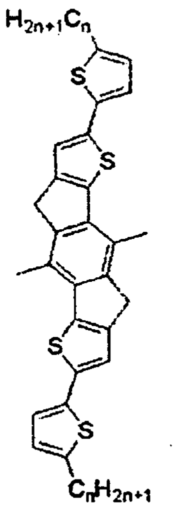
(3-8)

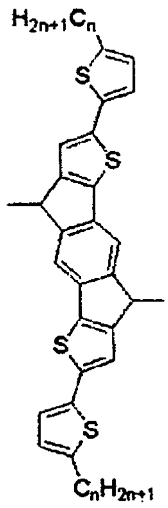
(3-9)

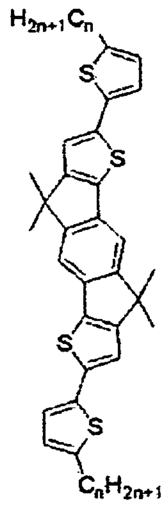
(3-10)

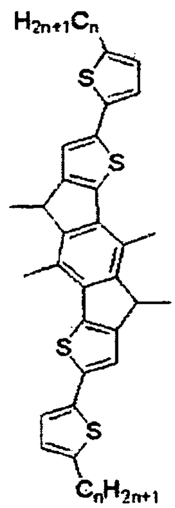
(3-11)

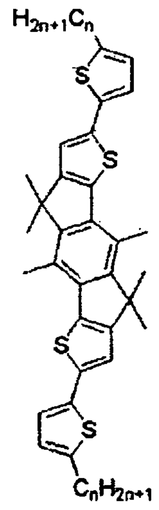
(3-12)

## [Formula 5]

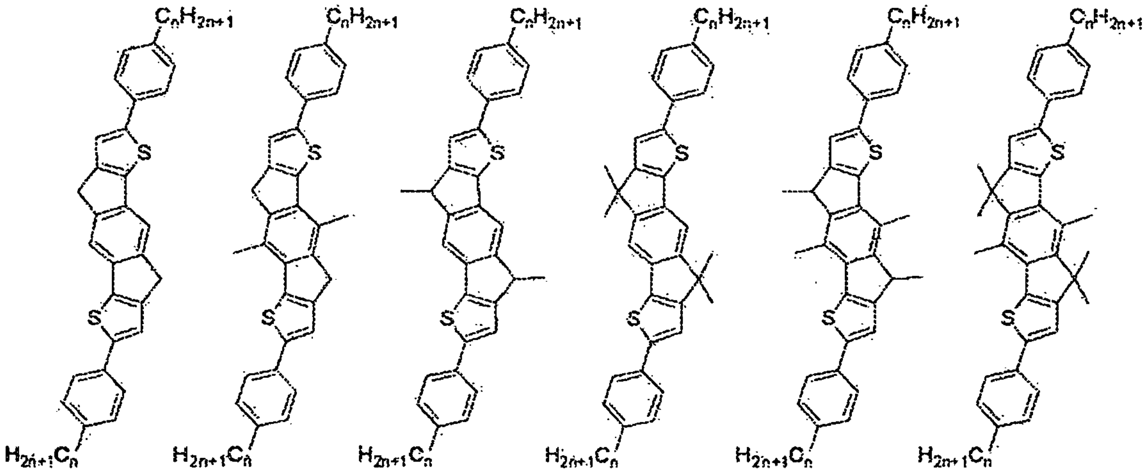

(3-13) (3-14) (3-15) (3-16) (3-17) (3-18)

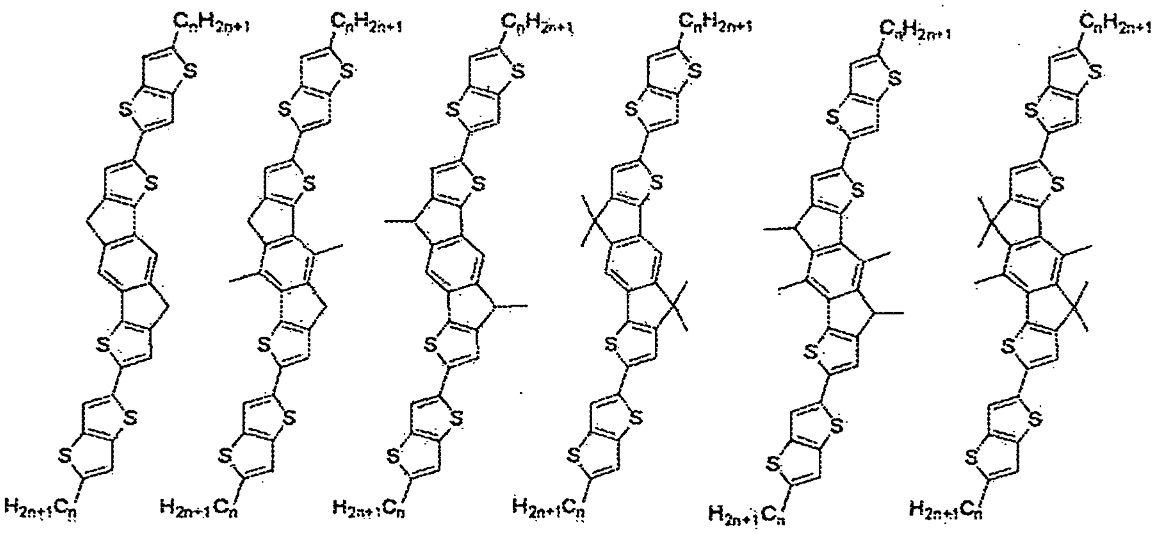

(3-19) (3-20) (3-21) (3-22) (3-23) (3-24)

[Formula 6]

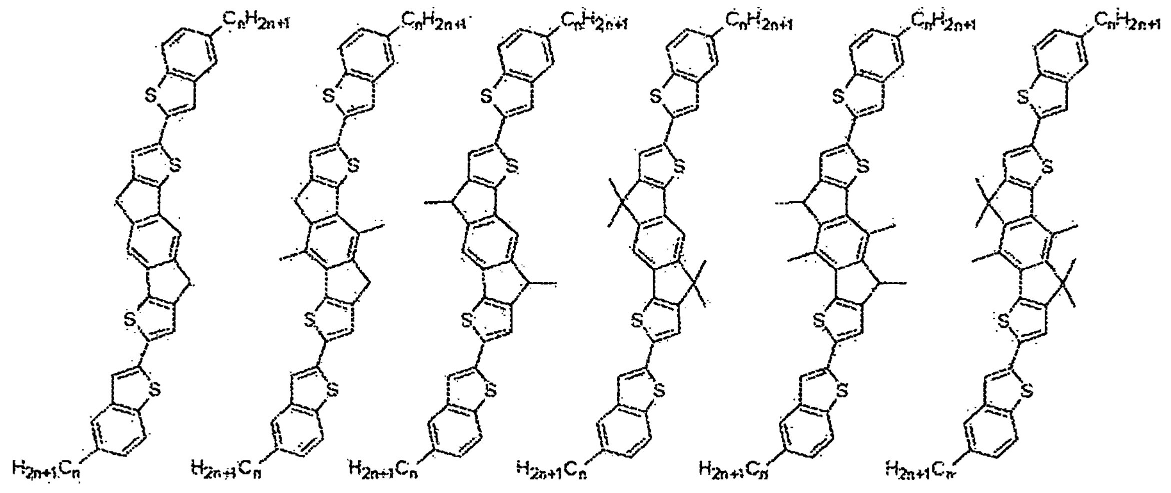

(3-25) (3-26) (3-27) (3-28) (3-29) (3-30)

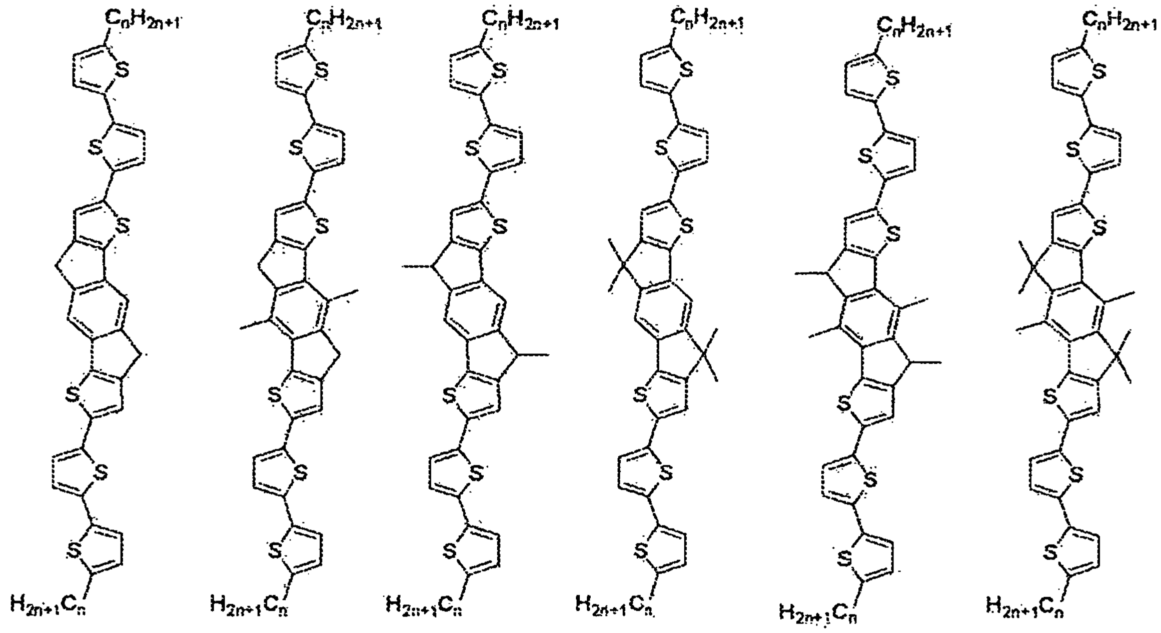

(3-31) (3-32) (3-33) (3-34) (3-35) (3-36)

[Formula 7]

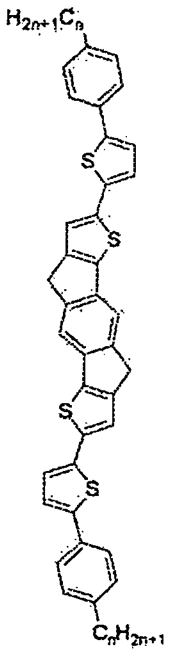
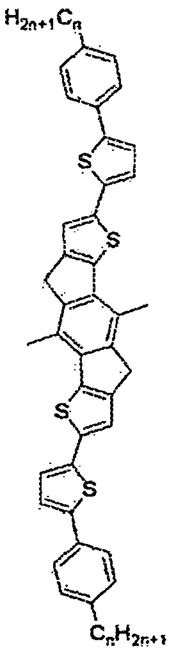
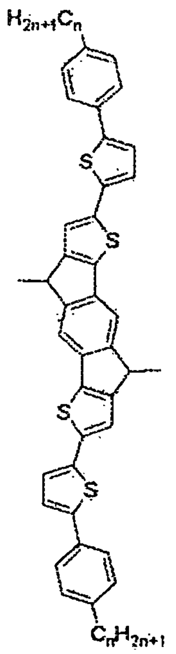
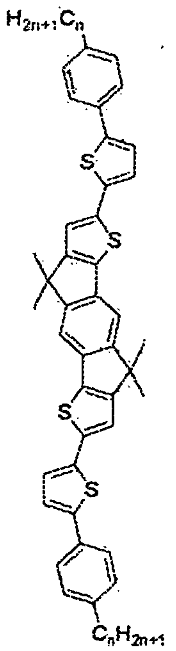
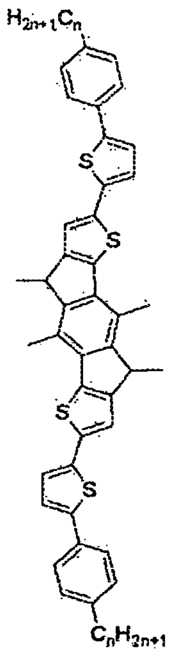
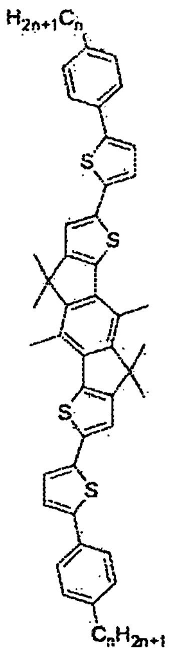

(3-37) (3-38) (3-39) (3-40) (3-41) (3-42)

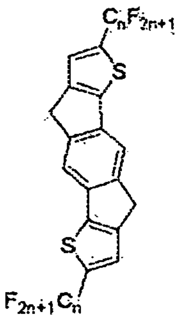
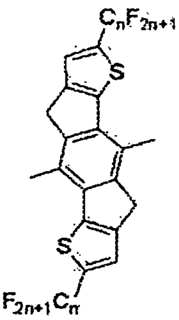
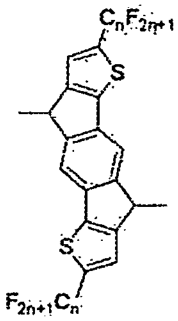
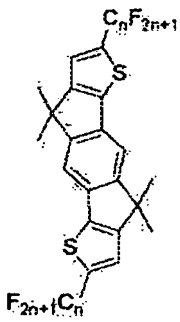
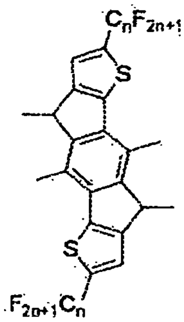
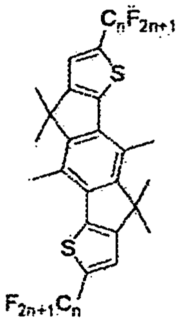

(3-43) (3-44) (3-45) (3-46) (3-47) (3-48)

[Formula 8]

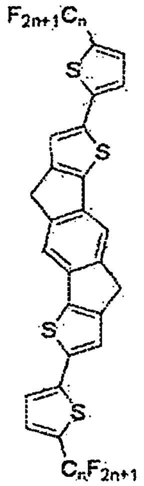

(3-49)

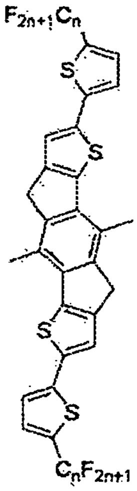

(3-50)

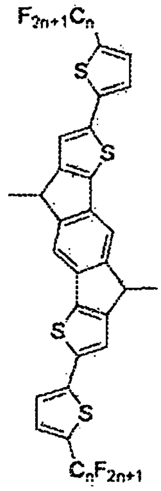

(3-51)

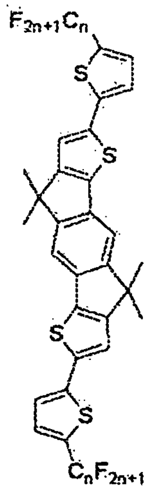

(3-52)

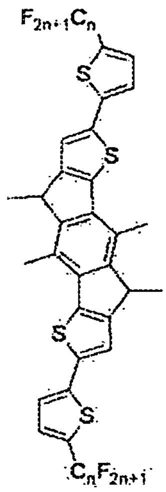

(3-53)

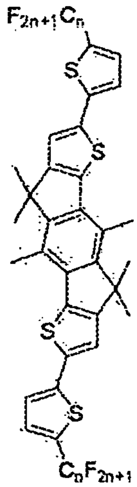

(3-54)

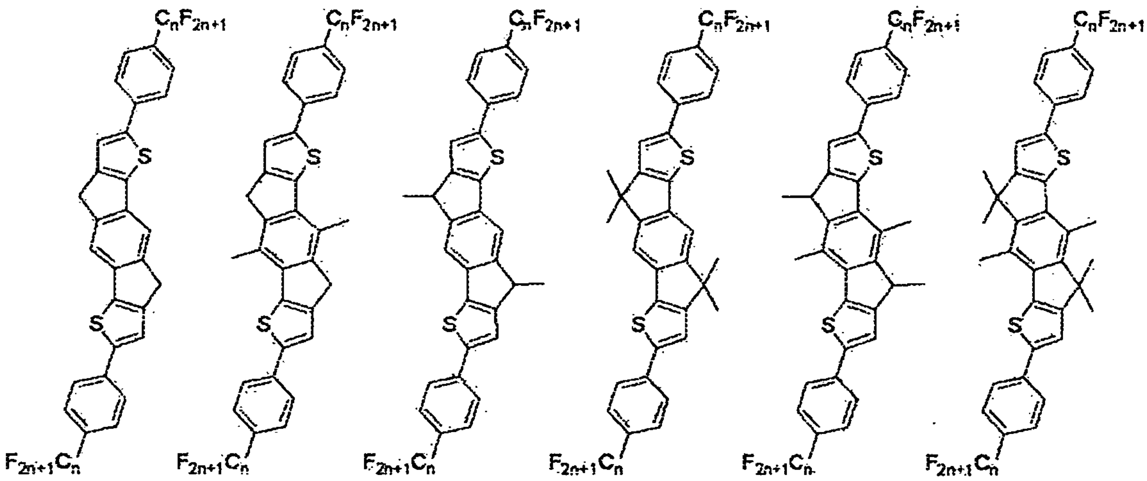

(3-55) (3-56) (3-57) (3-58) (3-59) (3-60)

[Formula 9]

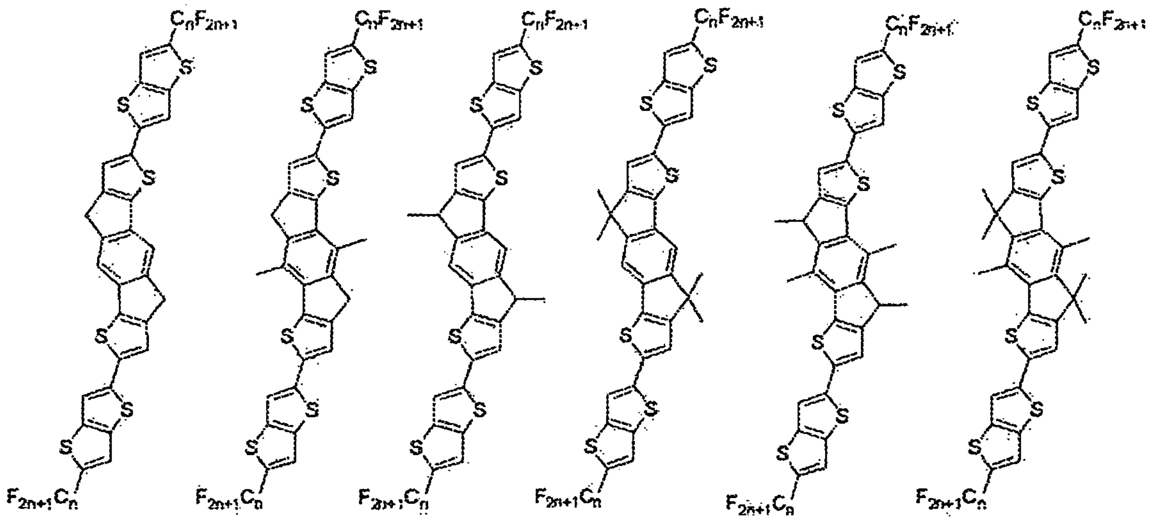

(3-61) (3-62) (3-63) (3-64) (3-65) (3-66)

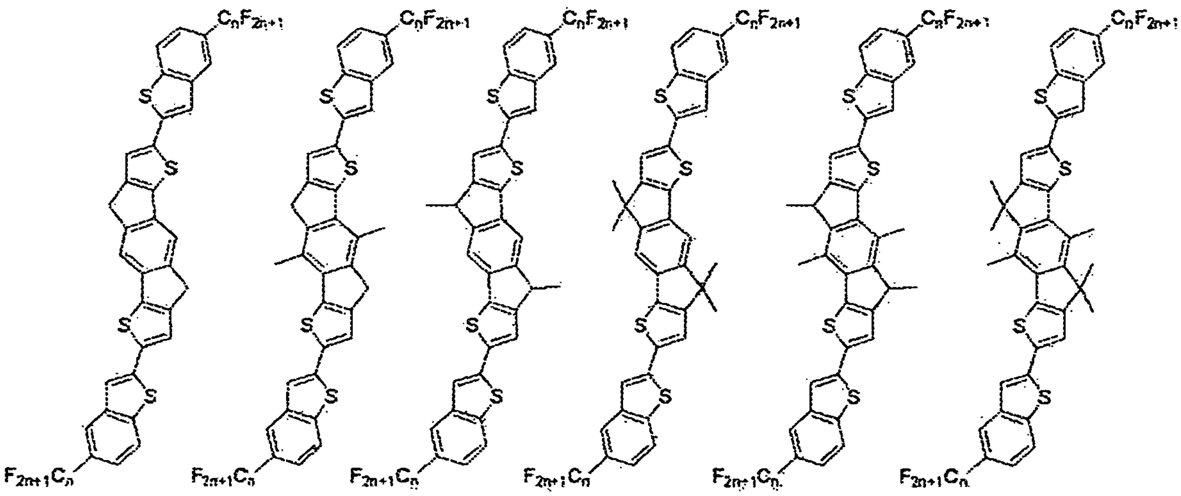

(3-67) (3-68) (3-69) (3-70) (3-71) (3-72)

[Formula 10]

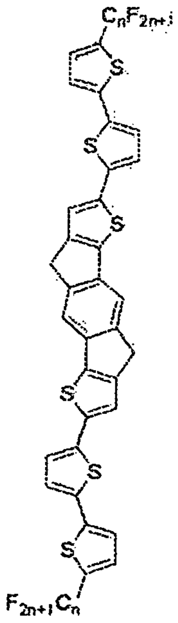
(3-73)

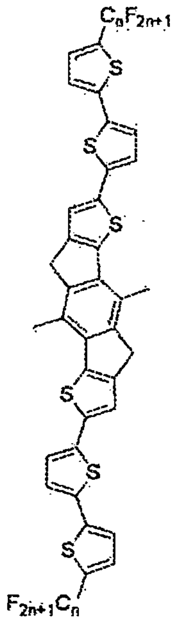
(3-74)

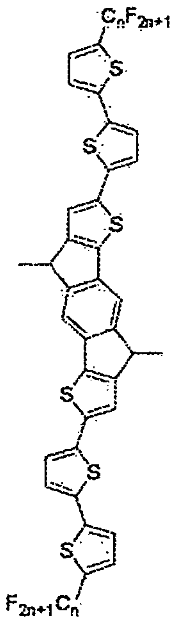
(3-75)

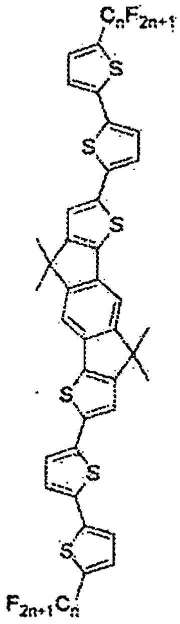
(3-76)

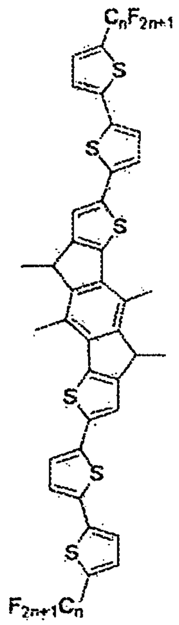
(3-77)

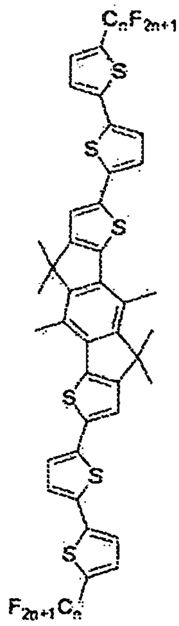
(3-78)

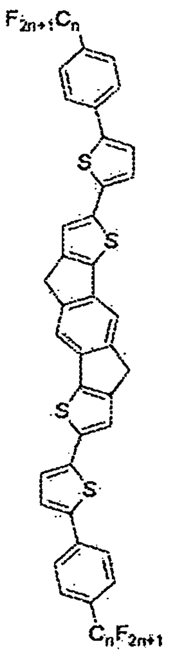
(3-79)

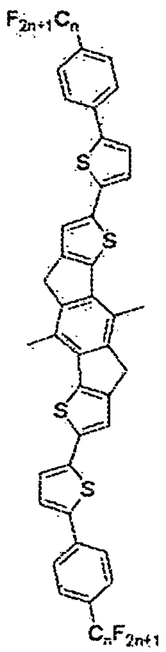
(3-80)

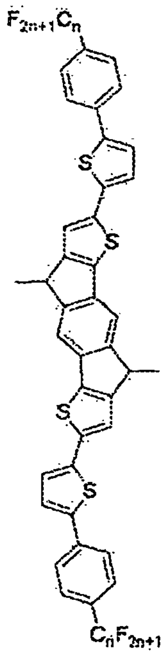
(3-81)

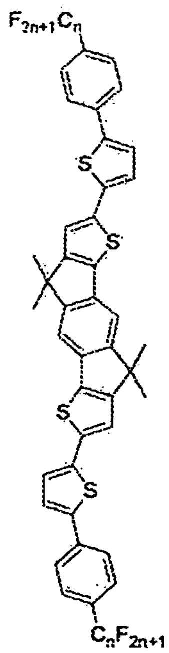
(3-82)

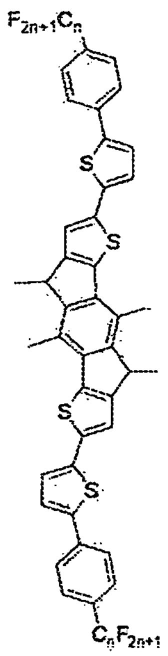
(3-83)

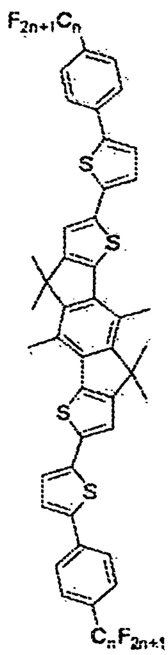
(3-84)

[Formula 11]

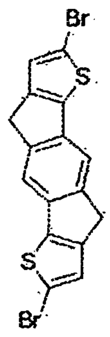
(3-85)

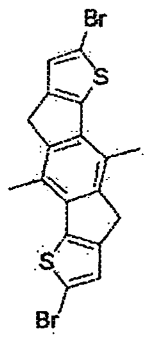
(3-86)

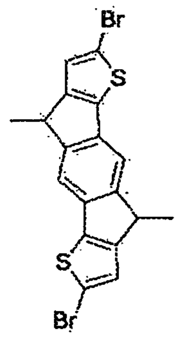
(3-87)

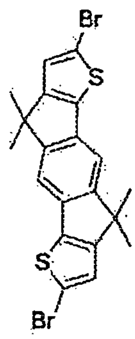
(3-88)

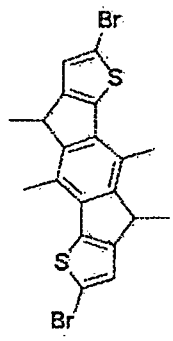
(3-89)

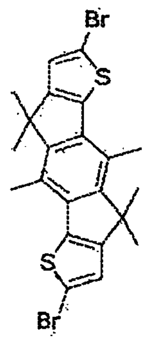
(3-90)

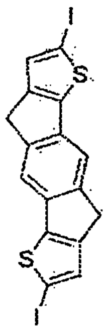
(3-91)

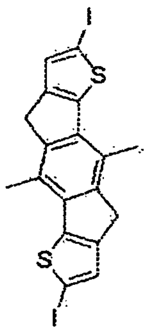
(3-92)

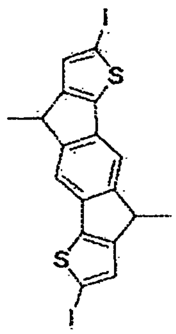
(3-93)

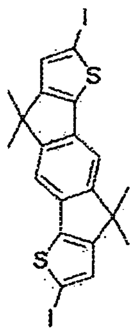
(3-94)

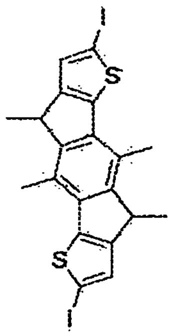
(3-95)

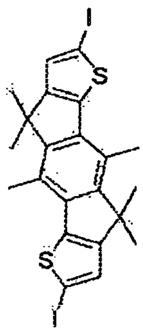
(3-96)

[Formula 12]

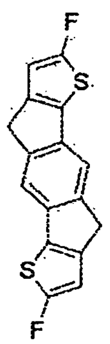
(3-97)

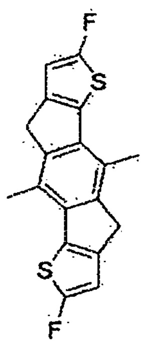
(3-98)

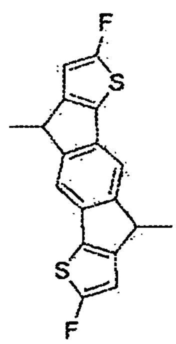
(3-99)

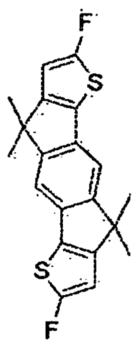
(3-100)

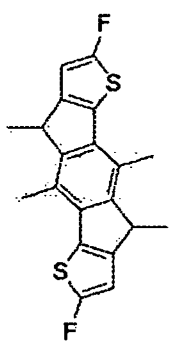
(3-101)

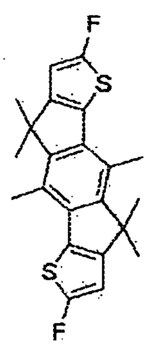
(3-102)

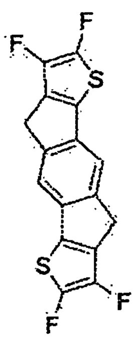
(3-103)

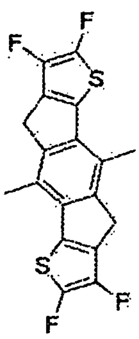
(3-104)

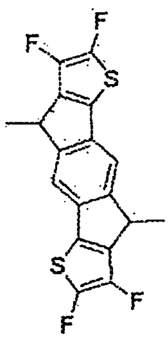
(3-105)

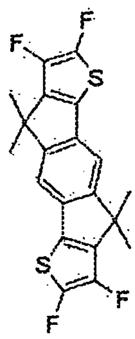
(3-106)

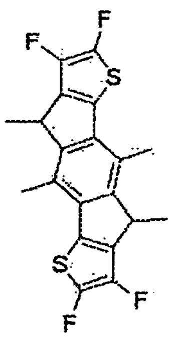
(3-107)

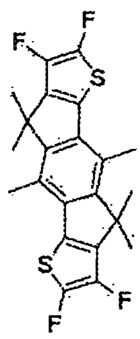
(3-108)

[Formula 13]

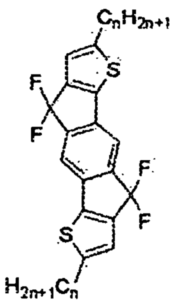
(3-109)

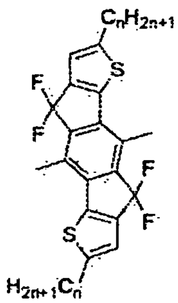
(3-110)

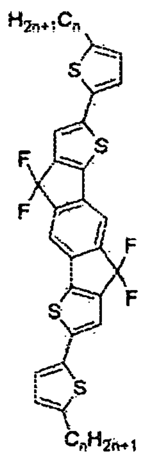
(3-111)

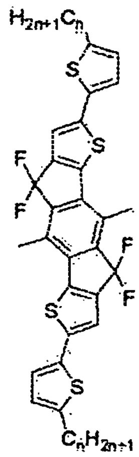
(3-112)

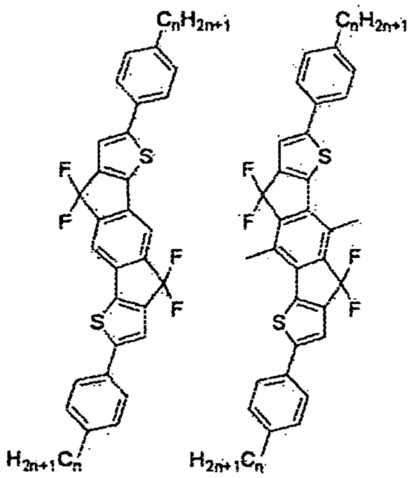
(3-113) (3-114)

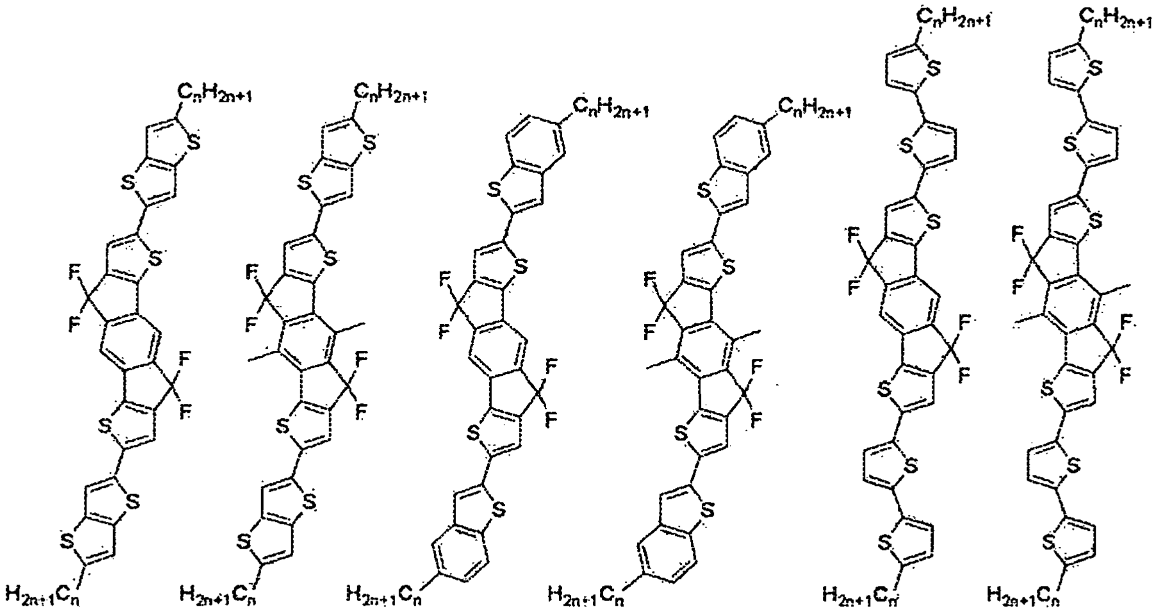
(3-115) (3-116) (3-117) (3-118) (3-119) (3-120)

[Formula 14]

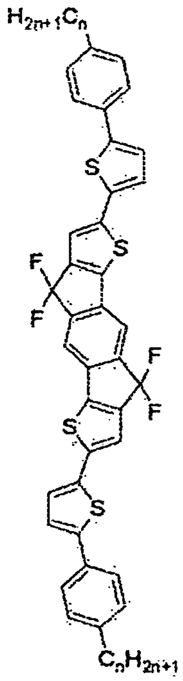
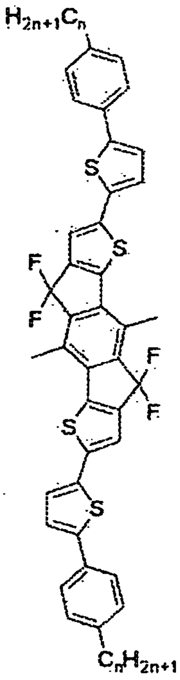
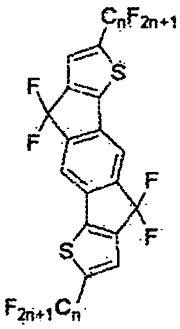
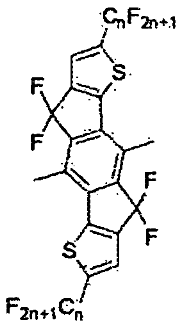
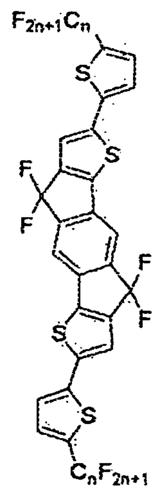
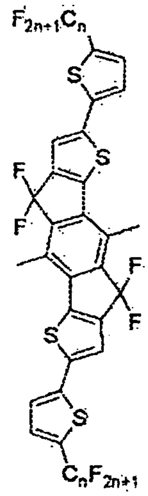

(3-121) (3-122) (3-123) (3-124) (3-125) (3-126)

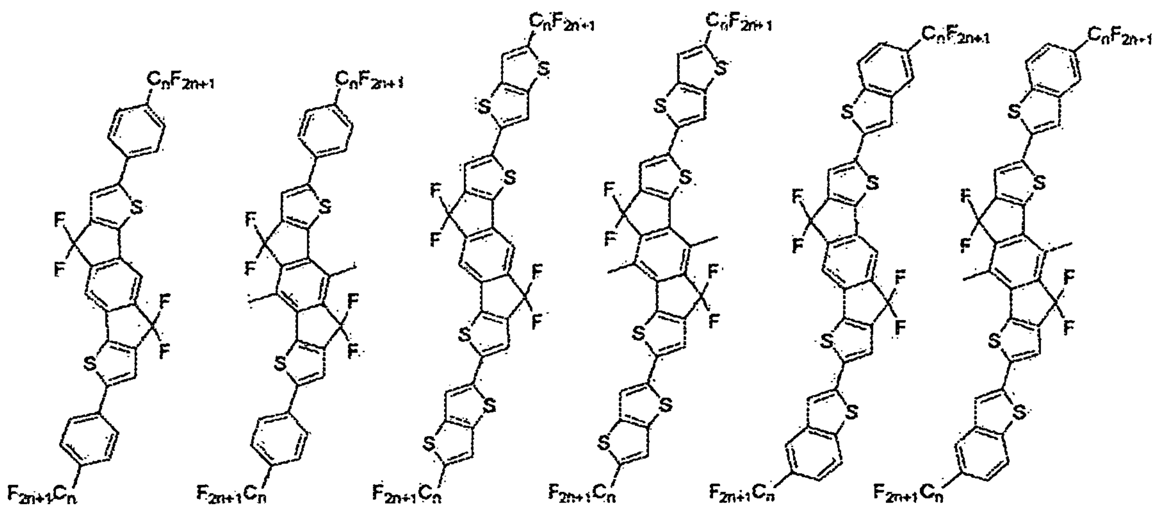

(3-127) (3-128) (3-129) (3-130) (3-131) (3-132)

[Formula 15]

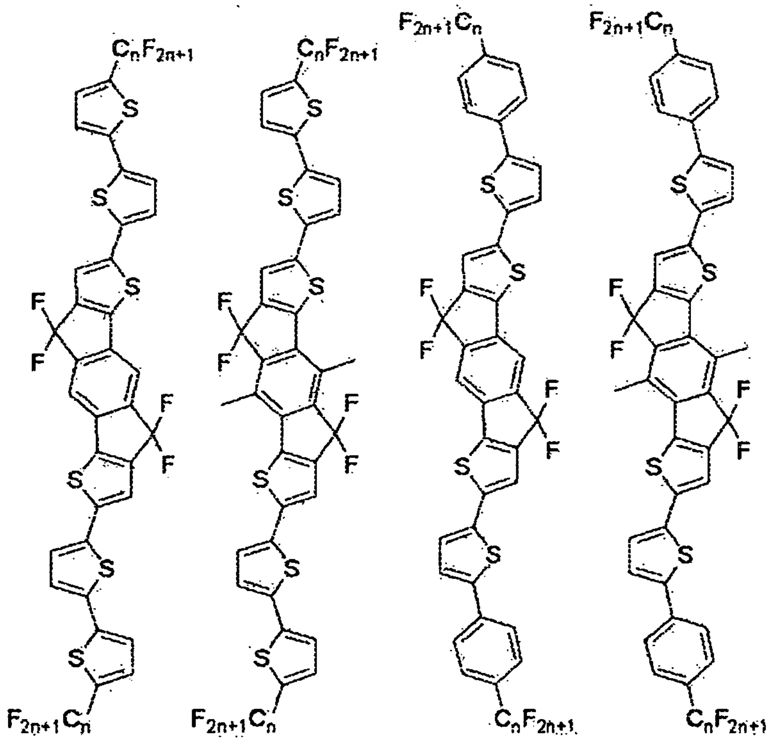
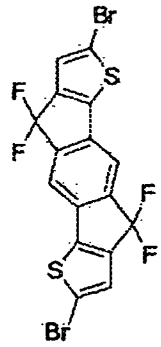
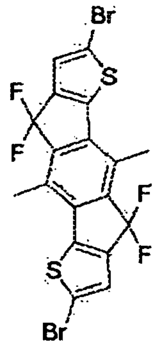

(3-133) (3-134) (3-135) (3-136) (3-137) (3-138)

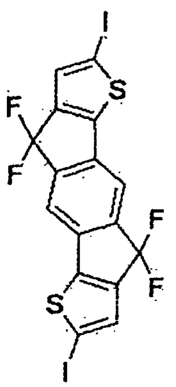
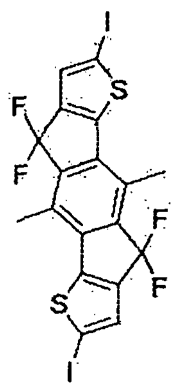
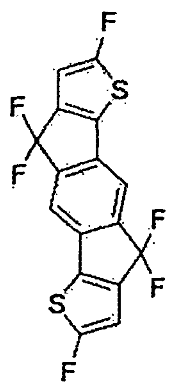
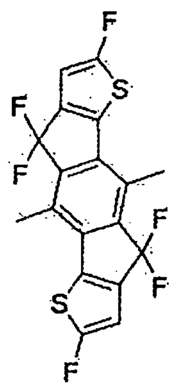
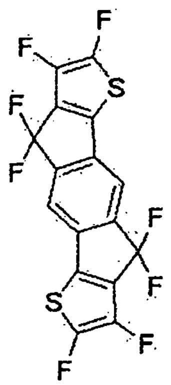
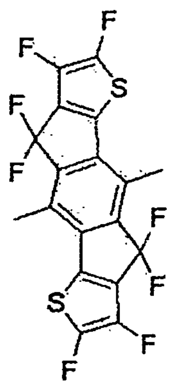

(3-139) (3-140) (3-141) (3-142) (3-143) (3-144)

The dihydroindacene compound (3) preferably includes compounds represented by the formulae (3-1), (3-4), (3-7), (3-10), (3-13), (3-16), (3-19), (3-22), (3-25), (3-28), (3-31), (3-34), (3-37), (3-40), (3-43), (3-46), (3-49), (3-52), (3-55), (3-58), (3-61), (3-64), (3-67), (3-70), (3-73), (3-76), (3-79), (3-82), (3-85), (3-88), (3-91), (3-94), (3-97), (3-100), (3-103), (3-106), (3-109), (3-111), (3-113), (3-115), (3-117), (3-119), (3-121), (3-123), (3-125), (3-127), (3-129), (3-131), (3-133), (3-135), (3-137), (3-139), (3-141) and (3-143), and more preferably includes compounds represented by the formulae (3-1), (3-7), (3-13), (3-19), (3-25), (3-31), (3-37), (3-43), (3-49), (3-55), (3-61), (3-67), (3-73), (3-79), (3-85), (3-91), (3-97), (3-103), (3-109), (3-111), (3-113), (3-115), (3-117), (3-119), (3-121), (3-123), (3-125), (3-127), (3-129), (3-131), (3-133), (3-135), (3-137), (3-139), (3-141) and (3-143).

The dihydroindacene compound (3) (provided that $R^2$ to $R^5$ are all hydrogen) can be produced by allowing a basic reagent to act on a dihydrazone compound (hereinafter, referred to as a dihydrazone compound (4)) represented by the following formula (4).

[Formula 16]

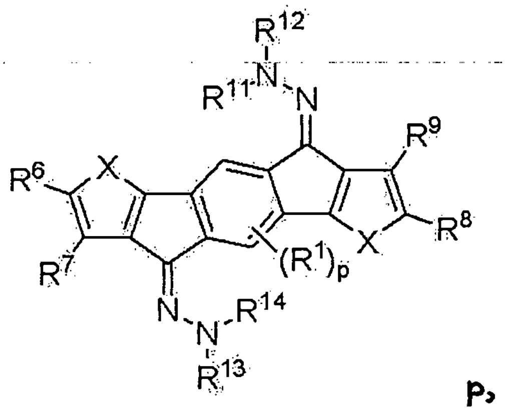

(4)

In the formula (4), $R^1$ P, $R^6$ to $R^9$ and X are each the same as described in the representations of R1, p, $R^6$ to $R^9$ and X in the formula (3); and

$R^{11}$ to $R^{14}$ are identical or different from each other, and each denote a hydrogen atom, alkyl which may be substituted, aryl which may be substituted, arylsulfonyl which may be substituted, or silyl which may be substituted.

Substituents of the dihydrazone compound (4) will be described further in detail.

A hydrogen atom, alkyl which may be substituted, and aryl which may be substituted, denoted as $R^{11}$ to $R^{14}$, are included in the same substituents as shown for $R^1$ to $R^9$ or $R^6$ to $R^9$ described above.

In $R^{11}$ to $R^{14}$, "arylsulfonyl" of "arylsulfonyl which may be substituted" includes arylsulfonyl groups having 6 to 30 carbon atoms. Specific examples thereof include a phenylsulfonyl group, a 2-tolylsulfonyl group, a 3-tolylsulfonyl group, a 4-tolylsulfonyl group, a 2,3-xylylsulfonyl group, a 2,4-xylylsulfonyl group, a 2,5-xylylsulfonyl group, a 2,6-xylylsulfonyl group, a 3,4-xylylsulfonyl group, a 3,5-xylylsulfonyl group, a 2,3,4-trimethylphenylsulfonyl group, a 2,3,5-trimethylphenylsulfonyl group, a 2,3,6-trimethylphenylsulfonyl group, a 2,4,6-trimethylphenylsulfonyl group, a 3.4.5-trimethylphenylsulfonyl group, a 2,3,4,5-tetramethylphenylsulfonyl group, a 2,3,4,6-tetramethylphenylsulfonyl group, a 2,3,5,6-tetramethylphenylsulfonyl group, a pentamethylphenylsulfonyl group, an ethylphenylsulfonyl group, a n-propylphenylsulfonylgroup, an isopropylphenylsulfonyl group, a n-butylphenylsulfonyl group, an s-butylphenylsulfonyl group, a t-butylphenylsulfonyl group, a n-pentylphenylsulfonyl group, a neopentylphenylsulfonyl group, a n-hexylphenylsulfonyl group, a n-octylphenylsulfonyl group, a n-decylphenylsulfonyl group, a n-dodecylphenylsulfonyl group, a n-tetradecylphenylsulfonyl group, a naphthylsulfonyl group, an anthracenylsulfonyl group and a fluorenylsulfonyl group, and preferably include a phenylsulfonyl group, a 4-tolylsulfonyl group, a n-hexylphenylsulfonyl group, a n-octylphenylsulfonyl group, a n-decylphenylsulfonyl group, a n-dodecylphenylsulfonyl group, a n-tetradecylphenylsulfonyl group, a naphthylsulfonyl group, an anthracenylsulfonyl group and a fluorenylsulfonyl group.

In $R^{11}$ to $R^{14}$, specific examples of arylsulfonyl groups substituted with a halogen and having 6 to 30 carbon atoms include these arylsulfonyl groups substituted with a halogen atom such as a fluorine atom, a chlorine atom, a bromine atom and an iodine atom.

"A silyl group which may be substituted" in $R^{11}$ to $R^{14}$ includes silyl groups substituted with hydrocarbon groups, and examples of the hydrocarbon groups include alkyl groups having 1 to 30 carbon atoms, such as a methyl group, an ethyl group, a n-propyl group, an isopropyl group, a n-butyl group, an s-butyl group, a t-butyl group, an isobutyl group, a n-pentyl group, a n-hexyl group, a cyclohexyl group, a n-heptyl group, a n-octyl group, a n-nonyl group and n-decyl group, and aryl groups such as a phenyl group. Specific examples of silyl groups substituted with such hydrocarbon groups having 1 to 30 carbon atoms include monosubstituted silyl groups substituted with hydrocarbon groups having 1 to 30 carbon atoms, such as a methylsilyl group, an ethylsilyl group and a phenylsilyl group, disubstituted silyl groups substituted with hydrocarbon groups having 1 to 30 carbon atoms, such as a dimethylsilyl group, a diethylsilyl group and a diphenylsilyl group, and trisubstituted silyl groups substituted with hydrocarbon groups having 1 to 30 carbon atoms, such as a trimethylsilyl group, a triethylsilyl group, a tri-n-propylsilyl group, a triisopropylsilyl group, a tri-n-butylsilyl group, a tri-s-butylsilyl group, a tri-t-butylsilyl group, a tri-isobutylsilyl group, a t-butyl-dimethylsilyl group, a tri-n-pentylsilyl group, a tri-n-hexylsilyl group, a tricyclohexylsilyl group and a triphenylsilyl group, and preferably include a trimethylsilyl group, a t-butyldimethylsilyl group and a triphenylsilyl group.

In $R^{11}$ to $R^{14}$, specific examples of halogen-substituted silyl groups substituted with hydrocarbon groups having 1 to 30 carbon atoms include, in addition to the hydrocarbon groups as described above, silyl groups substituted with a halogen atom such as a fluorine atom, a chlorine atom, a bromine atom and an iodine atom.

The group of preferable compounds in the dihydrazone compound (4) includes the following.

- A dihydrazone compound represented by the formula (4) in which:

  $R^{11}$ to $R^{14}$ are identical or different from each other, and are each a hydrogen atom, alkyl which has 1 to 30 carbon atoms and may be substituted, arylsulfonyl which has 6 to 30 carbon atoms and may be substituted, or silyl which may be substituted.

- A dihydrazone compound represented by the formula (4) in which $R^6$ and $R^8$ are identical or different from each other, and are each alkyl which has 1 to 30 carbon atoms and may be substituted, aryl which has 6 to 30 carbon atoms and may be substituted, heteroaryl which may be substituted, or a halogen.
- A dihydrazone compound represented by the formula (4) in which $R^1$, $R^7$ and $R^9$ are identical or different from each other, and are each a hydrogen atom, methyl or ethyl.
- A dihydrazone compound represented by the formula (4) in which X is a sulfur atom; $R^1$, $R^7$, $R^9$ and $R^{11}$ to $R^{14}$ are each a hydrogen atom; p = 2; $R^6$ and $R^8$ are each an alkyl group having 1 to 20 carbon atoms, or an alkoxy group having 1 to 20 carbon atoms.
- A dihydrazone compound represented by the formula (4) in which X is a sulfur atom; $R^1$, $R^7$, $R^9$ and $R^{11}$ to $R^{14}$ are each a hydrogen atom; p = 2; $R^6$ and $R^8$ are each 5-(C1-20 alkyl)thiophen-2-yl.
- A dihydrazone compound represented by the formula (4) in which X is a sulfur atom; $R^1$, $R^7$, $R^9$ and $R^{11}$ to $R^{14}$ are each a hydrogen atom; p = 2; $R^6$ and $R^8$ are each 4-(C1-20 alkyl)phenyl-1-yl, or 4-(C1-20 alkoxy)phenyl-1-yl.
- A dihydrazone compound represented by the formula (4) in which X is a sulfur atom.
- A dihydrazone compound represented by the formula (4) in which X is a sulfur atom; $R^1$, $R^7$, $R^9$ and $R^{11}$ to $R^{14}$ are each a hydrogen atom; p = 2; and $R^6$ and $R^8$ are each n-hexyl.
- A dihydrazone compound represented by the formula (4) in which X is a sulfur atom; $R^1$, $R^7$, $R^9$ and $R^{11}$ to $R^{14}$ are each a hydrogen atom; p = 2; $R^6$ and $R^8$ are each 5-n-hexylthiophen-2-yl.
- A dihydrazone compound represented by the formula (4) in which X is a sulfur atom; $R^1$, $R^7$, $R^9$ and $R^{11}$ to $R^{14}$ are each a hydrogen atom; p = 2; and $R^6$ and $R^8$ are each 4-n-hexylphenyl.

The dihydrazone compound (4) specifically includes compounds represented by the following formulae (4-1) to (4-74), but the compounds are not limited thereto. In the formulae, n is identical or different from each other, and each denotes an integer of 0 to 30.

[Formula 17]

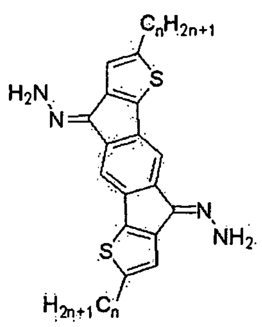

(4-1)

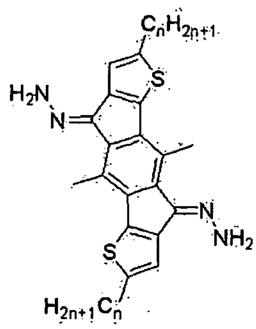

(4-2)

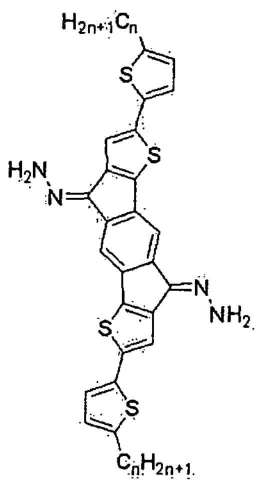

(4-3)

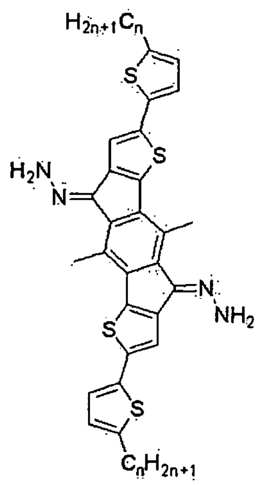

(4-4)

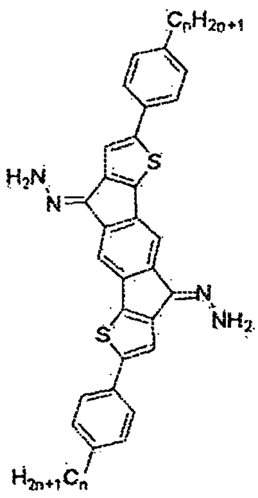 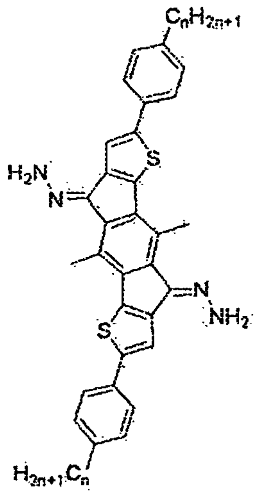 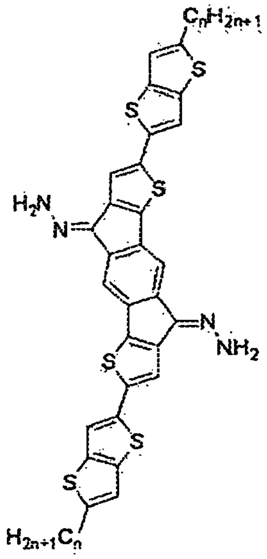 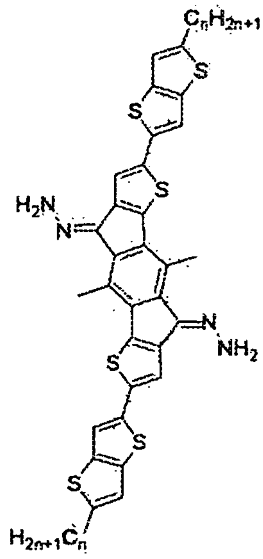

(4-5) (4-6) (4-7) (4-8)

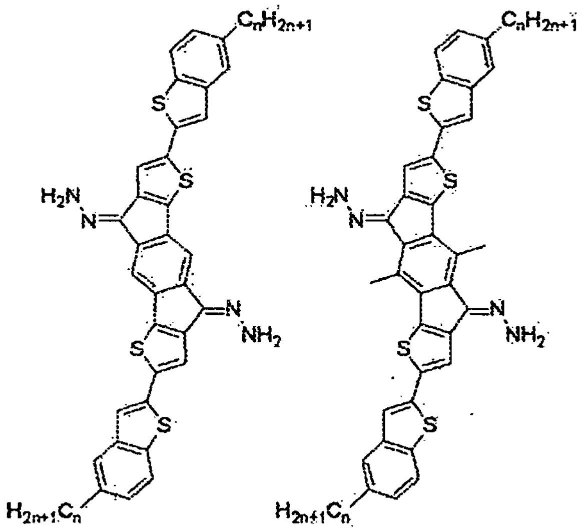 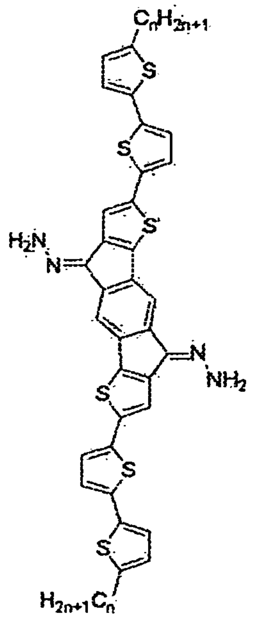 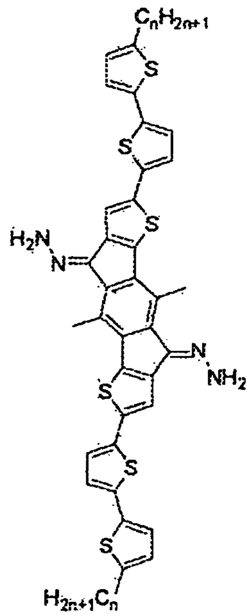

(4-9) (4-10) (4-11) (4-12)

[Formula 18]

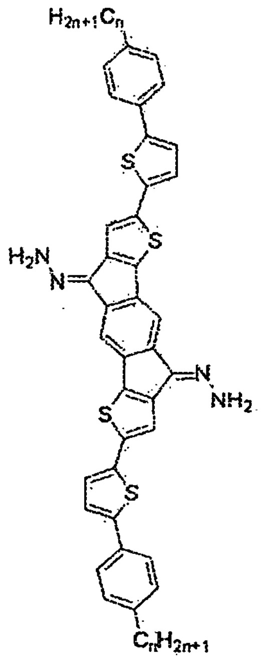

(4-13)

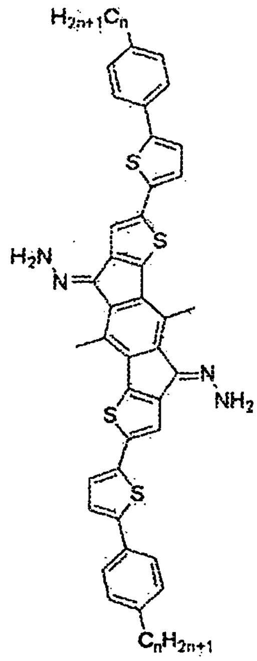

(4-14)

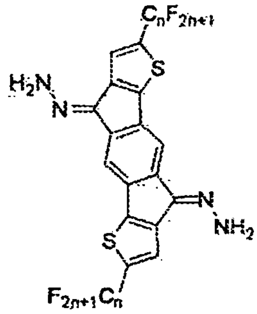

(4-15)

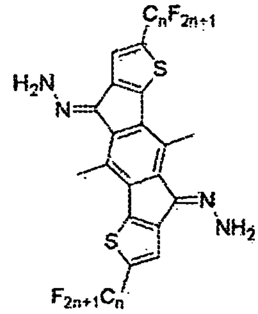

(4-16)

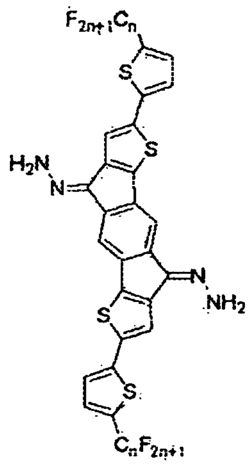

(4-17)

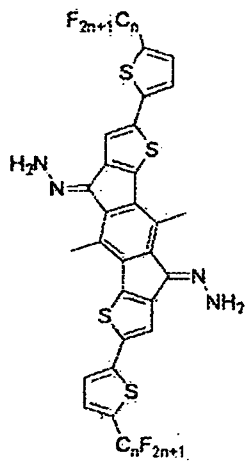

(4-18)

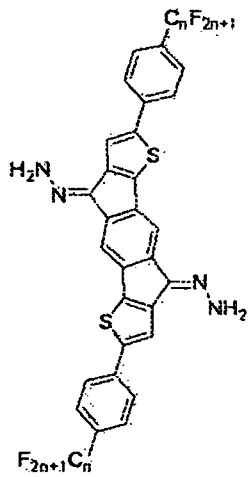

(4-19)

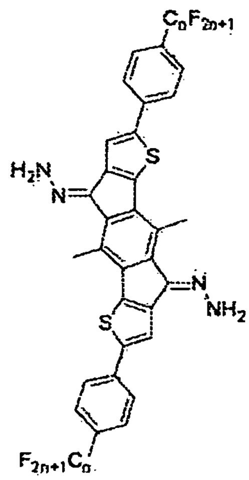

(4-20)

[Formula 19]

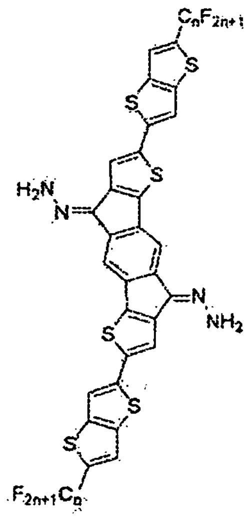

(4-21)

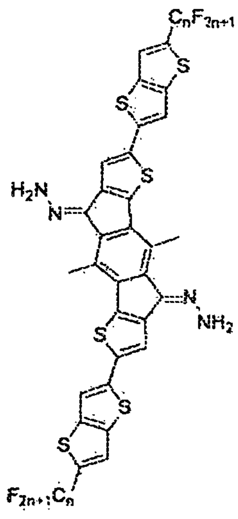

(4-22)

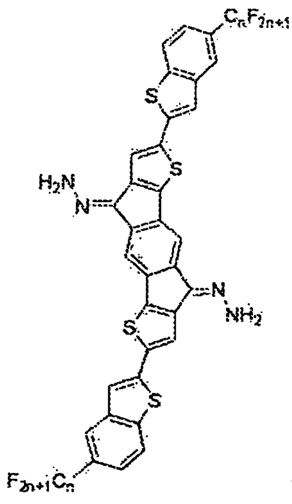

(4-23)

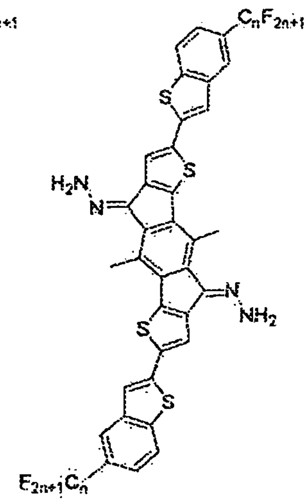

(4-24)

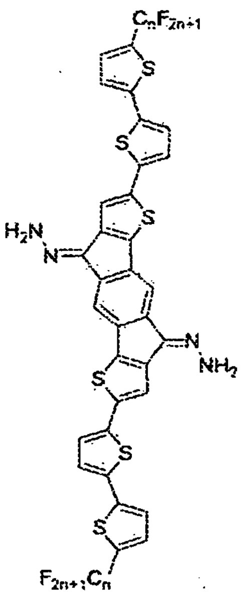

(4-25)

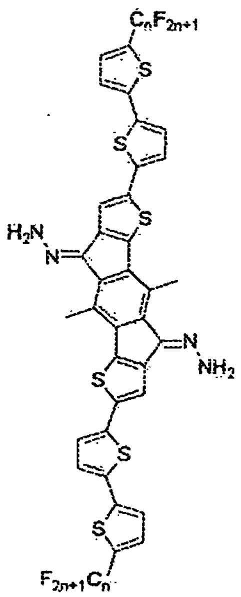

(4-26)

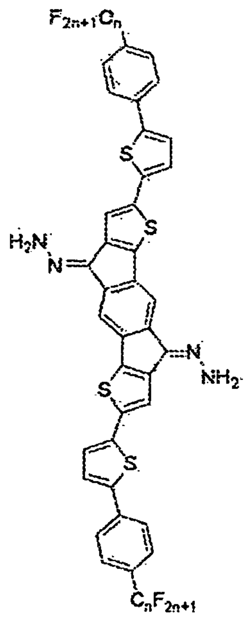

(4-27)

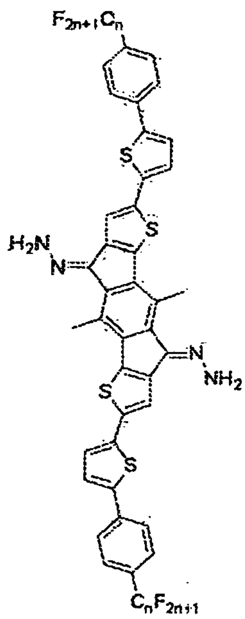

(4-28).

[Formula 20]

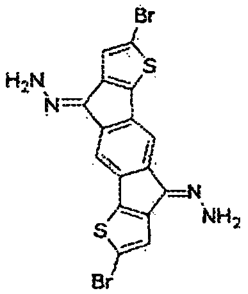
(4-29)

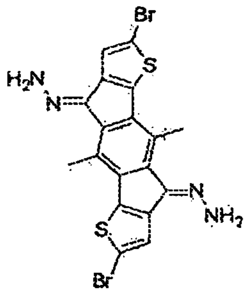
(4-30)

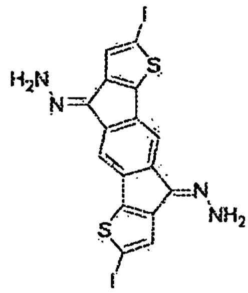
(4-31)

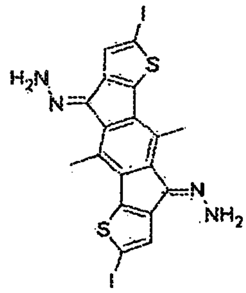
(4-32)

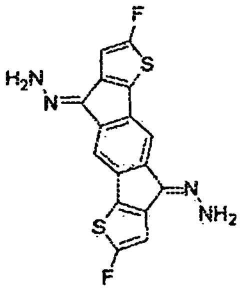
(4-33)

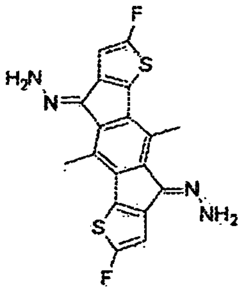
(4-34)

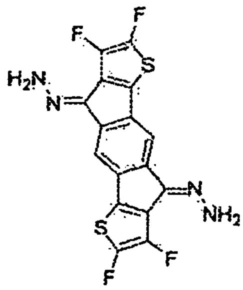
(4-35)

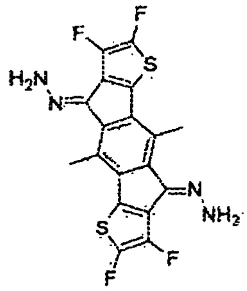
(4-36)

[Formula 21]

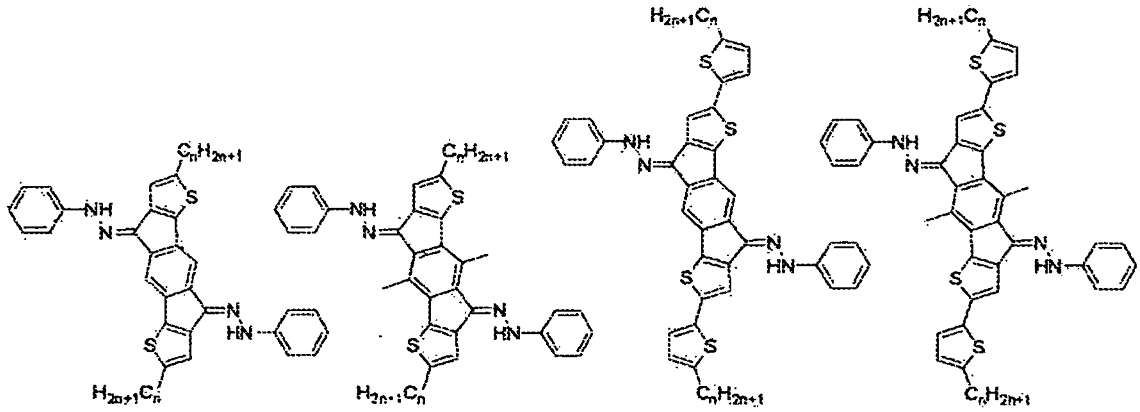
(4-37) (4-38) (4-39) (4-40)

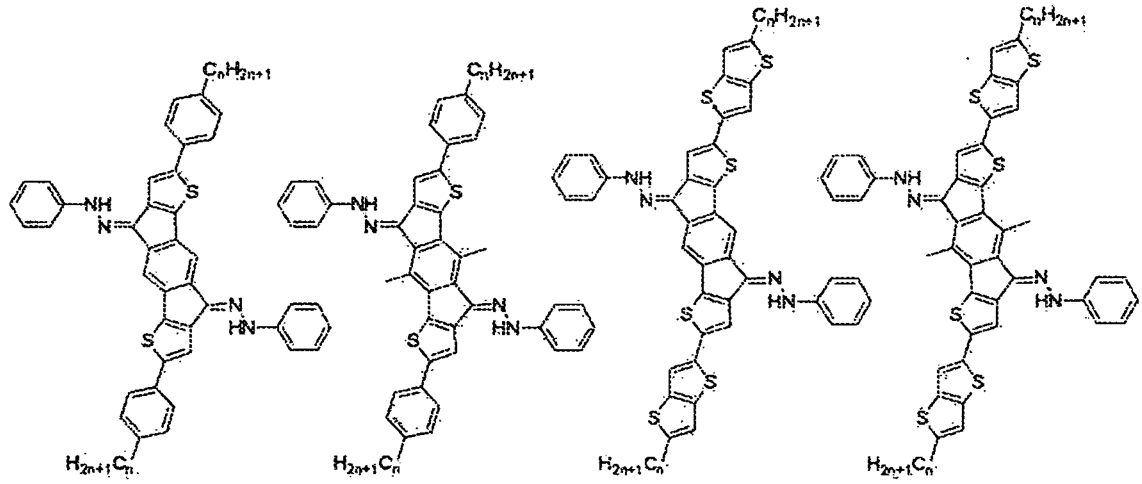

(4-41) (4-42) (4-43) (4-44)

[Formula 22]

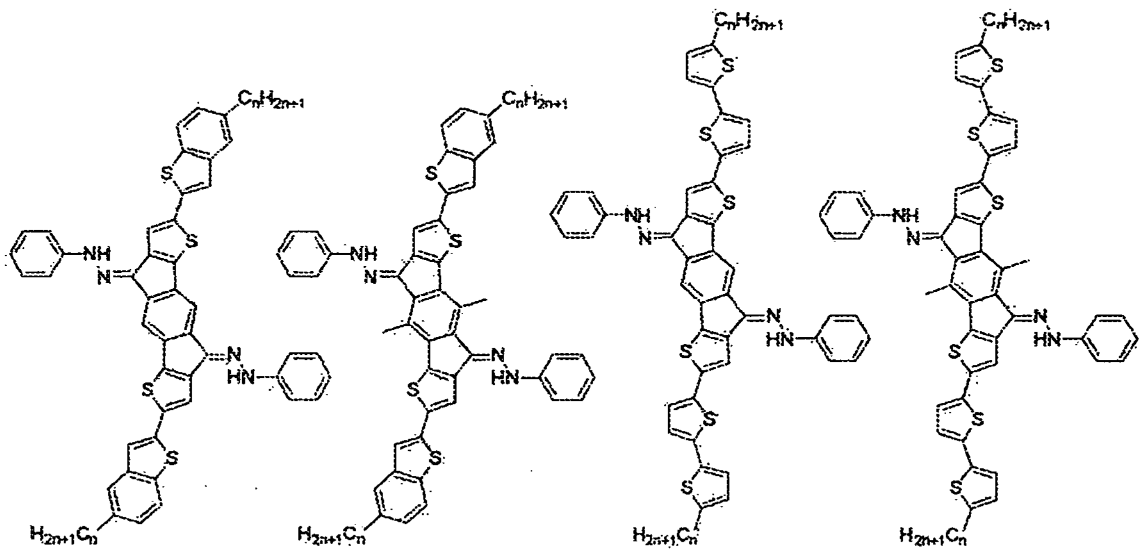

(4-45) (4-46) (4-47) (4-48)

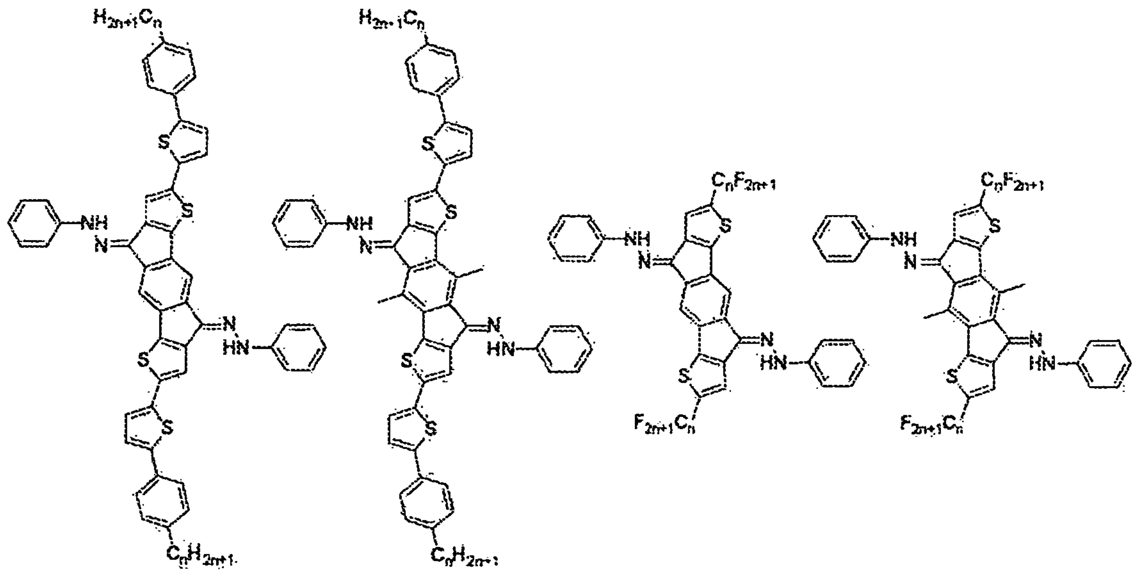

(4-49) (4-50) (4-51) (4-52)

[Formula 23]

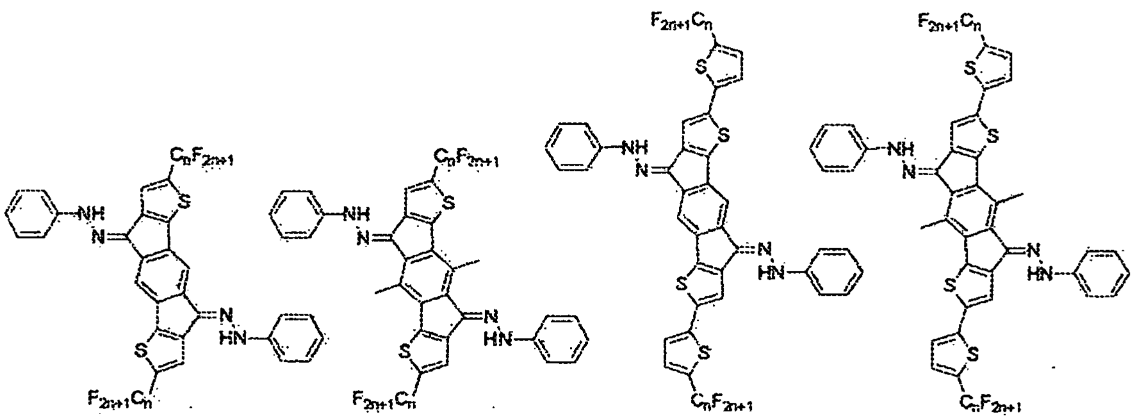

(4-53) (4-54) (4-55) (4-56)

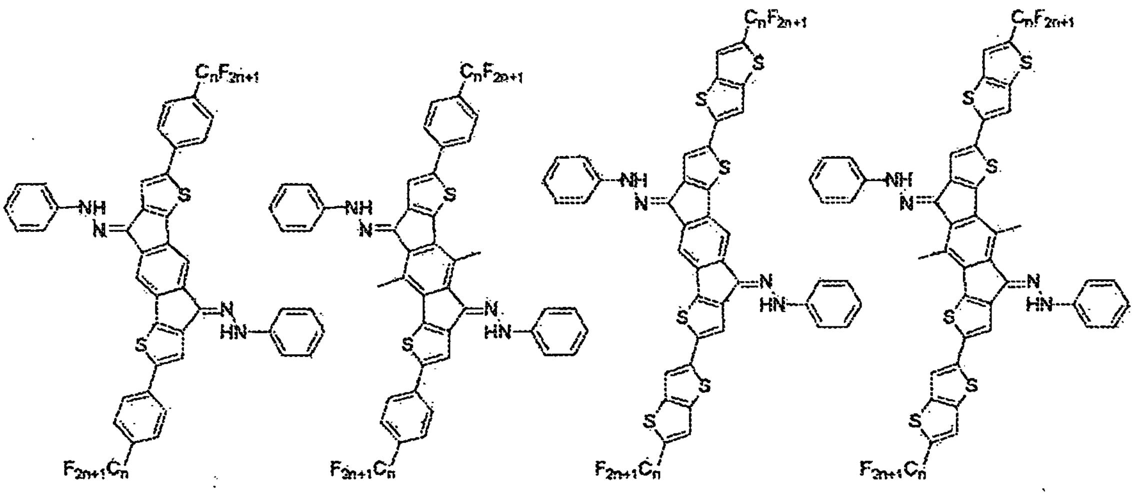

(4-57) (4-58) (4-59) (4-60)

[Formula 24]

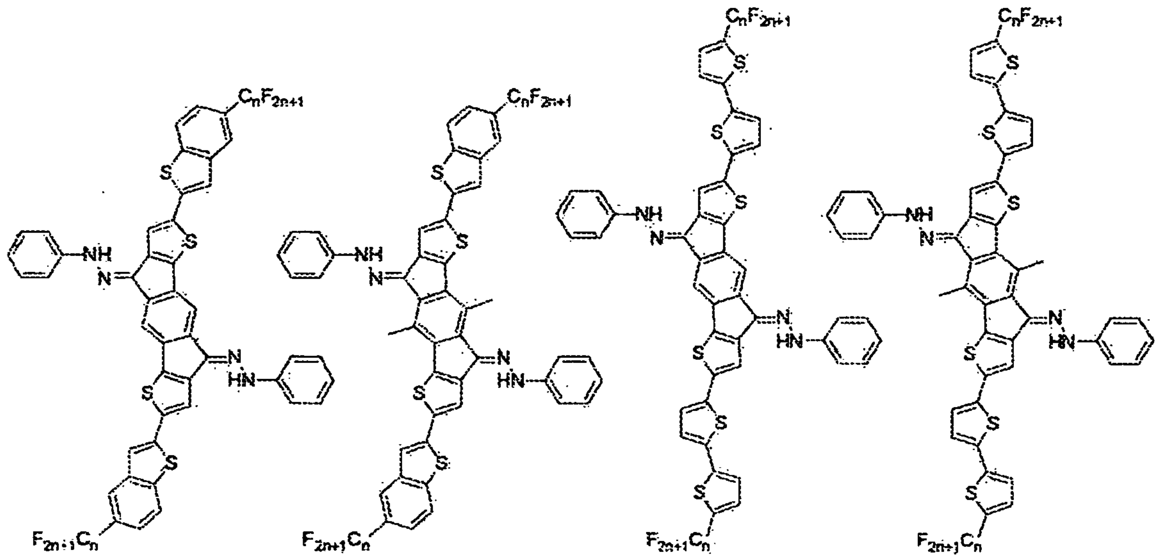

(4-61) (4-62) (4-63) (4-64)

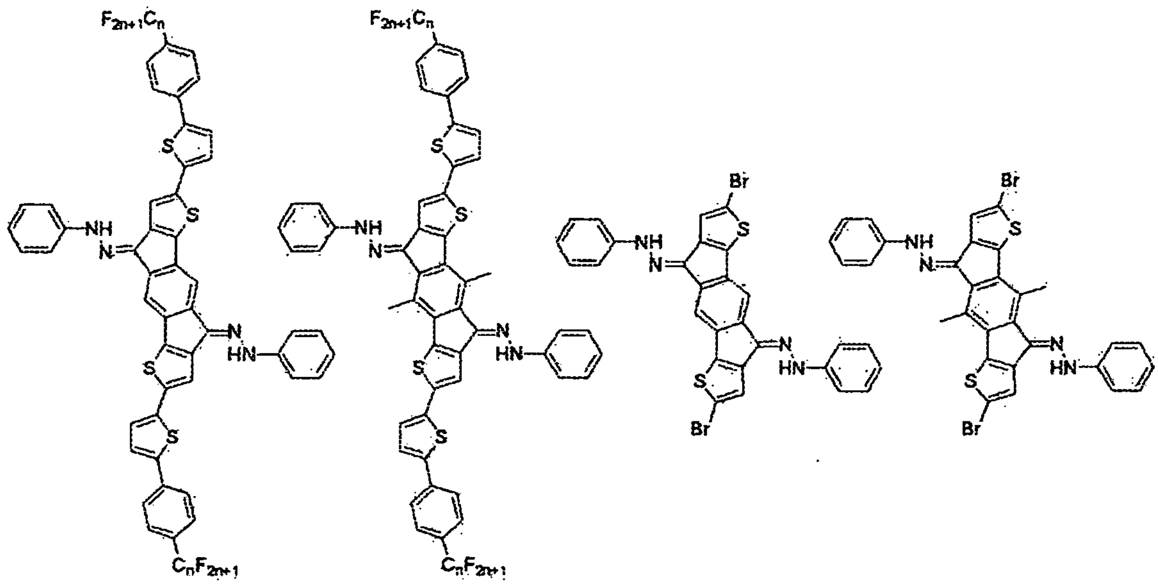

(4-65) (4-66) (4-67) (4-68)

[Formula 25]

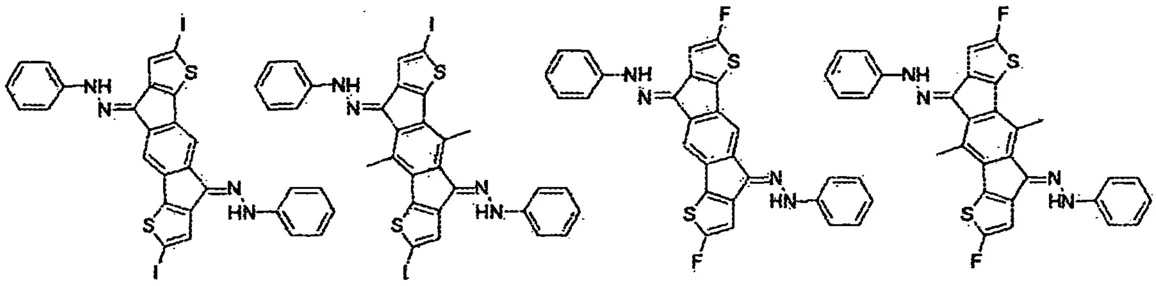

(4-69) (4-70) (4-71) (4-72)

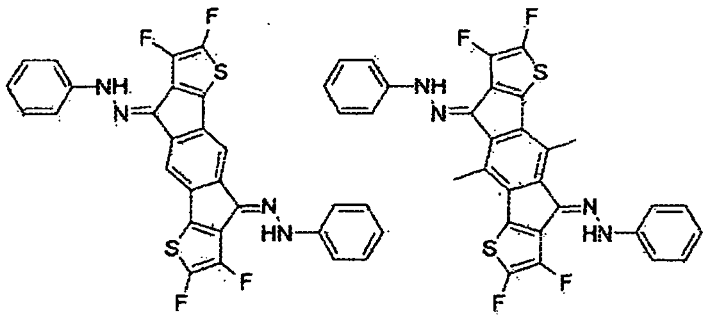

(4-73) (4-74)

The dihydrazone compound (4) preferably includes compounds represented by the formulae (4-1), (4-3), (4-5), (4-7), (4-9), (4-11), (4-13), (4-15), (4-17), (4-19), (4-21), (4-23), (4-25), (4-27), (4-29), (4-31), (4-33), (4-35), (4-37), (4-39), (4-41), (4-43), (4-45), (4-47), (4-49), (4-51), (4-53), (4-55), (4-57), (4-59), (4-61), (4-63), (4-65), (4-67), (4-69), (4-71) and (4-73), and more preferably includes compounds represented by the formulae (4-1), (4-3), (4-5), (4-7), (4-9), (4-11), (4-13), (4-29), (4-31), (4-37), (4-39), (4-41), (4-43), (4-45), (4-47), (4-49), (4-67) and (4-69).

The reaction in which a basic reagent is allowed to act on a dihydrazone compound (4) is commonly carried out in a solvent. Examples of a reaction solvent include water, aliphatic hydrocarbons such as pentane, hexane, heptane, cyclopentane, cyclohexane, decalin, dichloromethane and chloroform, aromatic hydrocarbons such as benzene, toluene, xylene, cumene, ethylbenzene, monochlorobenzene and dichlorobenzene, ethers such as diethyl ether, methyl-t-butyl ether, THF, 1,3-dioxolane, 1,3-dioxane, 1,4-dioxane, diethylene glycol, ethylene glycol, triethylene glycol and propylene glycol, alcohols such as methanol, ethanol, n-propanol, isopropanol, n-butanol and t-butanol, and DMSO. Preferable solvents to be used include water, dichloromethane, chloroform, benzene, toluene, diethyl ether, methyl-t-butyl ether, THF, 1,3-dioxolane, 1,3-dioxane, 1,4-dioxane, diethylene glycol, ethylene glycol, triethylene glycol, propylene glycol, n-butanol, t-butanol and DMSO, and more preferable ones include toluene, 1,4-dioxane, diethylene glycol, ethylene glycol, triethylene glycol, t-butanol and DMSO. These solvents may be used singly or as a mixture of two or more.

Examples of basic reagents to be used in the reaction include potassium hydroxide, sodium hydroxide, potassium carbonate, sodium carbonate, potassium t-butoxide, sodium t-butoxide, sodium, sodium methoxide, sodium ethoxide, potassium methoxide, potassium ethoxide and lithium hydrogenated aluminum. The basic reagents may be used singly or as a mixture of two or more.

The use amount of a basic reagent is usually 0.5 mole time to 25 mole times, preferably 2 mole times to 20 mole times, and more preferably 5 mole times to 15 mole times, relative to a dihydrazone compound (4). If the use amount of a basic reagent is too small, the proportion of a compound in which only one hydrazone has been converted to methylene increases.

The concentration of a reaction solution of a dihydrazone compound is not especially limited, but is usually in the range from 0.0001 mole to 10 moles, preferably from 0.001 mole to 5 moles, and more preferably from 0.01 mole to 1 mole, relative to 1 L of a solvent.

The concentration of a solution of a basic reagent to be dropped is not especially limited, but is usually in the range from 0.001 mole to 20 moles, preferably from 0.01 mole to 10 moles, and more preferably from 0.1 mole to 5 mole, relative to 1 L of a solvent.

The reaction of a basic reagent and a dihydrazone compound can usually be carried out by mixing a solution

of the basic reagent and a solution of the dihydrazone compound. The mixing may be carried out by mixing up both the solutions at one time, but it is preferable from the viewpoint of control of the reaction that the mixing is carried out by dropping one of the solutions in the other solution. The dropping time is not especially limited, but it is preferable that the dropping be carried out in the range where the rise in the internal temperature due to the reaction heat can be controlled.

The temperature during the dropping in the reaction of a basic reagent and a dihydrazone compound is usually in the range from -78°C to a boiling point of a solvent, preferably from 0°C to the boiling point of the solvent, and more preferably from 50°C to the boiling point of the solvent.

The temperature after the completion of the dropping is not especially limited, but the reaction may be carried out while a temperature at dropping is being held, or the reaction may be carried out by raising the temperature to the boiling point of the solvent. The reaction time is not especially limited, but is usually 1 min to 72 hours.

In the case of stopping the reaction, for example, water, dilute hydrochloric acid or the like is added to the reaction solution. After the stopping of the reaction, by carrying out a usual post-treatment, an operation such as extraction or washing, a crude product of a dihydroindacene compound can be obtained. The crude product of the dihydroindacene compound may be refined by an operation such as crystallization, sublimation, and various types of chromatographies.

Other production methods of a dihydroindacene compound in which $R^6$ and $R^8$ are not hydrogen in the formula (3) include a cross-coupling production method using a dihydroindacene compound in which $R^6$ and $R^8$ are bromine or iodine. For example, as described in "Organic Letters" (the U.S., 2005, vol. 7, No. 5, pp. 795-797), a dihydroindacene compound can be produced by using dibromoindenofluorene and carrying out the Suzuki coupling reaction.

As production methods of a dihydroindacene compound in which $R^2$ and $R^5$ are not hydrogen in the formula (3), various types of methods are used. For example, as described in "Journal of the American Chemical Society" (the U.S., 2005, vol. 127, pp. 11763-11768), a dihydroindacene compound in which $R^2$ to $R^5$ are alkyl groups can also be produced by allowing a halogen alkyl to act on a dihydroindacene compound in which $R^2$ to $R^5$ are hydrogen in the presence of a basic reagent.

A dihydrazone compound (4) can be obtained by allowing a hydrazine reagent to act on an indacenedione compound (hereinafter, referred to as an indacenedione compound (5)) represented by the following formula (5).

[Formula 26]

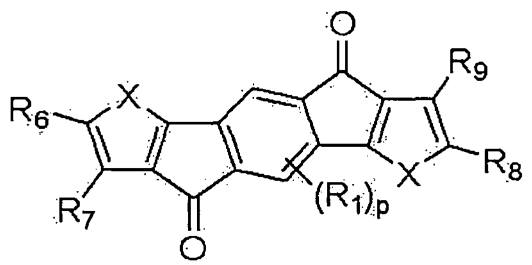

(5)

In the formula (5), $R^1$, p, $R^6$ to $R^9$ and X are each the same as described in the representations of R1, p, $R^6$ to $R^9$ and X in the formula (3);
provided that the case where X = S, $R^1$ = H, p = 2, and $R^7$ = $R^9$ = hexyl, and the case where X = S, $R^1$ = octyl, p = 2, and $R^6$ to $R^9$ = H are excluded.

$R^1$, p and X in the formula (5) include the same as described in the representations of $R^1$, p and X in the formula (3), and are each preferably the same one.

$R^6$ to $R^9$ include the same as described in the representation of $R^6$ to $R^9$ in the formula (3), and are each preferably the same one.

The group of preferable compounds in the indacenedione compound of the formula (5) includes the following.

- An indacenedione compound in which $R^6$ and $R^8$ are identical or different from each other, and are each alkyl which has 1 to 30 carbon atoms and may be substituted, aryl which has 6 to 30 carbon atoms and may be substituted, heteroaryl which may be substituted, or a halogen.
- An indacenedione compound in which $R^1$, $R^7$ and $R^9$ are identical or different from each other, and are each a hydrogen atom, methyl or ethyl.
- An indacenedione compound of the formula (5) in which X is a sulfur atom; $R^1$, $R^7$ and $R^9$ are each a hydrogen atom; p = 2; and $R^6$ and $R^8$ are each an alkyl group having 1 to 20 carbon atoms, or an alkoxy group having 1 to 20 carbon atoms.

- An indacenedione compound of the formula (5) in which X is a sulfur atom; $R^1$, $R^7$ and $R^9$ are each a hydrogen atom; p = 2; and $R^6$ and $R^8$ are each 5-(C1-20 alkyl)thiophen-2-yl.
- An indacenedione compound of the formula (5) in which X is a sulfur atom; $R^1$, $R^7$ and $R^9$, are each a hydrogen atom; p is 2; and $R^6$ and $R^8$ are each 4-(C1-20 alkyl)phenyl-1-yl, or 4-(C1-20 alkoxy)phenyl-1-yl.
- An indacenedione compound of the formula (5) in which X = S.
- An indacenedione compound of the formula (5) in which $R^7 = R^9 = H$, p = 2, and $R^6 = R^8$ = n-hexyl.
- An indacenedione compound of the formula (5) in which X = S; $R^1 = R^7 = R^9 = H$; p = 2; and $R^6 = R^8$ = 5-n-hexylthiophen-2-yl.
- An indacenedione compound of the formula (5) in which X = S; $R^1 = R^7 = R^9 = H$; p = 2; and $R^6 = R^8$ = 4-n-hexylphenyl.

The indacenedione compound represented by the formula (5) specifically includes compounds represented by the following formulae (5-1) to (5-36), but the compounds are not limited thereto. In the formulae, n is identical or different from each other, and each denotes an integer of 0 to 30.

[Formula 27]

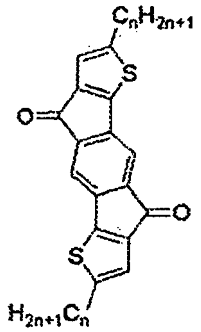

(5-1)

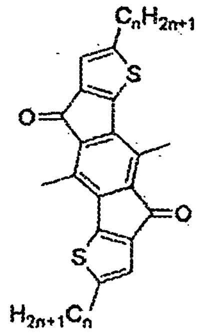

(5-2)

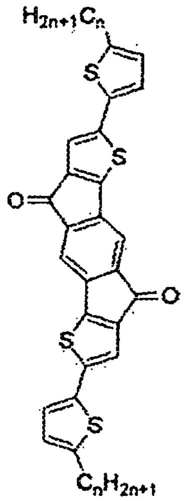

(5-3)

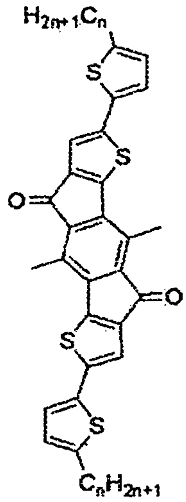

(5-4)

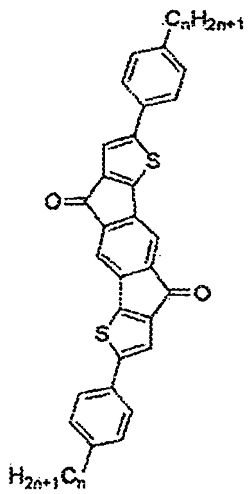

(5-5)

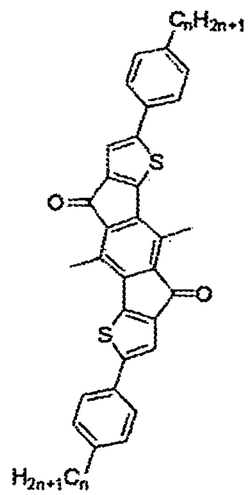

(5-6)

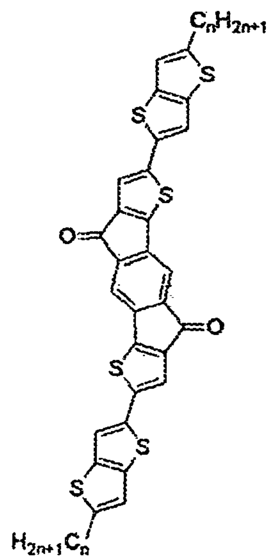

(5-7)

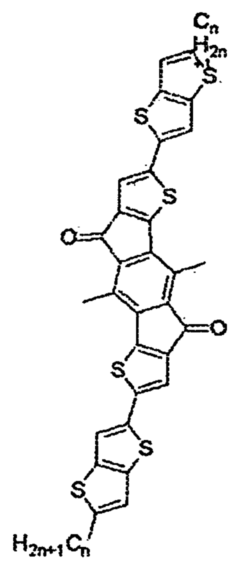

(5-8)

[Formula 28]

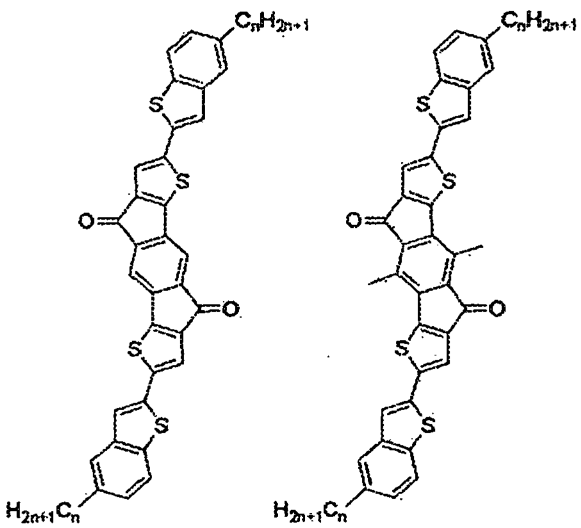

(5-9) (5-10)

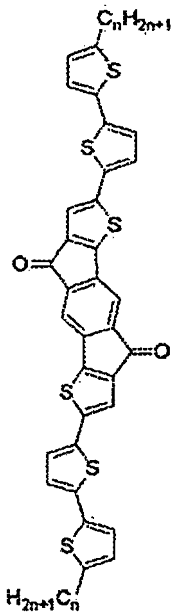

(5-11)

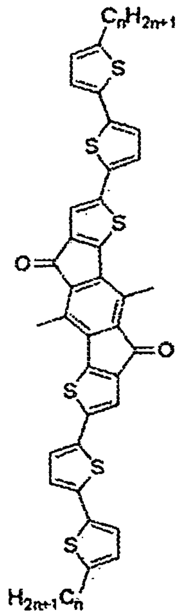

(5-12)

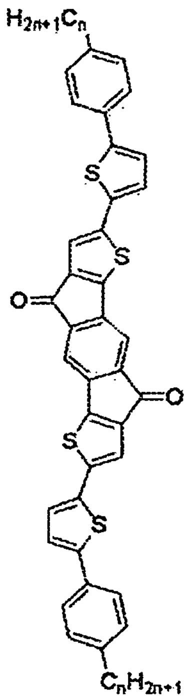

(5-13)

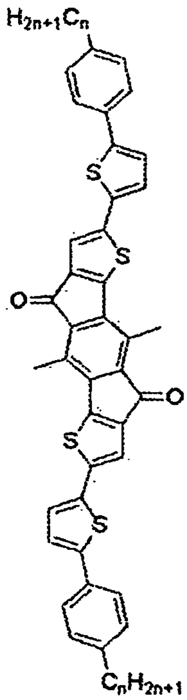

(5-14)

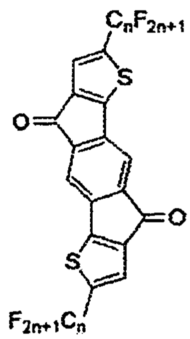

(5-15)

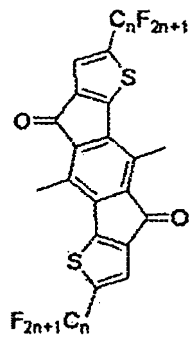

(5-16)

[Formula 29]

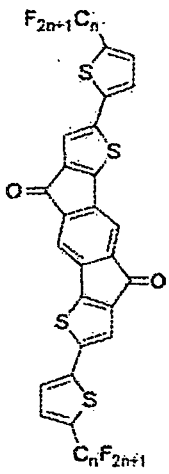
(5-17)

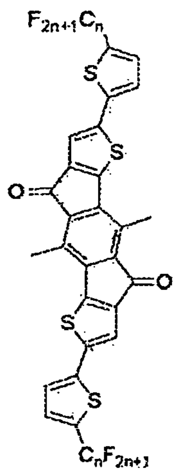
(5-18)

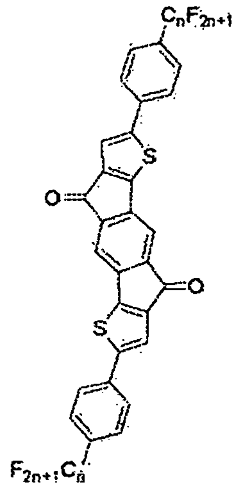
(5-19)

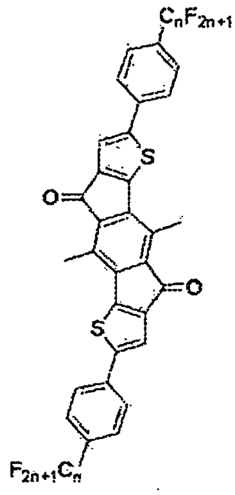
(5-20)

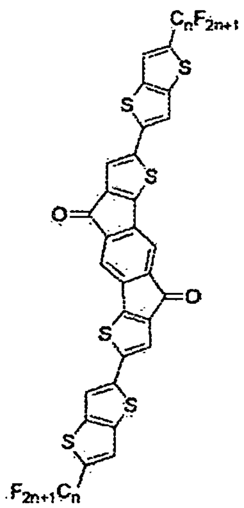
(5-21)

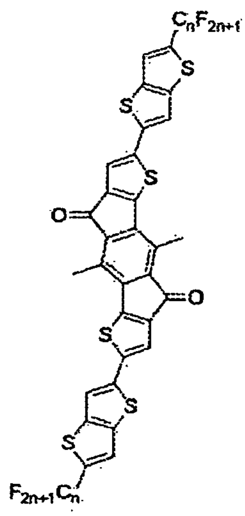
(5-22)

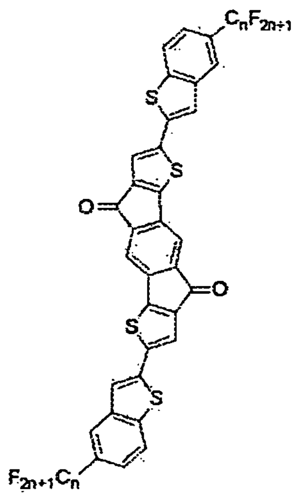
(5-23)

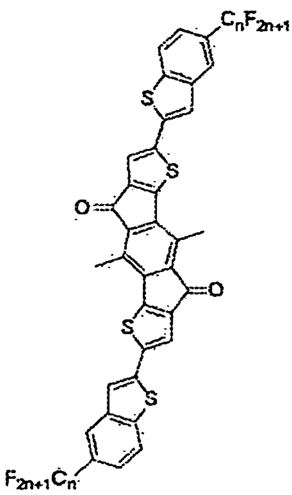
(5-24)

[Formula 30]

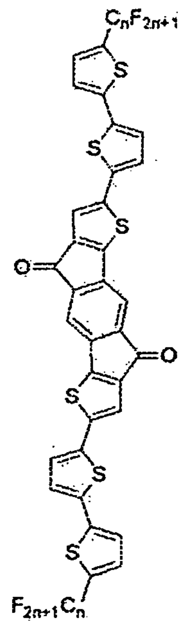

(5-25)

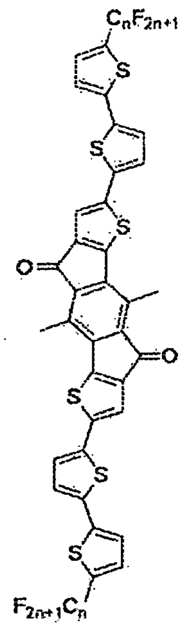

(5-26)

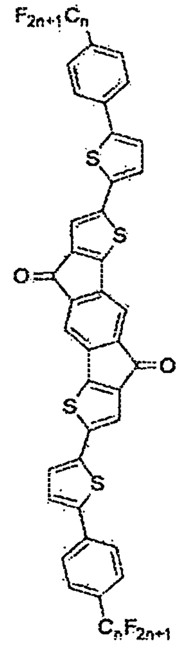

(5-27)

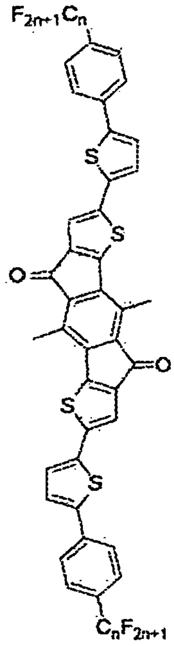

(5-28)

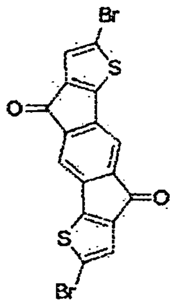

(5-29)

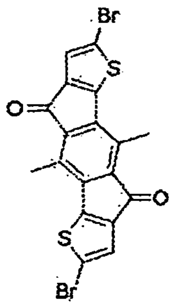

(5-30)

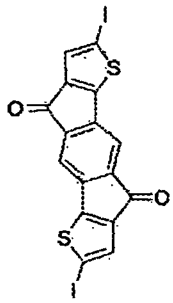

(5-31)

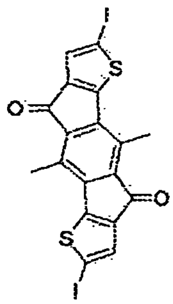

(5-32)

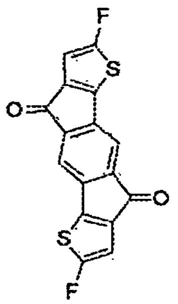

(5-33)

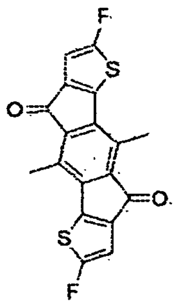

(5-34)

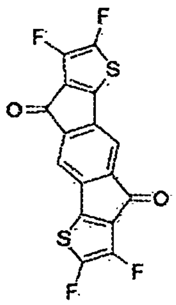

(5-35)

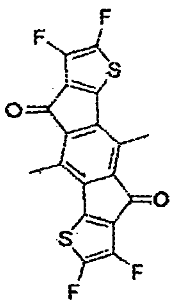

(5-36)

The indacenedione compound (5) preferably includes compounds represented by the formulae (5-1), (5-3), (5-5), (5-7), (5-9), (5-11), (5-13), (5-15), (5-17), (5-19), (5-21), (5-23), (5-25), (5-27), (5-29), (5-31), (5-33) and (5-35), and more preferably includes compounds represented by the formulae (5-1), (5-3), (5-5), (5-7), (5-9), (5-11), (5-13), (5-15), (5-17), (5-19), (5-21), (5-23), (5-25), (5-27), (5-29) and (5-31).

The reaction condition of the hydrazonation reaction of an indacenedione compound (5) is not especially limited, but an indacenedione compound can be obtained, for example, as described in "Organic Letters" (the U.S., 2001, vol. 3, No. 23, pp. 3647-3650), by stirring a ketone compound and phenylhydrazine in acetic acid. The prepared hydrazone compound may be used for a following reaction after being isolated, or may be used as it is as described in "Organic Letters" (the U.S., 2002, vol. 4, No. 13, pp. 2157-2159).

Examples of reaction solvents include water, aliphatic hydrocarbons such as pentane, hexane, heptane, cyclopentane, cyclohexane, decalin, dichloromethane and chloroform, aromatic hydrocarbons such as benzene, toluene, xylene, cumene, ethylbenzene, monochlorobenzene and dichlorobenzene, ethers such as diethyl ether, methyl-t-butyl ether, THF, 1,3-dioxolane, 1,3-dioxane, 1,4-dioxane, diethylene glycol, ethylene glycol, triethylene glycol and propylene glycol, alcohols such as methanol, ethanol, propanol, n-butanol and t-butanol, and DMSO.

Preferable solvents to be used include dichloromethane, chloroform, benzene, toluene, diethyl ether, methyl-t-butyl ether, THF, 1,3-dioxolane, 1,3-dioxane, 1,4-dioxane, diethylene glycol, ethylene glycol, triethylene glycol, propylene glycol, n-butanol, t-butanol and DMSO, and more preferable ones include benzene, toluene, THF, 1,4-dioxane, diethylene glycol, ethylene glycol, triethylene glycol, n-butanol, t-butanol and DMSO. These solvents may be used singly or as a mixture of two or more.

Hydrazines to be used in the reaction include hydrazine, hydrazine monohydrate, hydrazine hydrate, aryl-substituted hydrazines such as phenylhydrazine and tosylhydrazine, and N-t-butyldimethylsilylhydrazine.

The use amount of a hydrazine is usually 1.5 mole times to 100 mole times, preferably 2 mole times to 50 mole times, and more preferably 2 mole times to 25 mole times, relative to an indacenedione compound (5). If the use amount of a hydrazine is too small, the proportion of a compound in which only one ketone is hydrazonated increases.

The reaction temperature in the reaction of a hydrazine and an indacenedione compound (5) is usually in the range from -78°C to a boiling point of a solvent, preferably from 0°C to the boiling point of the solvent, and more preferably from 50°C to the boiling point of the solvent. The reaction time is not especially limited, but is usually 1 min to 24 hours.

In the case of stopping the reaction, for example, water, a weakly acidic aqueous solution or the like is added to the reaction solution. After the stopping of the reaction, by carrying out a usual post-treatment, an operation such as extraction or washing, a crude product of a dihydrazone compound can be obtained. For the reaction in the following denitrification procedure, the crude product of the dihydrazone compound may be supplied, or after the crude dihydrazone compound is refined by an operation such as crystallization, and various types of chromatographies, the refined dihydrazone compound may be supplied for the denitrification process.

An indacenedione compound (5) can be obtained by allowing an acidic reagent to act on a terephthalic acid compound (hereinafter, referred to as a terephthalic acid compound) represented by the following formula (6).

[Formula 31]

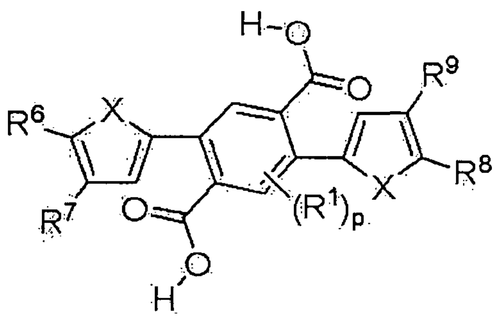

(6)

In the formula (6), $R^1$, P, $R^6$ to $R^9$ and X are each the same as described in the representations of $R^1$, P, $R^6$ to $R^9$ and X in the formula (3);
provided that the case where $R^1$ = H, p = 2, and $R^7$ = $R^9$ = n-hexyl, $R^6$ = $R^8$ = n-hexyl or hydrogen, and X = S is excluded.

Substituents of the terephthalic acid compound (6) will be described further.

In the formula (6), a group of compounds is preferable which have the same substituents as in a group of compounds indicated as the group of preferable compounds with respect to the formula (3).

The group of preferable compounds in the terephthalic acid compound (6) includes the following.

- A terephthalic acid compound represented by the formula (6) in which $R^6$ and $R^8$ are identical or different from each other, and are each alkyl which has 1 to 30 carbon atoms and may be substituted, aryl which has 6 to 30 carbon atoms and may be substituted, heteroaryl which may be substituted, or a halogen.
- A terephthalic acid compound represented by the formula (6) in which $R^1$, $R^7$ and $R^9$ are identical or different from each other, and are each a hydrogen atom, methyl or ethyl.
- A terephthalic acid compound of the formula (6) in which X is a sulfur atom; $R^1$, $R^7$ and $R^9$ are each a hydrogen atom; p = 2; and $R^6$ and $R^8$ are each an alkyl group having 1 to 20 carbon atoms, or an alkoxy group having 1 to 20 carbon atoms.
- A terephthalic acid compound of the formula (6) in which X is a sulfur atom; $R^1$, $R^7$ and $R^9$ are each a hydrogen

atom; p = 2; and $R^6$ and $R^8$ are each 5-(C1-20 alkyl)thiophen-2-yl.

- A terephthalic acid compound of the formula (6) in which X is a sulfur atom; $R^1$, $R^7$ and $R^9$ are each a hydrogen atom; p = 2; and $R^6$ and $R^8$ are each 4-(C1-20 alkyl)phenyl-1-yl, or 4-(C1-20 alkoxy)phenyl-1-yl.
- A terephthalic acid compound represented by the formula (6) in which X is a sulfur atom.
- A terephthalic acid compound represented by the formula (6) in which X is a sulfur atom; $R^1$, $R^7$ and $R^9$ are each a hydrogen atom; p = 2; and $R^6$ and $R^8$ are each n-hexyl.
- A terephthalic acid compound represented by the formula (6) in which X is a sulfur atom; $R^1$, $R^7$ and $R^9$ are each a hydrogen atom; p = 2; and $R^6$ and $R^8$ are each 5-n-hexylthiophen-2-yl.
- A terephthalic acid compound represented by the formula (6) in which X is a sulfur atom; $R^1$, $R^7$ and $R^9$ are each a hydrogen atom; p is 2; and $R^6$ and $R^8$ are each 4-n-hexylphenyl.

The terephthalic acid compound specifically includes compounds represented by the following formulae (6-1) to (6-36), but the compounds are not limited thereto. In the formulae, n is identical or different from each other, and each denotes an integer of 0 to 30.

[Formula 32]

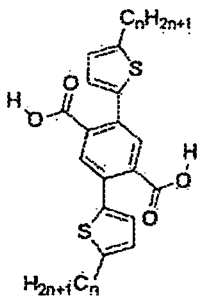

(6-1)

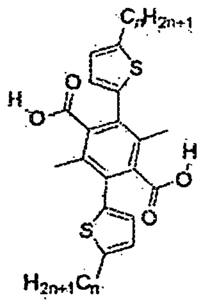

(6-2)

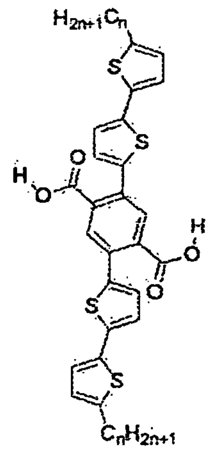

(6-3)

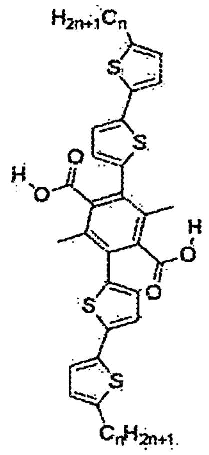

(6-4)

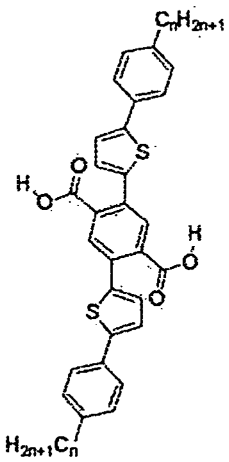

(6-5)

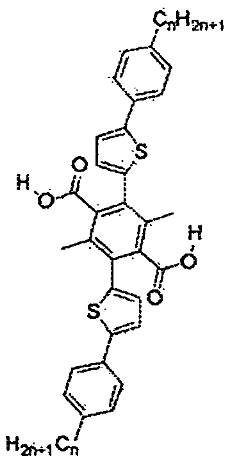

(6-6)

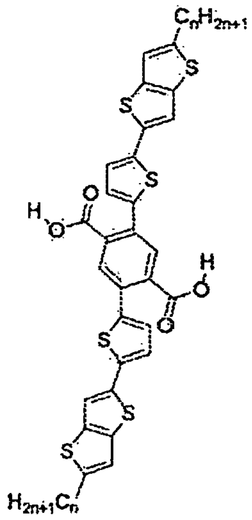

(6-7)

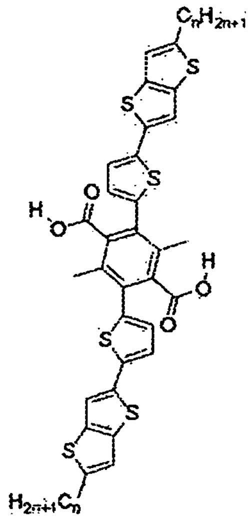

(6-8)

[Formula 33]

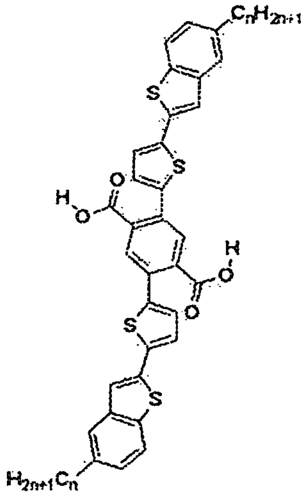

(6-9)

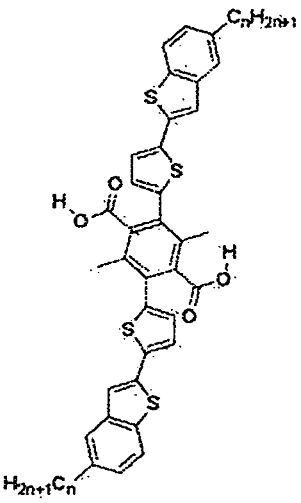

(6-10)

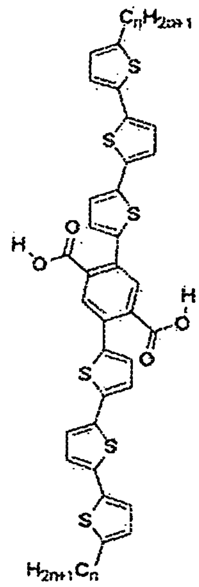

(6-11)

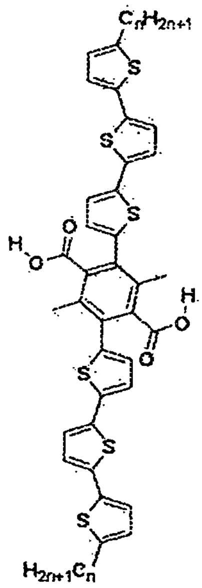

(6-12)

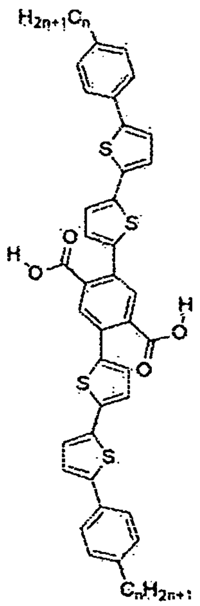

(6-13)

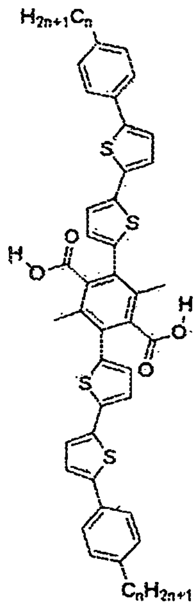

(6-14)

[Formula 34]

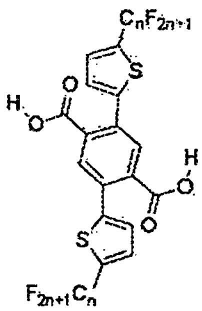

(6-15)

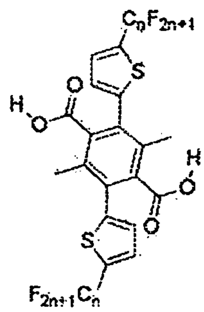

(6-16)

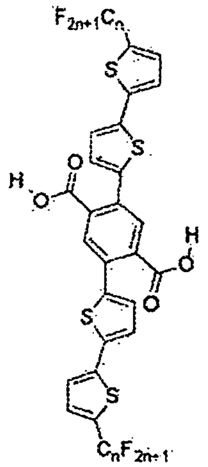

(6-17)

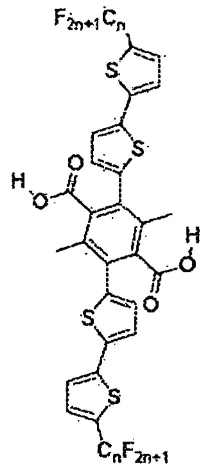

(6-18)

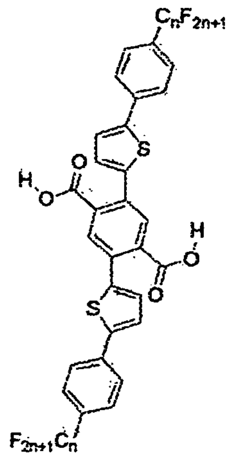

(6-19)

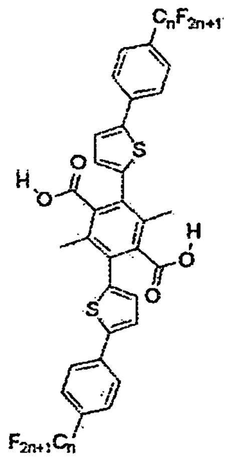

(6-20)

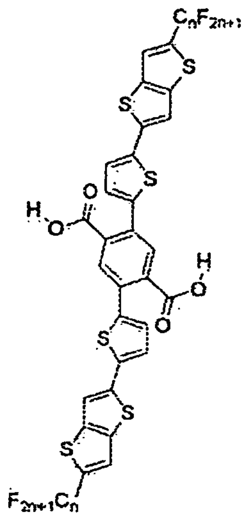

(6-21)

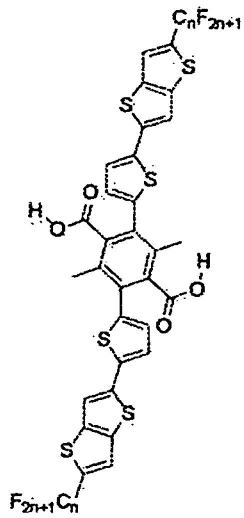

(6-22)

[Formula 35]

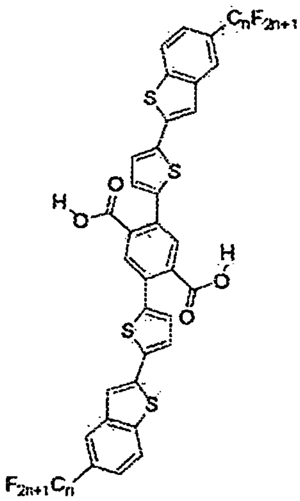

(6-23)

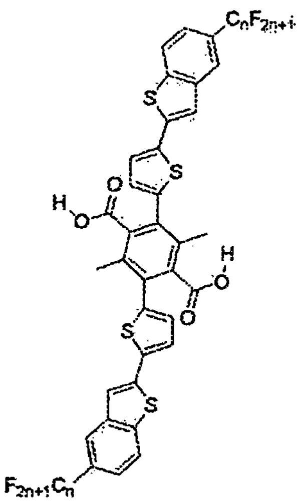

(6-24)

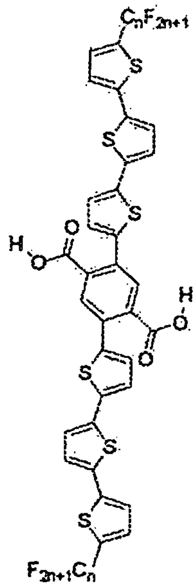

(6-25)

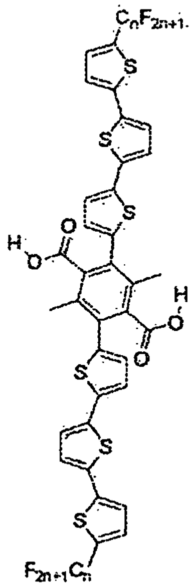

(6-26)

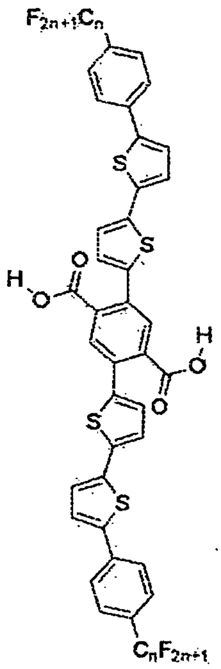

(6-27)

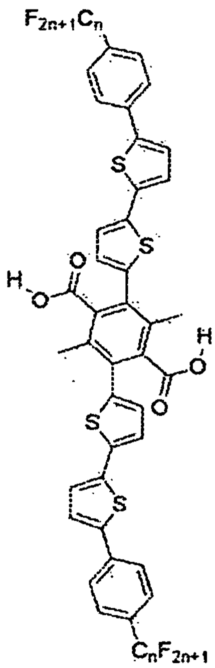

(6-28)

[Formula 36]

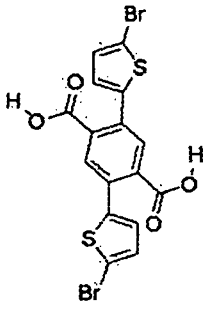

(6-29)

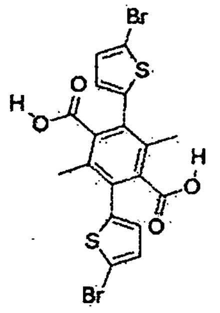

(6-30)

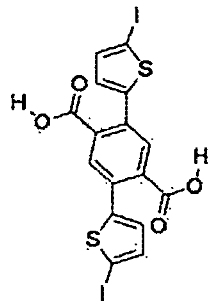

(6-31)

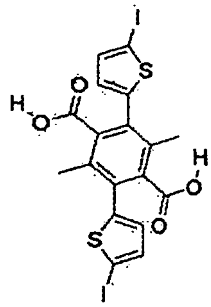

(6-32)

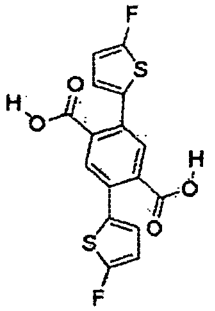

(6-33)

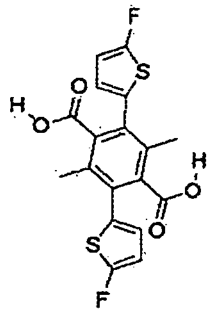

(6-34)

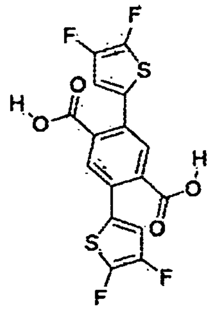

(6-35)

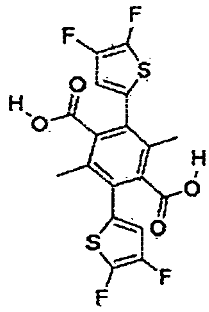

(6-36)

The terephthalic acid compound (6) preferably includes compounds represented by the formulae (6-1), (6-3), (6-5), (6-7), (6-9), (6-11), (6-13), (6-15), (6-17), (6-19), (6-21), (6-23), (6-25), (6-27), (6-29), (6-31), (6-33) and (6-35), and more preferably includes compounds represented by the formulae (6-1), (6-3), (6-5), (6-7), (6-9), (6-11), (6-13), (6-15), (6-17), (6-19), (6-21), (6-23), (6-25), (6-27), (6-29) and (6-31).

The reaction condition of the intramolecular acylation reaction of a terephthalic acid compound (6) is not especially limited, but for example, as described in the above-cited literature "Organic Letters"(the U.S., 2002, vol. 4, No. 13, pp. 2157-2159), an indacenedione compound (5) can be obtained by stirring a terephthalic acid compound in sulfuric acid.

Examples of acidic reagents to be used include zeolite, trifluoromethanesulfonic acid, sulfuric acid, phosphoric acid, polyphosphoric acid, diphosphorus pentaoxide, aluminum trihalides, lanthanide triflates, iron trichloride, zinc dichloride, titanium tetrachloride, tin tetrachloride, bismuth trichloride and mercury dichloride. These reagents may be used singly or as a mixture of two or more.

Preferable solvents to be used include dichloromethane, chloroform, benzene, chlorobenzene, nitromethane, nitrobenzene, 1,2-dichloroethane, toluene, diethyl ether, methyl-t-butyl ether, THF, 1,3-dioxolane, 1,3-dioxane, 1,4-dioxane, diethylene glycol, ethylene glycol, triethylene glycol, propylene glycol, n-butanol, t-butanol and DMSO, and more preferable ones include benzene, chlorobenzene, toluene, THF, 1,4-dioxane, diethylene glycol, ethylene glycol, triethylene glycol, n-butanol, t-butanol and DMSO. These solvents may be used singly or as a mixture of two or more, or an acidic reagent may be used without using any solvent.

The use amount of an acidic reagent is usually 1.5 or more mole times, and preferably 5 or more mole times, relative to a terephthalic acid compound (6). If the use amount of an acidic reagent is too small, the proportion of a compound in which only one acid group is intramolecularly acylated increases. The concentration of a solution of the acidic reagent is not especially limited.

The reaction temperature in the reaction of an acidic reagent and a terephthalic acid compound (6) is usually in the range from -78°C to 250°C, preferably from 0°C to 225°C, and more preferably from 20°C to 200°C. The reaction time is not especially limited, but is usually 1 min to 48 hours.

In the case of stopping the reaction, for example, water, a weakly basic aqueous solution or the like is added to a reaction solution. After the stopping of the reaction, by carrying out a usual post-treatment, an operation such as extraction or washing, a crude product of an indacenedione compound can be obtained. For the following hydrazonation reaction, the crude product of the indacenedione compound may be supplied, or after the crude indacenedione compound is refined by an operation such as crystallization, sublimation, and various types of chromatographies, the refined indacenedione compound may be supplied for the hydrazonation reaction.

A terephthalic acid compound (6) can be obtained by allowing a basic reagent to act on a terephthalate compound

(hereinafter, referred to as a terephthalate compound (7)) represented by the following formula (7).

[Formula 37]

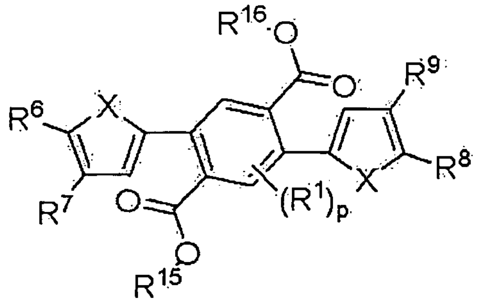

(7)

In the formula (7), $R^1$, P, $R^6$ to $R^9$ and X are each the same as described in the representations of R1, P, $R^6$ to $R^9$ and X in the formula (3); and

$R^{15}$ and $R^{16}$ are identical or different from each other, and each denote alkyl which may be substituted;

provided that the case where $R^1$ = $R^6$ to $R^9$ = H, p = 2, X = S, and $R^{15}$ = $R^{16}$ = methyl or ethyl, and the case where $R^1$ = H, p = 2, X = S, $R^7$ = $R^9$ = n-hexyl, $R^6$ = $R^8$ = n-hexyl, trimethylsilyl or hydrogen, and $R^{15}$ = $R^{16}$ = methyl are excluded.

Substituents of the terephthalate compound (7) will be described further.

In the terephthalate compound of the formula (7), a group of compounds is preferable which have the same substituents as in a group of compounds indicated as the group of preferable compounds with respect to the formula (3).

"Alkyl" of "alkyl which may be substituted" in $R^{15}$ and $R^{16}$ includes the same substituents as shown in $R^1$ described before.

The group of preferable compounds in the terephthalate compound of the formula (7) includes the following.

- A terephthalate compound of the formula (7) in which:

  $R^{15}$ and $R^{16}$ are identical or different from each other, and each denote alkyl which has 1 to 30 carbon atoms and may be substituted.

- A terephthalate compound of the formula (7) in which:

  $R^6$ and $R^8$ are identical or different from each other, and are each alkyl which has 1 to 30 carbon atoms and may be substituted, aryl which has 6 to 30 carbon atoms and may be substituted, heteroaryl which may be substituted, or a halogen.

- A terephthalate compound of the formula (7) in which:

  $R^1$, $R^7$ and $R^9$ are identical or different from each other, and are each a hydrogen atom, methyl or ethyl.

- A terephthalate compound of the formula (7) in which X is a sulfur atom; $R^1$, $R^7$ and $R^9$ are each a hydrogen atom; p = 2; and $R^6$ and $R^8$ are each an alkyl group having 1 to 20 carbon atoms, or an alkoxy group having 1 to 20 carbon atoms.
- A terephthalate compound of the formula (7) in which X is a sulfur atom; $R^1$, $R^7$ and $R^9$ are each a hydrogen atom; p is 2; and $R^6$ and $R^8$ are each 5-(C1-20 alkyl)thiophen-2-yl.
- A terephthalate compound of the formula (7) in which X is a sulfur atom; $R^1$, $R^7$ and $R^9$ are each a hydrogen atom; p is 2; and $R^6$ and $R^8$ are each 4-(C1-20 alkyl)phenyl-1-yl, or 4-(C1-20 alkoxy)phenyl-1-yl.
- A terephthalate compound of the formula (7) in which X = S.
- A terephthalate compound of the formula (7) in which X denotes a sulfur atom; $R^1$, $R^7$ and $R^9$ each denote a hydrogen atom; p is 2; $R^6$ and $R^8$ are each n-hexyl; and $R^{15}$ and $R^{16}$ are each methyl.
- A terephthalate compound of the formula (7) in which X denotes a sulfur atom; $R^1$, $R^7$ and $R^9$ each denote a hydrogen atom; p denotes 2; $R^6$ and $R^8$ are each 5-n-hexylthiophene-2-yl; and $R^{15}$ and $R^{16}$ are each methyl.

- A terephthalate compound of the formula (7) in which X denotes a sulfur atom; $R^1$, $R^7$ and $R^9$ each denote a hydrogen atom; p is 2; $R^6$ and $R^8$ are each 4-n-hexylphenyl; and $R^{15}$ and $R^{16}$ are each methyl.

The terephthalate compound (7) specifically includes compounds represented by the following formulae (7-1) to (7-36), but the compounds are not limited thereto. In the formulae, n is identical or different from each other, and each denotes an integer of 0 to 30.

[Formula 38]

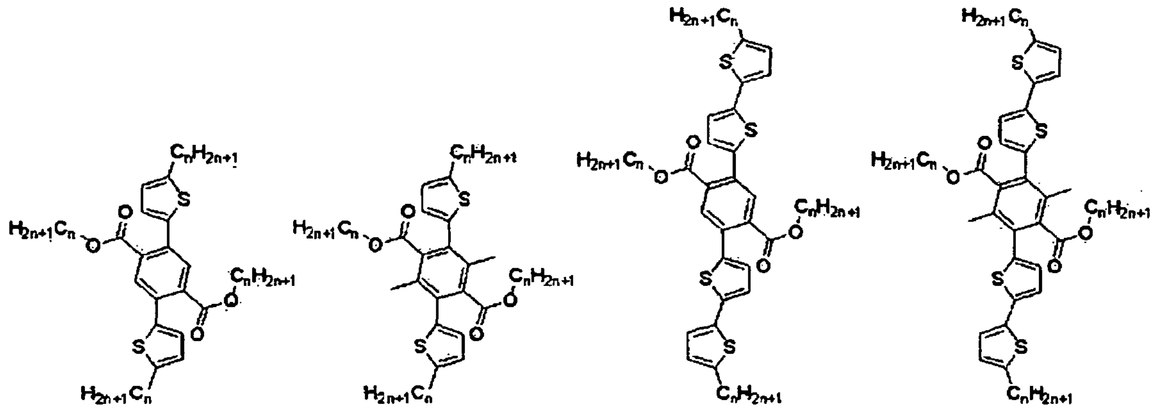

(7-1) (7-2) (7-3) (7-4)

[Formula 39]

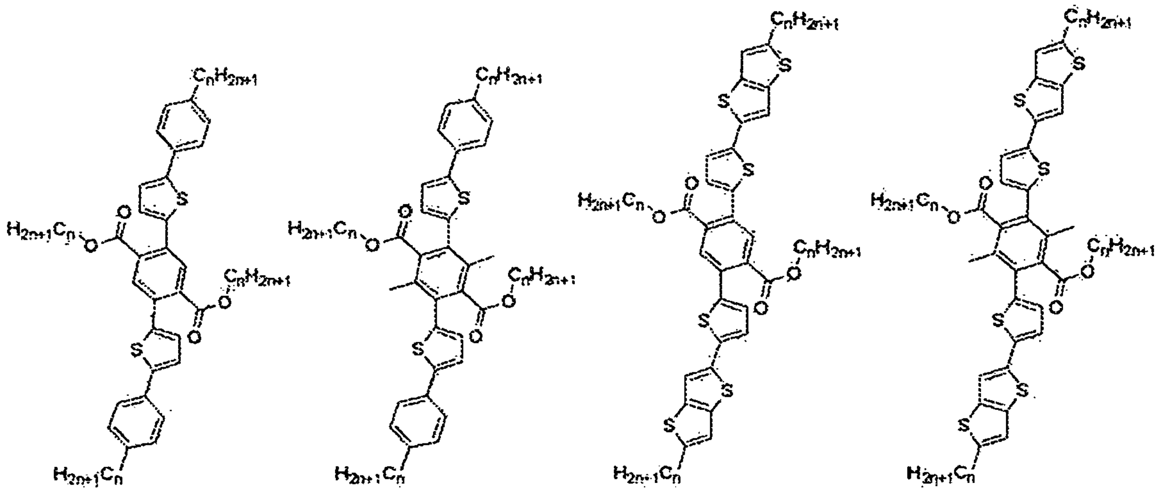

(7-5) (7-6) (7-7) (7-8)

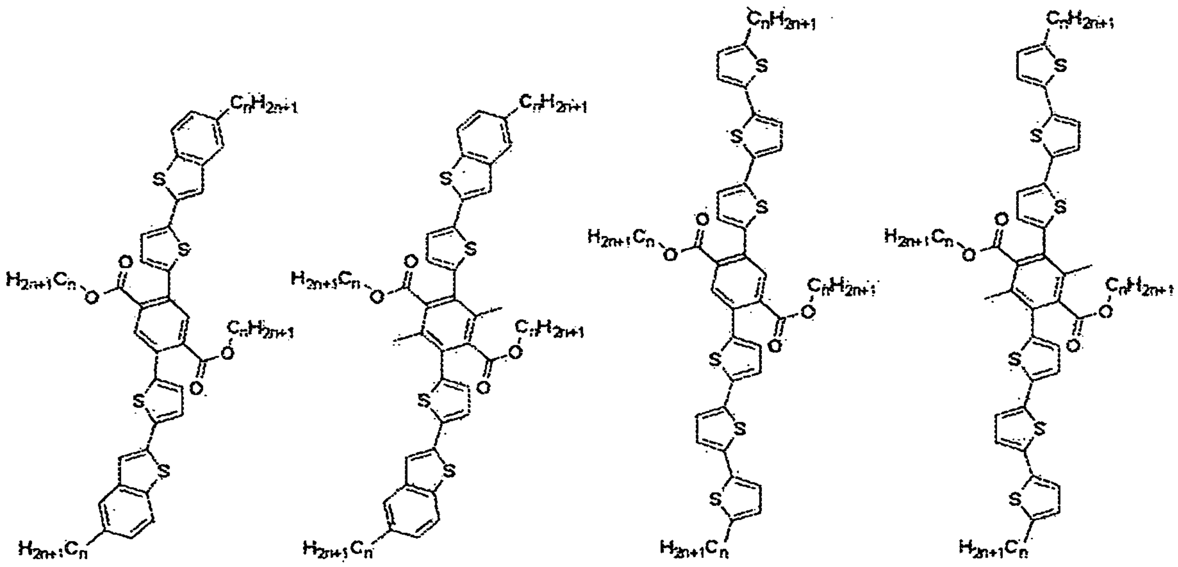

(7-9) (7-10) (7-11) (7-12)

[Formula 40]

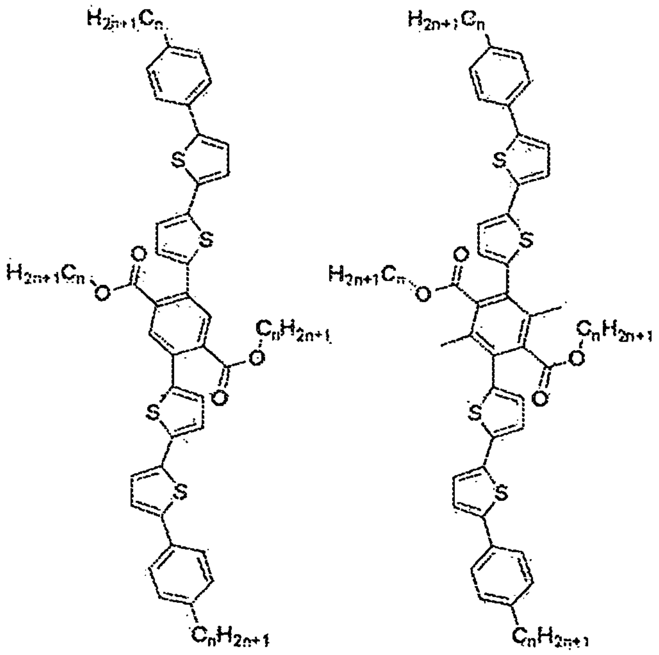

(7-13) (7-14)

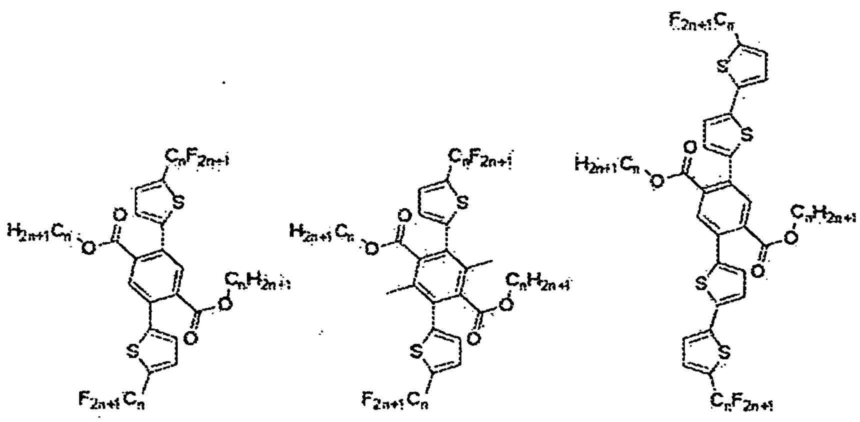

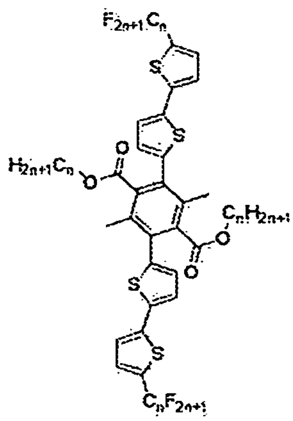

(7-15) (7-16) (7-17) (7-18)

[Formula 41]

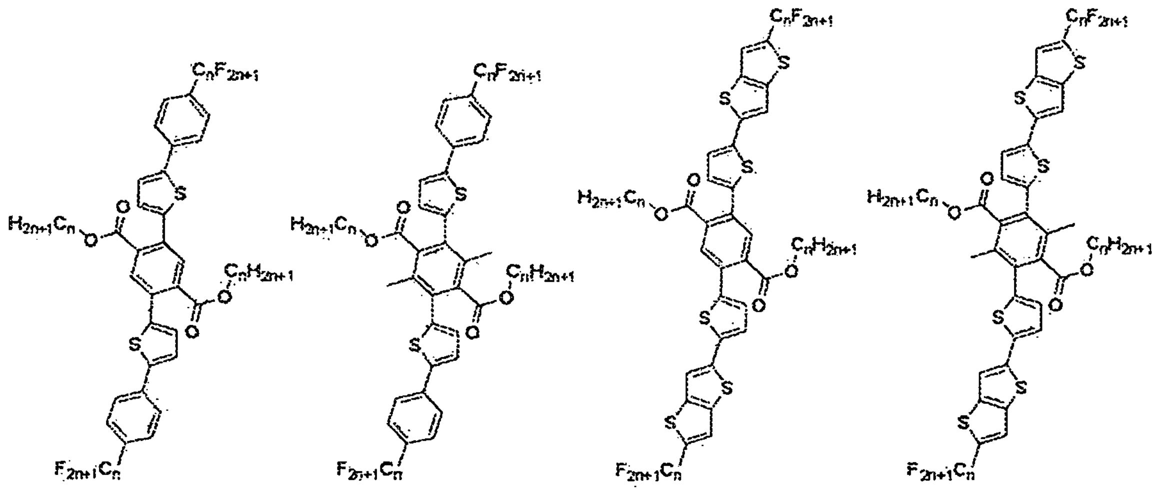

(7-19) (7-20) (7-21) (7-22)

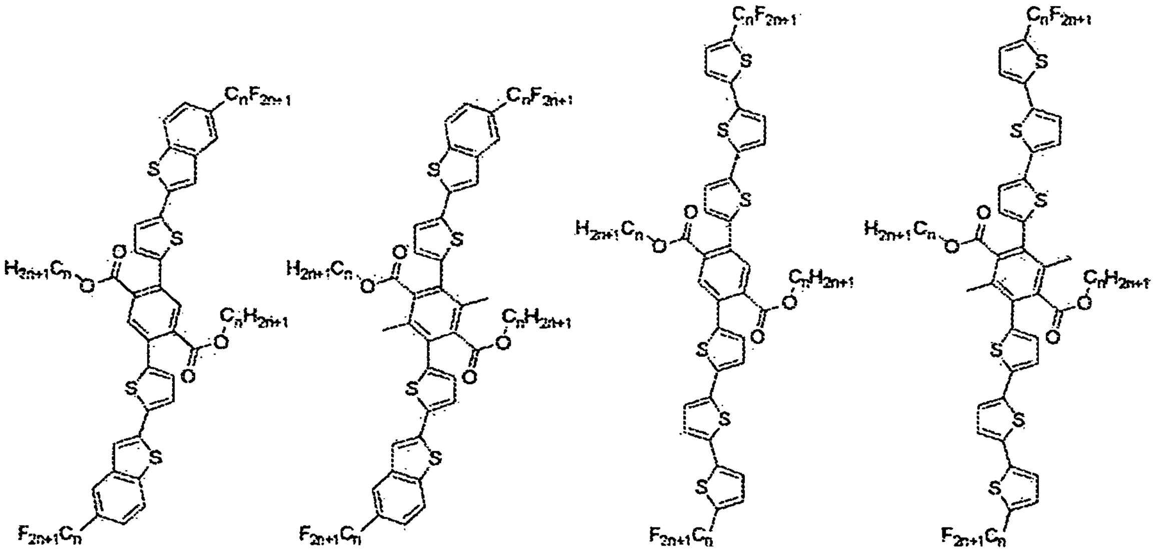

(7-23) (7-24) (7-25) (7-26)

[Formula 42]

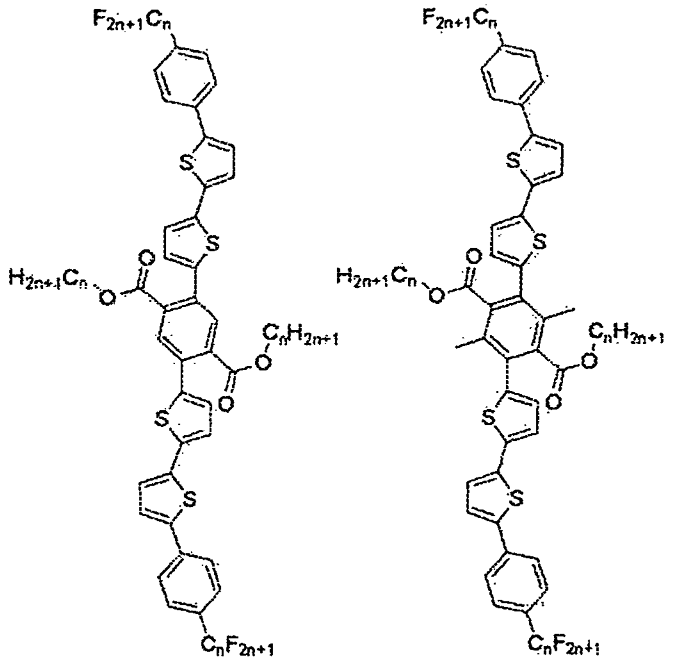

(7-27) (7-28)

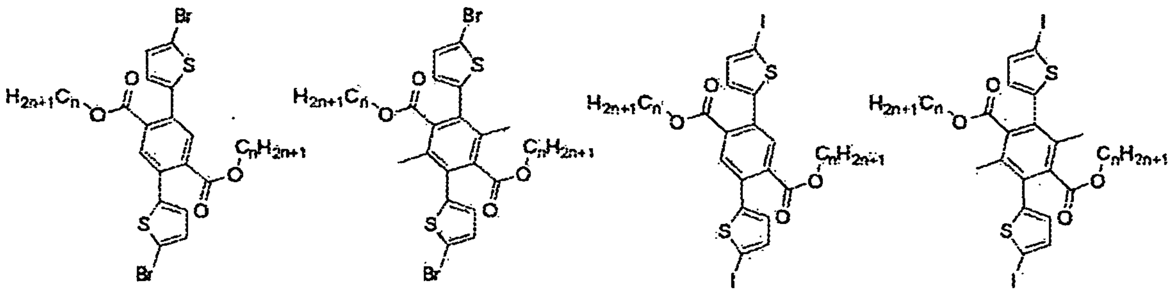

(7-29) (7-30) (7-31) (7-32)

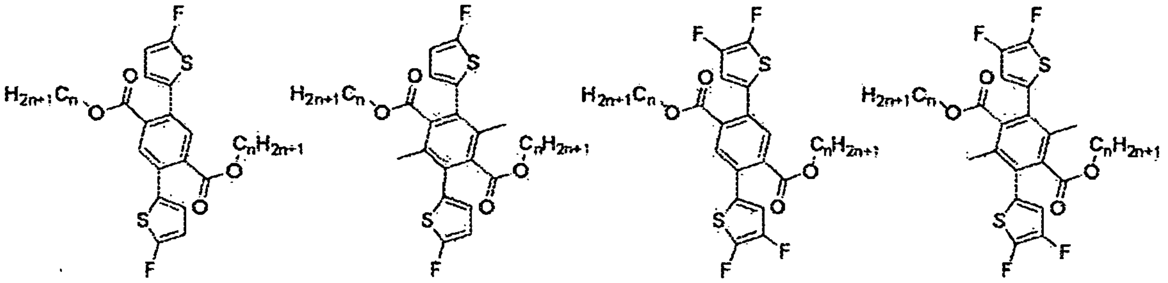

(7-33) (7-34) (7-35) (7-36)

The terephthalate compound (7) preferably includes compounds represented by the formulae (7-1), (7-3), (7-5), (7-7), (7-9), (7-11), (7-13), (7-15), (7-17), (7-19), (7-21), (7-23), (7-25), (7-27), (7-29), (7-31), (7-33) and (7-35), and more preferably includes compounds represented by the formulae (7-1), (7-3), (7-5), (7-7), (7-9), (7-11), (7-13), (7-15), (7-17), (7-19), (7-21), (7-23), (7-25), (7-27), (7-29) and (7-31).

The reaction condition of the hydrolysis reaction of a terephthalate compound (7) is not especially limited, but for example, as described in "Journal of the Organic Chemistry" (the U.S., 2007, vol. 72, No. 17, pp. 6364-6371), a terephthalic acid compound (6) can be obtained by stirring a terephthalate and sodium hydroxide in water and ethanol.

Examples of basic reagents to be used include sodium hydroxide, potassium hydroxide, barium hydroxide and lithium hydroxide. These reagents may be used singly or as a mixture of two or more.

Solvents to be used include hydrophilic solvents such as water, acetone, methanol, ethanol, n-propanol, isopropanol, n-butanol and THF. These solvents may be used singly or as a mixture of two or more.

The use amount of a basic reagent is usually 1.5 mole times to 100 mole times, preferably 2 mole times to 50 mole times, and more preferably 2 mole times to 30 mole times, relative to a terephthalate compound. If the use amount of a basic reagent is too small, the proportion of a compound in which only one ester group is hydrolyzed increases.

The concentration of a solution of the basic reagent is not especially limited, but is usually in the range from 0.001 mol to 20 mol, preferably from 0.01 mol to 10 mol, and more preferably from 0.1 mol to 5 mol, relative to 1 L of a solvent.

The reaction temperature in the reaction of an acidic reagent and a terephthalate compound is usually in the range from -78°C to 250°C, preferably from 0°C to 225°C, and more preferably from 25°C to 200°C. The reaction time is not especially limited, but is usually 1 min to 48 hours.

In the case of stopping the reaction, for example, water, dilute hydrochloric acid or the like is added to a reaction solution. After the stopping of the reaction, by carrying out a usual post-treatment, an operation such as extraction or washing, a crude product of a terephthalic acid compound can be obtained. For the following intramolecular acylation reaction, the crude product of the terephthalic acid compound may be supplied, or after the crude terephthalic acid compound is refined by an operation such as crystallization, sublimation, and various types of chromatographies, the refined terephthalic acid compound may be supplied for the intramolecular acylation reaction.

Then, an organic thin film device will be described. The organic thin film device according to the present invention comprises an organic thin film transistor, that is, an organic thin film transistor having an organic semiconductor layer comprising a dihydroindacene compound or an indacenedione compound. The present invention can provide an organic thin film device comprising an organic thin film transistor having a high carrier mobility.

The organic transistor according to the present invention includes an organic field-effect transistor. With respect to the structure of the organic field-effect transistor, all that is needed is usually such that, as shown in Figure 1, a source electrode 14 and a drain electrode 15 are provided contacting with an active layer (organic semiconductor layer 16), and a gate electrode 12 is further provided interposing an insulating layer (dielectric layer)(gate insulating film 13) contacting with the active layer 16 between the gate electrode 12 and the active layer 16. Examples of the element structure include the following structures (1) to (4).

(1) Substrate/gate electrode/insulator layer/source electrode · drain electrode/semiconductor layer
(2) Substrate/semiconductor layer/source electrode · drain electrode/insulator layer/gate electrode
(3) Substrate/source electrode (or drain electrode)/semiconductor layer + insulator layer + gate electrode/drain electrode (or source electrode)
(4) Substrate/gate electrode/insulator layer/semiconductor layer/source electrode · drain electrode

Here, the source electrode, the drain electrode and the gate electrode may each be provided in a plural number. A plurality of semiconductor layers may be provided in the same plane, or as a laminate.

Methods for disposing the organic semiconductor material according to the present invention as an organic semiconductor film, and as a semiconductor layer of an organic semiconductor device or an organic thin film transistor include formation methods in a vacuum process such as a vacuum vapor deposition method, a sputtering method, a CVD method and a molecular beam epitaxial growth method, and preferably include the vacuum vapor deposition method.

A method for disposing an organic semiconductor layer using the vacuum vapor deposition method is a method in which an organic semiconductor material is heated under vacuum in a crucible or a metal boat, and the evaporated organic semiconductor material is vapor deposited on a substrate or an insulator material. The degree of vacuum at vapor deposition is usually $1 \times 10^{-1}$ Pa or less, and preferably $1 \times 10^{-3}$ Pa or less. The temperature of the substrate at vapor deposition is usually 0°C to 300°C, and preferably 20°C to 200°C. The rate of vapor deposition is usually 0.001 nm/sec to 10 nm/sec, and preferably 0.01 nm/sec to 1 nm/sec. The film thickness of an organic semiconductor layer formed from the organic semiconductor material is usually 1 nm to 10 $\mu$m, and preferably 5 nm to 1 $\mu$m.

A method for disposing an organic semiconductor film may use a solution process. The solution process is a method of dissolving or dispersing an organic semiconductor material in a solvent, and coating the solution or dispersion on a substrate or an insulator layer.

Coating methods include those such as a casting method, a dip coating method, a die coater method, a roll coater method, a bar coater method and a spin coat method, an ink jet method, a screen printing method, an offset printing method and a microcontact printing method. These methods may be used singly or in combination of two or more.

In the present invention, materials forming a source electrode, a drain electrode and a gate electrode are not especially limited as long as they are conductive materials, and platinum, gold, silver, nickel, chromium, copper, iron, tin, antimonial lead, tantalum, indium, palladium, tellurium, rhenium, iridium, aluminum, ruthenium, germanium, molybdenum, tungsten, tin·antimony oxide, indium·tin oxide (ITO), fluorine-doped zinc oxide, zinc, carbon, graphite, glassy carbon, silver paste and carbon paste, lithium, beryllium, sodium, magnesium, potassium, calcium, scandium, titanium,

manganese, zirconium, gallium, niobium, a sodium-potassium alloy, a magnesium/copper mixture, a magnesium/silver mixture, a magnesium/aluminum mixture, a magnesium/indium mixture, an aluminum/aluminum oxide mixture, a lithium/aluminum mixture, and the like are used, but especially preferable are platinum, gold, silver, copper, aluminum, indium, ITO and carbon. Alternatively, well-known conductive polymers improved in electric conductivity by doping or the like, for example, conductive polyaniline, conductive polypyrrole and conductive polythiophene, a complex of a polyethylene dioxythiophene and a polystyrene sulfonic acid, and the like are suitably used. Particularly materials having a small electric resistance at the contact surface with a semiconductor layer are preferable. These electrode materials may be used singly or as a mixture of two or more. The film thickness of an electrode is, differing by the material, usually 0.1 nm to 10 μm, preferably 0.5 nm to 5 μm, and more preferably 1 nm to 3 μm. In the case of serving both as a gate electrode and a substrate, the film thickness may be larger than the above-mentioned film thickness.

Formation methods of an electrode film to be used includevarious types of methods using the materials described above as raw materials. The methods specifically include a vacuum vapor deposition method, a sputtering method, a coating method, a thermal transfer method, a printing method and a sol-gel method. At the film formation, or after the film formation, patterning is preferably carried out according to needs. Methods of patterning to be used include various types of methods. The methods specifically include a photolithography in combination of patterning and etching of a photoresist. The methods further include printing methods such as an ink jet printing, a screen printing, an offset printing and a letterpress printing, and a method of a soft lithography such as a microcontact printing method.

Patterning may be carried out using these methods singly or by mixing two or more thereof.

A gate insulating layer to be used includes various types of insulating films. Inorganic oxides include silicon oxide, aluminum oxide, tantalum oxide, titanium oxide, tin oxide, vanadium oxide, barium strontium titanate, barium zirconate titanate, lead zirconate titanate, lead lanthanum titanate, strontium titanate, barium titanate, barium magnesium fluoride, bismuth titanate, strontium bismuth titanate, strontium bismuth tantalate, bismuth niobate tantalate and yttrium trioxide, and preferable are silicon oxide, aluminum oxide, tantalum oxide, and titanium oxide. Inorganic nitrides such as silicon nitride and aluminum nitride are included. Organic compound films include polyimide, polyamide, polyester, polyacrylate, photocuring resins based on photoradical polymerization or photocationic polymerization, copolymers containing an acrylonitrile component, polyvinylphenol, polyvinyl alcohol, novolac resins and cyanoethylpullulans, and preferably include polyimide, polyvinylphenol and polyvinyl alcohol. These insulating layer materials may be used singly or in combination of two or more. The film thickness of an insulating layer is, differing by the material, usually 0.1 nm to 100 μm, preferably 0.5 nm to 50 μm, and more preferably 5 nm to 10 μm.

Formation methods of an insulating layer to be used include various types of methods using the materials described above as raw materials. The methods specifically include coating methods such as spin coating, spray coating, dip coating, casting, bar coating and blade coating, printing methods such as screen printing, offset printing and ink jet printing, and dry process methods such as a vacuum vapor deposition method, a molecular beam epitaxial growth method, an ion cluster beam method, an ion plating method, a sputtering method, an atmospheric pressure plasma method and a CVD method. The methods besides include a sol-gel method and a method of forming an oxide film on a metal such as alumite on aluminum and a thermally oxidized film of silicon.

Materials for a substrate include glass, paper, ceramics and flexible resin sheets. Resin films specifically include polyethylene terephthalate (PET), polyethylene naphthalate (PEN), polyether sulfone (PES), polyether imide, polyether ether ketone, polyphenylene sulfide, polyarylate, polyimide, polycarbonate (PC), cellulose triacetate (TAC) and cellulose acetate propionate (CAP). The thickness of a substrate is usually 1 μm to 10 mm, and preferably 5 μm to 5 mm.

In portions of an insulator layer and a substrate contacting with an organic semiconductor layer, a surface treatment may be carried out on the insulator layer and the substrate. The surface treatment on an insulator layer on which an organic semiconductor layer is to be laminated allows improvement in transistor characteristics of an element. The surface treatment specifically includes a hydrophobizing treatment with hexamethyldisilazane, octadecyltrichlorosilane, octyltrichlorosilane or the like, an acid treatment with hydrochloric acid, sulfuric acid, a hydrogen peroxide solution or the like, an alkali treatment with sodium hydroxide, potassium hydroxide, calcium hydroxide, ammonia or the like, an ozone treatment, a fluorinating treatment, a plasma treatment with oxygen, argon or the like, a treatment to form a Langmuir·Blodgett film, a treatment to form a thin film of another insulator or another semiconductor, a mechanical treatment, an electric treatment with corona discharge or the like, and a rubbing treatment utilizing fiber or the like.

Examples of methods of surface treatments include a vacuum vapor deposition method, a sputtering method, a coating method, a printing method and a sol-gel method.

A protective film composed of a resin or an inorganic compound may be disposed on an organic semiconductor layer. The formation of a protective film can suppress the influence of outside air and stabilize the driving of a transistor.

## Examples

Hereinafter, the present invention will be described further in detail by way of experimental examples, but the present invention is not limited to these Examples.

[Example 1]

<Production of dimethyl 2,5-bis(5-hexyl-2-thienyl)terephthalate (Compound 3)>

[Formula 43]

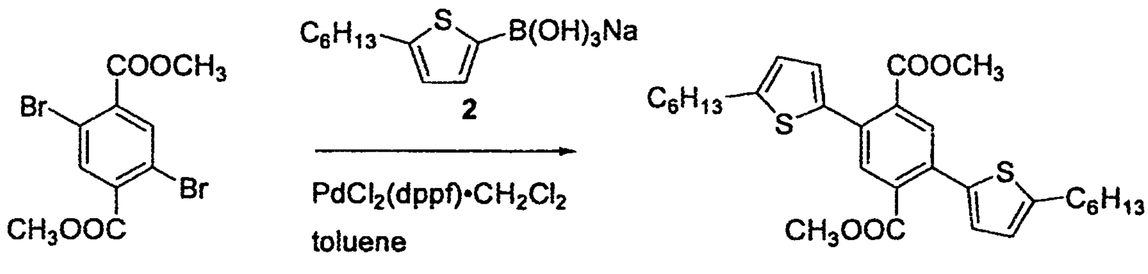


1 3

A mixed liquid of methyl p-dibromoterephthalate (Compound 1)(9.86 g, 28 mmol) synthesized according to the method of a literature (Macromolecules, 1999, 32, 2455), sodium 2-hexylthiophene-5-borate (Compound 2)(15.5 g, 61.6 mmol) synthesized according to the method of a literature (Org. Lett. 2006, 8, 4071), $PdCl_2(dppf) \cdot CH_2Cl_2$ (1.83 g, 2.24 mmol), and toluene (1L) was refluxed under a nitrogen atmosphere for 9 hours. After the reaction mixed liquid was allowed to cool to room temperature, water was added to the reaction mixed liquid and the reaction mixed liquid was subjected to extraction with chloroform. The obtained organic layer was dried with sodium sulfate, and filtered, and thereafter, the solvent was distilled out under reduced pressure. The obtained mixture was separated and refined by a silica gel column chromatography to obtain dimethyl 2,5-bis(5-hexyl-2-thienyl)terephthalate (Compound 3)(10.8 g, 20.5 mmol) in a yield of 73%.

The physical properties of dimethyl 2,5-bis(5-hexyl-2-thienyl)terephthalate (Compound 3) were as follows.
$^1H$-NMR ($CDCl_3$, $\delta$ ppm): 7.61 (s, 2H), 7.06 (d, 2H), 6.86 (d, 2H), 3.76 (s, 6H), 2.82 (t, 4H), 1.63-1.80 (m, 4H), 1.28-1.45 (m, 12H), 0.90 (t, 6H)

[Example 2]

<Production of 2,5-bis(5-hexyl-2-thienyl)terephthalic acid (Compound 4)>

[Formula 44]

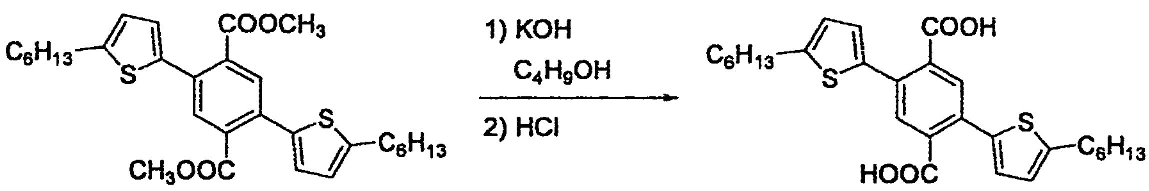


3 4

Dimethyl 2,5-bis(5-hexyl-2-thienyl)terephthalate (Compound 3)(10.0 g, 19.0 mmol), potassium hydroxide (21.3 g, 380 mmol) and butanol (0.6 L) were refluxed for 15 hours. After the reaction mixed liquid was allowed to cool to room temperature, dilute hydrochloric acid was added to the reaction mixed liquid. The produced precipitate was filtered, washed with water, and dried under reduced pressure at 60°C to obtain 2,5-bis(5-hexyl-2-thienyl)terephthalic acid (Compound 4)(8.28 g, 16.6 mmol) in a yield of 87%.

The physical properties of 2,5-bis(5-hexyl-2-thienyl)terephthalic acid (Compound 4) were as follows.
$^1H$-NMR (DMSO-$d_6$, $\delta$ ppm): 13.39 (s, 2H), 7.61 (s, 2H), 7.06 (d, 2H), 6.86 (d, 2H), 2.81 (t, 4H), 1.64-1.75 (m, 4H), 1.25-1.44 (m, 12H), 0.87 (t, 6H)

[Example 3]

<Production of 2,7-dihexylthiopheno[2',3'-6,5]s-indaceno[1,2-b]thiophene-4,9-dione (Compound 5)>

[Formula 45]

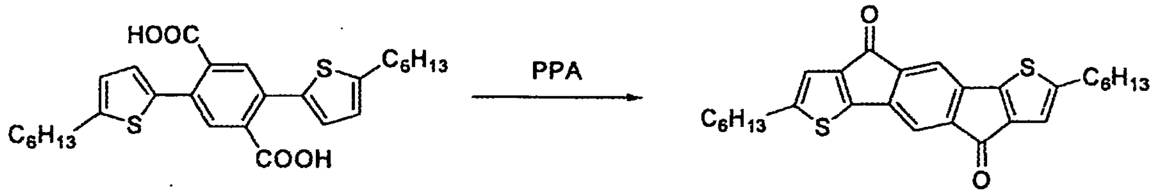


4

5

A mixture of 2,5-bis(5-hexyl-2-thienyl)terephthalic acid (Compound 4)(6.28 g, 12.6 mmol) and polyphosphoric acid (PPA)(126 mL) was stirred at 140°C for 9 hours. The resultant was cooled to 0°C; water was dropped therein; and the resultant was subjected to extraction with chloroform. The obtained organic layer was washed with a 10% sodium hydroxide aqueous solution and with water, dried with sodium sulfate, and filtered, and thereafter, the solvent was distilled out under reduced pressure. The obtained mixture was separated and refined by a silica gel column chromatography to obtain 2,7-dihexylthiopheno[2',3'-6,5]s-indaceno[1,2-b]thiophene-4,9-dione
(Compound 5)(4.48 g, 9.68 mmol) in a yield of 77%.

The physical properties of 2,7-dihexylthiopheno[2',3'-6,5]s-indaceno[1,2-b]thiophene-4,9-dione (Compound 5) were as follows.
$^{1}$H-NMR ($CDCl_3$, δ ppm): 7.80 (s, 2H), 7.12 (s, 2H), 2.78 (t, 4H), 1.62-1.73 (m, 4H), 1.26-1.41 (m, 12H), 0.90 (t, 6H)
$^{13}$C-NMR ($CDCl_3$, δ ppm): 186.0, 155.2, 151.4, 140.6, 139.7, 139.6, 126.2, 125.2, 124.5, 117.9, 113.7, 31.4, 31.1, 30.5, 30.1, 28.6, 22.5, 14.0 HRMS (APPI+): calcd for C28H31O2S2, 463.1760; found 463.1745

[Example 4]

<Production of 2,7-dihexyl-4,9-dihydrothiopheno[2',3'-6,5]s-indaceno[1,2-b]thiophene (Compound 6)>

[Formula 46]

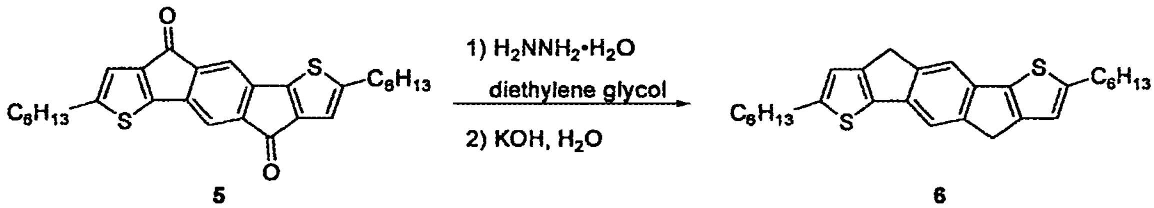


5

6

A mixture of 2,7-dihexylthiopheno[2',3'-6,5]s-indaceno[1,2-b]thiophene-4,9-dione (Compound 5)(4.18 g, 9.03 mmol), hydrazine monohydrate (4.65 mL, 95.7 mmol) and diethylene glycol (180 mL) was stirred at 80°C for 1 hour, and then at 180°C for 1 hour. The mixed liquid was heated at 160°C; and a potassium hydroxide aqueous solution (2.14 M, 43.1 mL) was dropped therein, and the mixed liquid was refluxed for 2 hours. After the reaction mixed liquid was allowed to cool to room temperature, water was added to the reaction mixed liquid, and the reaction mixed liquid was subjected to extraction with chloroform. The obtained organic layer was dried with sodium sulfate, and filtered, and thereafter, the solvent was distilled out under reduced pressure. The obtained mixture was separated and refined by a silica gel column chromatography to obtain 2,7-dihexyl-4,9-dihydrothiopheno[2',3'-6,5]s-indaceno[1,2-b]thiophene
(Compound 6)(1.54 g, 3.54 mmol) in a yield of 39%.

The physical properties of 2,7-dihexyl-4,9-dihydrothiopheno[2',3'-6,5]s-indaceno[1,2-b]thiophene (Compound 6) were as follows.

$^1H$-NMR ($CDCl_3$, δ ppm): 7.49 (s, 2H), 6.80 (s, 2H), 3.65 (s, 4H), 2.87 (t, 4H), 1.66-1.77 (m, 4H), 1.26-1.46 (m, 12H), 0.90 (t, 6H)
elemental anal: calcd for C28H31O2S2: C77.36, H7.88; found C77.29, H7.77

[Example 5]

<Production of dimethyl 2,5-bis(2-thienyl)terephthalate (Compound 8)>

[Formula 47]

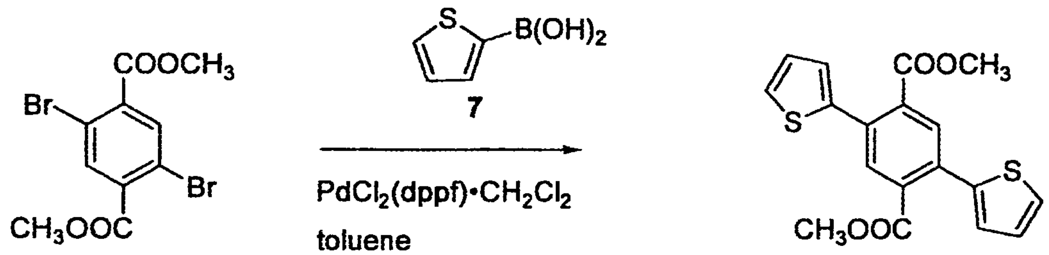


1 8

A mixed liquid of methyl p-dibromoterephthalate (Compound 1)(65.1 g, 185 mmol) synthesized according to the method of a literature (Macromolecules, 1999, 32, 2455), thiopehne-2-boric acid (Compound 7)(made by Tokyo Chemical Industry Co., Ltd.)(71.0 g, 555 mmol), $PdCl_2(dppf) \cdot CH_2Cl_2$ (15.1 g, 18.5 mmol), potassium carbonate (84.4 g, 610 mmol) and toluene (6.5 L) was refluxed under a nitrogen atmosphere for 6 hours. After the reaction mixed liquid was allowed to cool to room temperature, water was added to the reaction mixed liquid, and the reaction mixed liquid was subjected to extraction with chloroform. The obtained organic layer was dried with sodium sulfate, and filtered, and thereafter, the solvent was distilled out under reduced pressure. The obtained mixture was recrystallized using hexane and chloroform to obtain dimethyl 2,5-bis(2-thienyl)terephthalate (Compound 8)(45.9 g, 128 mmol) in a yield of 69%.

The physical properties of dimethyl 2,5-bis(2-thienyl)terephthalate (Compound 8) were as follows.
$^1H$-NMR ($CDCl_3$, δ ppm): 7.82 (s, 2H), 7.38 (dd, 2H), 7.07-7.12 (m, 4H), 3.78 (s, 6H)

[Example 6]

<Production of dimethyl 2,5-bis(5-bromo-2-thienyl)terephthalate (Compound 9)>

[Formula 48]

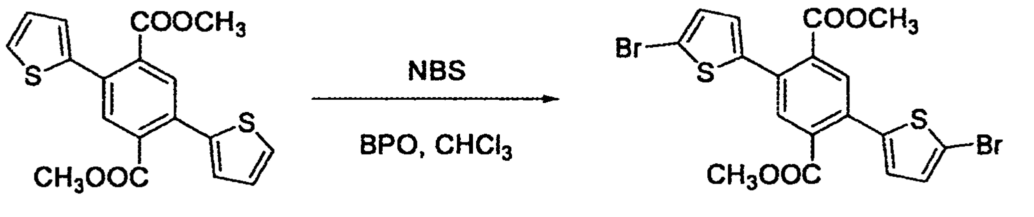


8 9

A mixture of dimethyl 2,5-bis(2-thienyl)terephthalate (Compound 8)(40.0 g, 112 mmol), benzoyl peroxide (BPO)(an amount of a catalyst), N-bromosuccinimide (NBS)(59.6 g, 335 mmol) and chloroform (8.3 L) was stirred. After 12 hours, N-bromosuccinimide (NBS)(45.0 g, 253 mmol) and BPO (an amount of a catalyst) were added to the mixture, and stirred further for 12 hours. Ethanol (2.8 L) was added thereto, and chloroform only was distilled out. Ethanol (2.8 L) was added to the mixture, and the mixture was exposed to an ultrasonic washing machine for 10 min, and thereafter filtered, and the filter cake was washed with water. The obtained solid was dried to obtain dimethyl 2,5-bis(5-bromo-2-thienyl)terephthalate (Compound 9)(32.8 g, 63.4 mmol) in a crude yield of 57%.

The physical properties of dimethyl 2,5-bis(5-bromo-2-thienyl)terephthalate (Compound 9) were as follows.
$^1$H-NMR ($CDCl_3$, δ ppm): 7.78 (s, 2H), 7.04 (d, 2H), 6.86 (d, 2H), 3.81 (s, 6H)

[Example 7]

<Production of Compound 11>

[Formula 49]

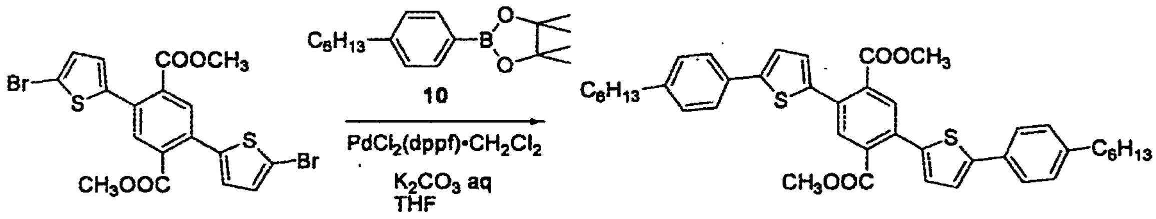


9 11

A mixed liquid of dimethyl 2,5-bis(5-bromo-2-thienyl)terephthalate (Compound 9)(20.0 g, 38.7 mmol), 2-(4-hexyl-phenyl)-4,4,5,5-tetramethyl[1,3,2]dioxaboran (Compound 10)(24.6 g, 85.2 mmol) synthesized according to the method of a literature (Tetrahedron Lett., 2006, 47, 8313), $PdCl_2$(dppf)·$CH_2Cl_2$ (6.3 g, 7.7 mmol), potassium carbonate (813.4 g, 96.9 mmol), water (400 mL) and THF (2.0 L) was refluxed under a nitrogen atmosphere for 9 hours. After the reaction mixed liquid was allowed to cool to room temperature, water was added to the reaction mixed liquid, and the reaction mixed liquid was subjected to extraction with chloroform. The obtained organic layer was dried with magnesium sulfate, and filtered, and thereafter, the solvent was distilled out under reduced pressure. The obtained mixture was separated and refined by a silica gel column chromatography (chloroform : hexane = 2 : 1) to obtain Compound 11 (11.6 g, 17.1 mmol) in a yield of 44%.

The physical properties of Compound 11 were as follows.
$^1$H-NMR ($CDCl_3$, δ ppm): 7.85 (s, 2H), 7.54 (d, 4H), 7.19-7.29 (m, 6H), 7.01 (d, 2H), 3.82 (s, 6H), 2.63 (t, 4H), 1.56-1.65 (m, 4H), 1.24-1.33 (m, 12H), 0.89 (t, 6H)

[Example 8]

<Production of Compound 12>

[Formula 50]

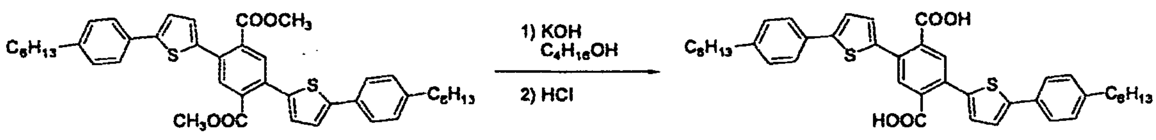


11 12

Compound 11 (10.0 g, 14.7 mmol), potassium hydroxide (1.82 g, 32.4 mmol) and butanol (0.25 L) were refluxed for 8 hours. After the mixed liquid was allowed to cool to room temperature, water (0.8 L) was added thereto, and butanol only was distilled out. Hydrochloric acid was dropped in the obtained mixture until precipitate came not to be produced, and the mixture was filtered. The filter cake was washed with water, and dried under reduced pressure at 80°C for 2 hours to obtain Compound 12 (8.56 g, 13.2 mmol) in a yield of 77%.

The physical properties of Compound 12 were as follows.
$^1$H-NMR (DMSO-$d_6$, δ ppm): 7.76 (s, 2H), 7.59 (d, 4H), 7.49 (d, 2H), 7.24-7.27 (m, 6H), 2.59 (t, 4H), 1.55-1.60 (m, 4H), 1.27-1.28 (m, 12H), 0.85 (t, 6H)

[Example 9]

<Production of Compound 13>

[Formula 51]

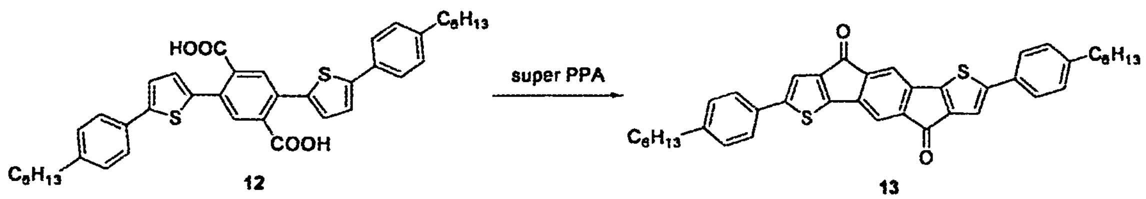


12 13

Compound 12 (1.70 g, 2.61 mmol) was added to a super polyphosphoric acid (super PPA)(PPA: 52.2 mL, diphosphorus pentoxide: 18.1 g) prepared according to the method of a literature (J. Am. Chem. Soc., 2001, 123, 4763), and stirred at 150°C for 42 hours. Ice was charged to the mixture cooled to 0°C until heat generation came to vanish. Chloroform was further poured therein, and the mixture was celite-filtered, and thereafter, the obtained filtrate was washed with a 10% sodium hydroxide aqueous solution and with water, dried with sodium sulfate, and filtered. The solvent in the filtrate was distilled out under reduced pressure to obtain Compound 13 (1.03 g, 1.67 mmol) in a yield of 64%.

The physical properties of Compound 13 were as follows.
HRMS (EI+): calcd for C40H38O2S2, 614.23969; found 614.23132

[Example 10]

<Production of Compound 14>

[Formula 52]

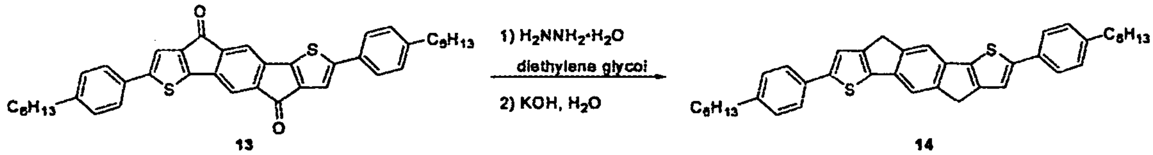


13 14

A mixture of Compound 13 (1.19 g, 1.83 mmol), hydrazine monohydrate (0.86 mL, 17.8 mmol) and diethylene glycol (32 mL) was stirred at 80°C for 1 hour, and then at 180°C for 4 hours. The mixed liquid was allowed to cool to room temperature; and a potassium hydroxide aqueous solution (4.3 M, 4 mL) was dropped thereto, and refluxed for 27 hours. After the reaction mixed liquid was allowed to cool to room temperature, water was added to the reaction mixed liquid, and the reaction mixed liquid was filtered, and the filter cake was washed with water. The filter cake was dissolved in chloroform, and water was added to the solution to subject the solution to extraction with water. The obtained organic layer was dried with magnesium sulfate, and after the filtration, the solvent was distilled out under reduced pressure to obtain Compound 14 (0.23 g, 0.39 mmol) in a yield of 24%.

The physical properties of Compound 14 were as follows.
HRMS (APPI+): calcd for C40H43S2 [M+H], 587.2791; found 587.2800

[Example 11]

<Production of Compound 15>

[Formula 53]

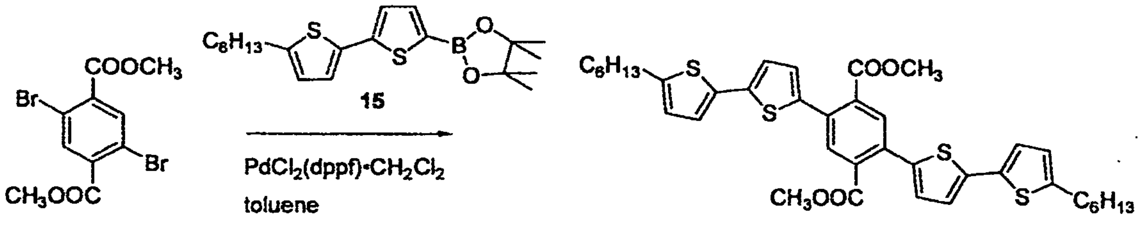


1 16

A mixed liquid of methyl p-dibromoterephthalate (Compound 1)(10.6 g, 30.0 mmol) synthesized according to the method of a literature (Macromolecules, 1999, 32, 2455), Compound 15 (made by Sigma-Aldrich Corp.)(24.8 g, 66.0 mmol), PdCh(dppf)$CH_2Cl_2$ (2.45 g, 3.00 mmol), potassium carbonate (13.7 g, 13.7 mmol), water (60 mL) and THF (1.2 L) was refluxed under a nitrogen atmosphere for 16 hours. After the reaction mixed liquid was allowed to cool to room temperature, water was added to the reaction mixed liquid, and the reaction mixed liquid was subjected to extraction with chloroform. The obtained organic layer was dried with sodium sulfate, and filtered, and thereafter, the solvent was distilled out under reduced pressure. The obtained mixture was separated and refined by a silica gel column chromatography to obtain Compound 16 (7.89 g, 15.0 mmol) in a yield of 50%.

The physical properties of Compound 16 were as follows.

$^1$H-NMR ($CDCl_3$, δ ppm): 7.81 (s, 2H), 7.06 (d, 2H), 6.97-7.04 (m, 4H), 6.70 (d, 2H), 3.64 (s, 6H), 2.80 (t, 4H), 1.56-1.63 (m, 4H), 1.32-1.41 (m, 12H), 0.90 (t, 6H)

[Example 12]

<Production of Compound 17>

[Formula 54]

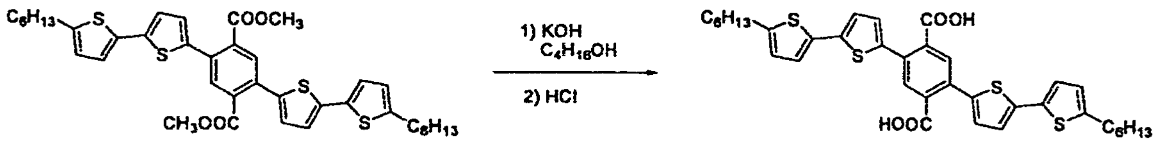


16 17

Compound 16 (15.8 g, 22.9 mmol), potassium hydroxide (2.83 g, 50.4 mmol) and butanol (0.40 L) were refluxed for 5 hours. After the mixed liquid was allowed to cool to room temperature, hydrochloric acid was dropped therein until precipitate came not to be produced, and the resultant was filtered. The filter cake was washed with water, and dried under reduced pressure at 80°C for 2 hours to obtain Compound 17 (20.0 g, 13.3 mmol) in a yield of 90%.

The physical properties of Compound 17 were as follows.

$^1$H-NMR (DMSO-$d_6$, δ ppm): 7.76 (s, 2H), 7.14-7.26 (m, 6H), 6.83 (d, 2H), 2.80 (t, 4H), 1.60-1.66 (m, 4H), 1.23-1.34 (m, 12H), 0.82 (t, 6H)

[Example 13]

<Production of Compound 18>

[Formula 55]

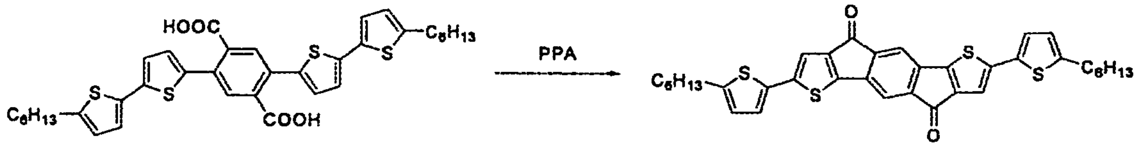


17 18

A mixture of Compound 17 (9.94 g, 15.0 mmol) and a polyphosphoric acid (PPA)(150 mL) was stirred at 180°C for 16 hours. The resultant was cooled to 0°C, and water was dropped therein, and the resultant was subjected to extraction with chloroform. The obtained organic layer was washed with a 10% potassium hydroxide aqueous solution and with water, and dried with sodium sulfate. The sodium sulfate was filtered, and thereafter, the solvent was distilled out under reduced pressure to obtain Compound 18 (5.52 g, 8.85 mmol) in a yield of 59%.

The physical properties of Compound 18 were as follows.

$^1$H-NMR ($CDCl_3$, $\delta$ ppm): 7.16 (s, 2H), 7.07 (s, 2H), 7.02 (d, 2H), 6.70 (d, 2H), 2.80 (t, 4H), 1.63-1.71 (m, 4H), 1.31-1.38 (m, 12H), 0.90 (t, 6H)

[Example 14]

<Production of Compound 19>

[Formula 56]

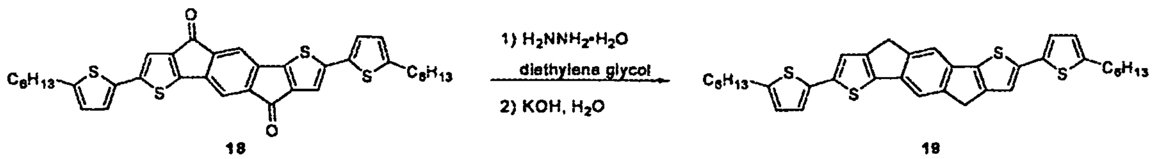


18 19

A mixture of Compound 18 (0.30 g, 0.48 mmol), hydrazine monohydrate (0.25 mL, 5.09 mmol) and diethylene glycol (10 mL) was stirred at 80°C for 1 hour, and then at 180°C for 3 hours. After the reaction mixed liquid was allowed to cool to room temperature, water was added to the reaction mixed liquid, and the reaction mixed liquid was subjected to extraction with chloroform. The obtained organic layer was dried with sodium sulfate, and filtered, and thereafter, the solvent was distilled out under reduced pressure to obtain a red solid (130 mg). Diethylene glycol (10 mL) and a potassium hydroxide aqueous solution (0.81 M, 2.5 mL) were added to the red solid, and refluxed for 5 hours. After the reaction mixed liquid was allowed to cool to room temperature, water was added to the reaction mixed liquid, and filtered; and the filter cake was washed with water, and dried under vacuum. The obtained solid was separated and refined by a silica gel column chromatography to obtain Compound 19 (0.1 g, 0.17 mmol) in a yield of 35%.

The physical properties of Compound 19 were as follows.

$^1$H-NNM ($CDCl_3$, $\delta$ ppm): 7.53 (s, 2H), 7.12 (s, 2H), 7.01 (d, 2H), 6.69 (d, 2H), 3.75 (s, 4H), 2.80 (t, 4H), 1.64-1.75 (m, 4H), 1.26-1.42 (m, 12H), 0.90 (t, 6H)

[Example 15]

<Production of an organic transistor having a vacuum vapor deposited film of 2,7-dihexyl-4,9-dihydrothiopheno[2',3'-6,5]s-indaceno[1,2-b]thiophene (Compound 6) as an organic semiconductor layer>

5 nm of titanium and further 25 nm of gold were vapor deposited on a n-doped silicon wafer with a 50-nm $SiO_2$ thermally oxidized film subjected to an octadecyltrichlorosilane treatment, using a metal mask. Then, the synthesized 2,7-dihexyl-4,9-dihydrothiopheno[2',3'-6,5]s-indaceno[1,2-b]thiophene (Compound 6) was vacuum vapor deposited thereon to form an organic semiconductor layer composed of 2,7-dihexyl-4,9-dihydrothiopheno [2',3'-6,5]s-indaceno[1,2-b]thiophene (Compound 6). Here, the organic semiconductor layer composed of 2,7-dihexyl-4,9-dihydrothiopheno[2',3'-6,5]s-indaceno[1,2-b]thiophene (Compound 6) was formed under the following condition.

The degree of vacuum in an apparatus chamber used in the vacuum vapor deposition method was $1 \times 10^{-4}$ Pa or lower. The temperature of a substrate was in the range of room temperature (24°C) or higher and 80°C or lower. 2,7-Dihexyl-4,9-dihydrothiopheno[2',3'-6,5]s-indaceno[1,2-b]thiophene (Compound 6) refined by sublimation was placed in a tungsten boat, and terminals of the boat were heated. The film thickness of the organic semiconductor layer was about 40 nm.

Lastly, a gold layer of 30 nm in thickness was formed on the organic semiconductor layer by the vacuum vapor deposition method using a metal mask to form a source electrode and a drain electrode. Here, the channel width and the channel length of an organic transistor obtained by the formation of the source electrode and the drain electrode were 500 μm or more and 1,000 μm or less, and 50 μm, respectively.

An organic transistor having a vacuum vapor deposited film of 2,7-dihexyl-4,9-dihydrothiopheno[2',3'-6,5]s-indaceno[1,2-b]thiophene (Compound 6) as an organic semiconductor layer, as shown in Figure 1, was thus produced.

[Example 16]

<Measurement with respect to the organic transistor having a vacuum vapor deposited film of 2,7-dihexyl-4,9-dihydrothiopheno[2',3'-6,5]s-indaceno[1,2-b]thiophene (Compound 6) as an organic semiconductor layer>

An electric characteristic of the produced organic transistor having an organic semiconductor layer composed of 2,7-dihexyl-4,9-dihydrothiopheno[2',3'-6,5]s-indaceno[1,2-b]thiophene (Compound 6) was measured. The result of the measurement is shown in Figure 2. As shown in Figure 2, variation curves of the drain currents (Id) versus the drain voltages (Vd) at certain gate voltages (Vg) were good, and exhibited linear regions (voltage-proportional regions) at low drain voltages and saturated regions at high drain voltages. From the fact that if a negative gate voltage applied to the gate electrode was increased, a negative drain current also increased, the produced organic transistor having an organic semiconductor layer composed of 2,7-dihexyl-4,9-dihydrothiopheno[2',3'-6,5]s-indaceno[1,2-b]thiophene (Compound 6) was confirmed to be a p-type organic transistor. The field-effect mobility μ of a carrier of an organic transistor can be calculated using the following equation (a) representing the drain current Id in the saturated region of the electric characteristic of the organic transistor.
Expression

$$Id = (W / 2L)\, \mu\, Ci\, (Vg - Vt)^2 \cdots (a)$$

In the equation (a), L and W represent a gate length and a gate width of an organic transistor, respectively; Ci represents a capacity per unit area of a gate insulating film; Vg represents a gate voltage; and Vt represents a threshold voltage of the gate voltage. As a result of the calculation, using the equation (a), of the field-effect mobility μ of the carrier of the produced organic transistor having an organic semiconductor layer composed of 2,7-dihexyl-4,9-dihydrothiopheno[2',3'-6,5]s-indaceno[1,2-b]thiophene (Compound 6), the field-effect mobility and the on/off ratio of the carrier of the organic transistor having an organic semiconductor layer composed of 2,7-dihexyl-4,9-dihydrothiopheno[2',3'-6,5]s-indaceno[1,2-b]thiophene (Compound 6), produced at a temperature of the substrate of 80°C, were 0.078 $cm^2/Vs$ and $10^8$.

[Example 17]

<Production of an organic transistor having a spin coated film of 2,7-dihexyl-4,9-dihydrothiopheno[2',3'-6,5]s-indaceno[1,2-b]thiophene (Compound 6) as an organic semiconductor layer>

A 0.5-wt% chloroform solution of the synthesized 2,7-dihexyl-4,9-dihydrothiopheno[2',3'-6,5]s-indaceno[1,2-b]thiophene (Compound 6) was spin coated on a n-doped silicon wafer with a $SiO_2$ thermally oxidized film subjected to an octadecyltrichlorosilane treatment by the spin coating method to form a thin film composed of Compound 6. The formed thin film was further kept at a temperature of 80°C for 30 min.

Gold layers were formed as films on the obtained thin film by the vacuum vapor deposition method using a metal mask to form a source electrode and a drain electrode. Here, the channel width and the channel length of an organic TFT obtained by forming the source electrode and the drain electrode were 2 mm and 20 μm, respectively.

An organic transistor having a spin coated film of 2,7-dihexyl-4,9-dihydrothiopheno[2',3'-6,5]s-indaceno[1,2-b]thiophene (Compound 6) as an organic semiconductor layer was thus produced.

[Example 18]

<Measurement with respect to the organic transistor having a spin coated film of 2,7-dihexyl-4,9-dihydrothiopheno[2',3'-6,5]s-indaceno[1,2-b]thiophene (Compound 6) as an organic semiconductor layer>

As a result of measuring the electric characteristic of the organic transistor produced in Example 17, as in Example 16, the field-effect mobility and the on/off ratio of the carrier were 0.007 $cm^2/Vs$ and $10^6$.

Heretofore, embodiments and Examples according to the present invention have been described specifically, but the present invention is not limited to these embodiments and Examples, and embodiments and Examples according to the present invention may be modified or changed without departing from the gist and the scope of the present invention.

## Industrial Applicability

The present invention can be applied to an organic thin film transistor having a higher carrier mobility, a method for producing the organic thin film transistor, and an organic thin film device containing the organic thin film transistor.

## Claims

**1.** A dihydroindacene compound represented by the following formula (3):

[Formula 3]

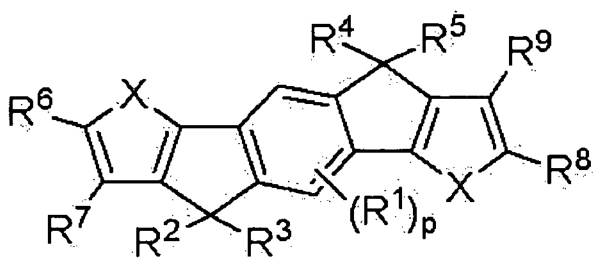

(3)

wherein $R^1$ is a hydrogen atom, alkyl that has 1 to 30 carbon atoms and may be substituted, alkenyl that has 2 to 30 carbon atoms and may be substituted, alkynyl that has 2 to 30 carbon atoms and may be substituted, or alkoxy that has 1 to 30 carbon atoms and may be substituted;
p is 0, 1, or 2;
$R^2$ to $R^5$ are identical or different from each other, and are each a hydrogen atom, or alkyl that has 1 to 30 carbon atoms and may be substituted;
$R^6$ to $R^9$ are identical or different from each other, and are each a hydrogen atom, alkyl that has 1 to 30 carbon atoms and may be substituted, alkenyl that has 2 to 30 carbon atoms and may be substituted, alkynyl that has 2 to 30 carbon atoms and may be substituted, alkoxy that has 1 to 30 carbon atoms and may be substituted, aryl that has 6 to 30 carbon atoms and may be substituted, silyl that may be substituted, a heteroaryl that may be substituted, or a halogen; and
X is identical or different from each other, and is each sulfur, oxygen, selenium, or $SO_2$,
provided that a compound represented by the following formula (2) is excluded:

[Formula 2]

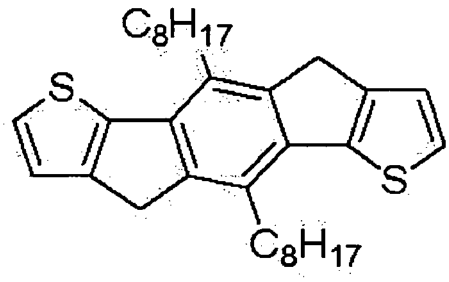

(2)

2. The dihydroindacene compound according to claim 1, wherein $R^6$ and $R^8$ are identical or different from each other, and are each a hydrogen atom, alkyl that has 1 to 30 carbon atoms and may be substituted, aryl that has 6 to 30 carbon atoms and may be substituted, heteroaryl that may be substituted, or a halogen, and X is a sulfur atom.

3. The dihydroindacene compound according to claim 1, wherein X is a sulfur atom; $R^1$ to $R^5$, $R^7$ and $R^9$ each denote a hydrogen atom; p = 2; and $R^6$ and $R^8$ are each an alkyl group having 1 to 20 carbon atoms, or an alkoxy group having 1 to 20 carbon atoms.

4. The dihydroindacene compound according to claim 1, wherein X is a sulfur atom; $R^1$ to $R^5$, $R^7$ and $R^9$ each denote a hydrogen atom; p = 2; and $R^6$ and $R^8$ are each 5-(C1-20 alkyl)thiophen-2-yl.

5. The dihydroindacene compound according to claim 1, wherein X is a sulfur atom; $R^1$ to $R^5$, $R^7$ and $R^9$ each denote a hydrogen atom; p = 2; and $R^6$ and $R^8$ are each 4-(C1-20 alkyl)phenyl-1-yl, or 4-(C1-20 alkoxy)phenyl-1-yl.

6. An organic semiconductor device using the compound according to any one of claims 1 to 5.

7. A conductive thin film comprising the compound according to any one of claims 1 to 5.

8. A light emitting thin film comprising the compound according to any one of claims 1 to 5.

9. An organic semiconductor thin film comprising the compound according to any one of claims 1 to 5.

10. An organic transistor comprising the organic semiconductor thin film according to claim 9.

11. A light emitting element comprising the light emitting thin film according to claim 8.

## Patentansprüche

1. Dihydroindacen-Verbindung, die durch folgende Formel (3) dargestellt wird:

[Formel 3]

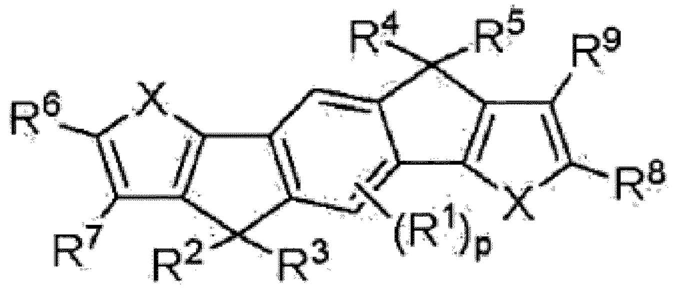

(3)

wobei $R^1$ eines von Folgendem ist: ein Wasserstoffatom, ein Alkyl, das 1 bis 30 Kohlenstoffatome aufweist und substituiert sein kann, ein Alkenyl, das 2 bis 30 Kohlenstoffatome aufweist und substituiert sein kann, ein Alkynyl, das 2 bis 30 Kohlenstoffatome aufweist und substituiert sein kann, oder ein Alkoxy, das 1 bis 30 Kohlenstoffatome aufweist und substituiert sein kann,
wobei p 0, 1 oder 2 ist;
$R^2$ bis $R^5$ gleich oder voneinander verschieden sind, und jeweils ein Wasserstoffatom sind, oder ein Alkyl, das 1 bis 30 Kohlenstoffatome aufweist und substituiert sein kann,
$R^6$ bis $R^9$ gleich oder voneinander verschieden sind, und jeweils ein Wasserstoffatom sind, ein Alkyl, das 1 bis 30 Kohlenstoffatome aufweist und substituiert sein kann, ein Alkenyl, das 2 bis 30 Kohlenstoffatome aufweist und substituiert sein kann, ein Alkynyl, das 2 bis 30 Kohlenstoffatome aufweist und substituiert sein kann, ein Alkoxy, das 1 bis 30 Kohlenstoffatome aufweist und substituiert sein kann, ein Aryl, das 6 bis 30 Kohlenstoffatome aufweist und substituiert sein kann, Silyl, das substituiert sein kann, ein Heteroaryl, das substituiert sein kann, oder ein Halogen; und
X gleich oder verschieden voneinander sind, und jeweils Schwefel, Sauerstoff, Selen oder $SO_2$ ist,
vorausgesetzt, dass eine Verbindung, die durch folgende Formel (2) dargestellt wird, ausgeschlossen ist:

[Formel 2]

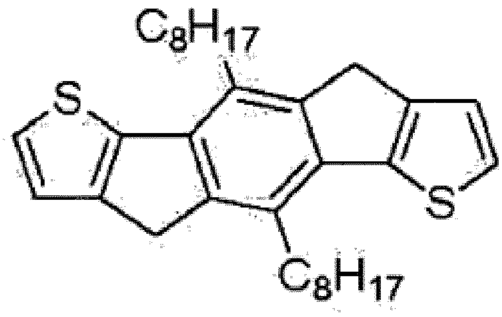

(2)

2. Dihydroindacen-Verbindung nach Anspruch 1,
wobei $R^6$ und $R^8$ gleich oder voneinander verschieden sind und jeweils Folgendes sind: ein Wasserstoffatom, ein Alkyl, das 1 bis 30 Kohlenstoffatome aufweist und substituiert sein kann, ein Aryl, das 6 bis 30 Kohlenstoffatome aufweist und substituiert sein kann, Heteroaryl, das substituiert sein kann, oder ein Halogen, und wobei X ein Schwefelatom ist.

3. Dihydroindacen-Verbindung nach Anspruch 1,
wobei X ein Schwefelatom ist, $R^1$ bis $R^5$, $R^7$ und $R^9$ jeweils ein Wasserstoffatom bezeichnen; p = 2; und $R^6$ und $R^8$ jeweils eine Alkylgruppe mit 1 bis 20 Kohlenstoffatomen oder eine Alkoxygruppe mit 1 bis 20 Kohlenstoffatomen sind.

4. Dihydroindacen-Verbindung nach Anspruch 1,
wobei X ein Schwefelatom ist, $R^1$ bis $R^5$, $R^7$ und $R^9$ jeweils ein Wasserstoffatom bezeichnen; p = 2; und $R^6$ und $R^8$ jeweils 5-(C1-20-alkyl)thiophen-2-yl sind.

5. Dihydroindacen-Verbindung nach Anspruch 1, wobei X ein Schwefelatom ist, $R^1$ bis $R^5$, $R^7$ und $R^9$ jeweils ein Wasserstoffatom bezeichnen; p = 2; und $R^6$ und $R^8$ jeweils 4-(C1-20-alkyl)phenyl-1-yl oder 4-(C1-20-alkoxy)phenyl-1-yl sind.

6. Organische Halbleitervorrichtung, die die Verbindung nach einem der Ansprüche 1 bis 5 verwendet.

7. Leitfähiger Dünnfilm, der die Verbindung nach einem der Ansprüche 1 bis 5 umfasst.

8. Lichtemittierender Dünnfilm, der die Verbindung nach einem der Ansprüche 1 bis 5 umfasst.

9. Organischer Halbleiterdünnfilm, der die Verbindung nach einem der Ansprüche 1 bis 5 umfasst.

10. Organischer Transistor, der den organischen Halbleiterdünnfilm nach Anspruch 9 umfasst.

11. Lichtemittierendes Element, das den lichtemittierenden Dünnfilm nach Anspruch 8 umfasst.

**Revendications**

1. Composé de dihydroindacène représenté par la formule suivante (3) :

[Formule 3]

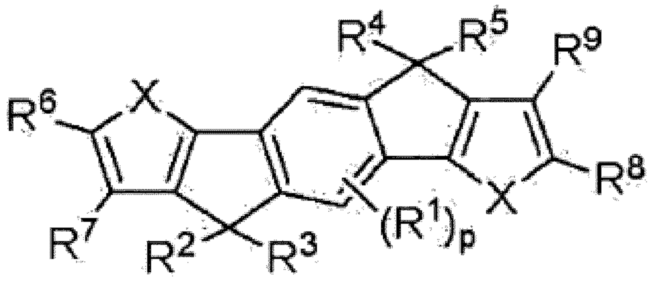

(3)

dans laquelle $R^1$ est un atome d'hydrogène, un groupe alkyle qui comporte 1 à 30 atomes de carbone et peut être substitué, un groupe alcényle qui comporte 2 à 30 atomes de carbone et peut être substitué, un groupe alkynyle qui comporte 2 à 30 atomes de carbone et peut être substitué, ou un groupe alcoxy qui comporte 1 à 30 atomes de carbone et peut être substitué ;
p vaut 0, 1, ou 2 ;
les groupes $R^2$ à $R^5$ sont identiques ou différents les uns des autres, et sont chacun un atome d'hydrogène, ou un groupe alkyle qui comporte 1 à 30 atomes de carbone et peut être substitué ;
les groupes $R^6$ à $R^9$ sont identiques ou différents les uns des autres, et sont chacun un atome d'hydrogène, un groupe alkyle qui comporte 1 à 30 atomes de carbone et peut être substitué, un groupe alcényle qui comporte 2 à 30 atomes de carbone et peut être substitué, un groupe alkynyle qui comporte 2 à 30 atomes de carbone et peut être substitué, un groupe alcoxy qui comporte 1 à 30 atomes de carbone et peut être substitué, un groupe aryle qui comporte 6 à 30 atomes de carbone et peut être substitué, un groupe silyle qui peut être substitué, un groupe hétéroaryle qui peut être substitué, ou un atome d'halogène ; et
les groupes X sont identiques ou différents les uns des autres, et sont chacun un atome de soufre, un atome d'oxygène, un atome de sélénium, ou un groupe $SO_2$,
sous réserve qu'un composé représenté par la formule suivante (2) soit exclu :

[Formule 2]

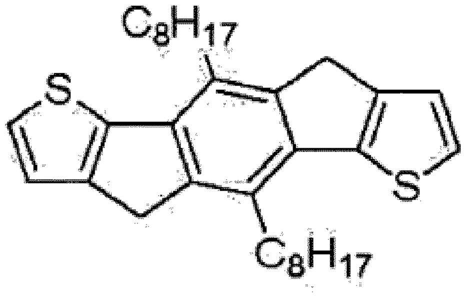

(2)

2. Composé de dihydroindacène selon la revendication 1, dans lequel les groupes $R^6$ et $R^8$ sont identiques ou différents les uns des autres, et sont chacun un atome d'hydrogène, un groupe alkyle qui comporte 1 à 30 atomes de carbone et peut être substitué, un groupe aryle qui comporte 6 à 30 atomes de carbone et peut être substitué, un groupe hétéroaryle qui peut être substitué, ou un atome d'halogène, et X est un atome de soufre.

3. Composé de dihydroindacène selon la revendication 1, dans lequel X est un atome de soufre ; les groupes $R^1$ à $R^5$, $R^7$ et $R^9$ représentent chacun un atome d'hydrogène ; p vaut 2 ; et $R^6$ et $R^8$ sont chacun un groupe alkyle qui comporte 1 à 20 atomes de carbone, ou un groupe alcoxy qui comporte 1 à 20 atomes de carbone.

4. Composé de dihydroindacène selon la revendication 1, dans lequel X est un atome de soufre ; les groupes $R^1$ à $R^5$, $R^7$ et $R^9$ représentent chacun un atome d'hydrogène ; p vaut 2 ; et $R^6$ et $R^8$ sont chacun un groupe 5-(alkyle C1-20)thiophén-2-yl.

5. Composé de dihydroindacène selon la revendication 1, dans lequel X est un atome de soufre ; les groupes $R^1$ à $R^5$, $R^7$ et $R^9$ représentent chacun un atome d'hydrogène ; p vaut 2 ; et $R^6$ et $R^8$ sont chacun un groupe 4-(alkyle C1-20)phényl-1-yl, ou 4-(alcoxy C1-C20)phényl-1-yl.

6. Dispositif semi-conducteur organique utilisant le composé selon l'une quelconque des revendications 1 à 5.

7. Film mince conducteur comprenant le composé selon l'une quelconque des revendications 1 à 5.

8. Film mince émetteur de lumière comprenant le composé selon l'une quelconque des revendications 1 à 5.

9. Film mince semi-conducteur organique comprenant le composé selon l'une quelconque des revendications 1 à 5.

10. Transistor organique comprenant le film mince semi-conducteur organique selon la revendication 9.

11. Élément émetteur de lumière comprenant le film mince émetteur de lumière selon la revendication 8.

Fig. 1

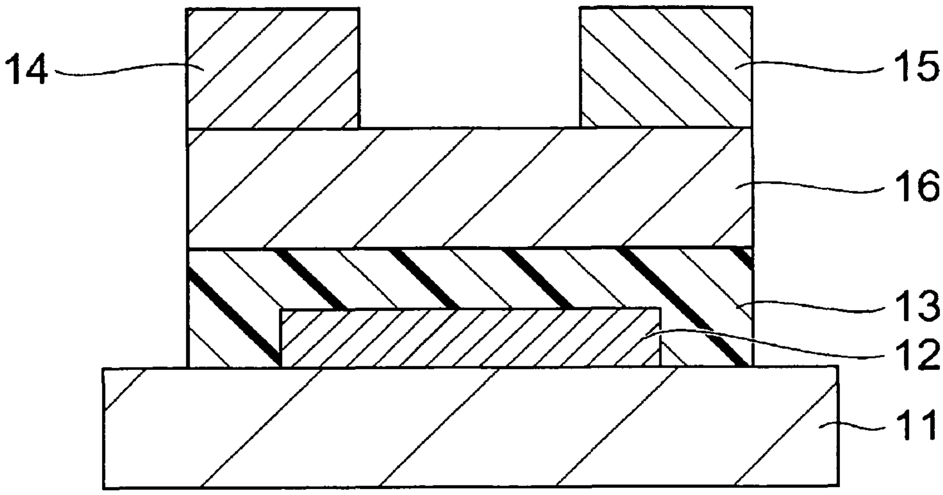

Fig. 2

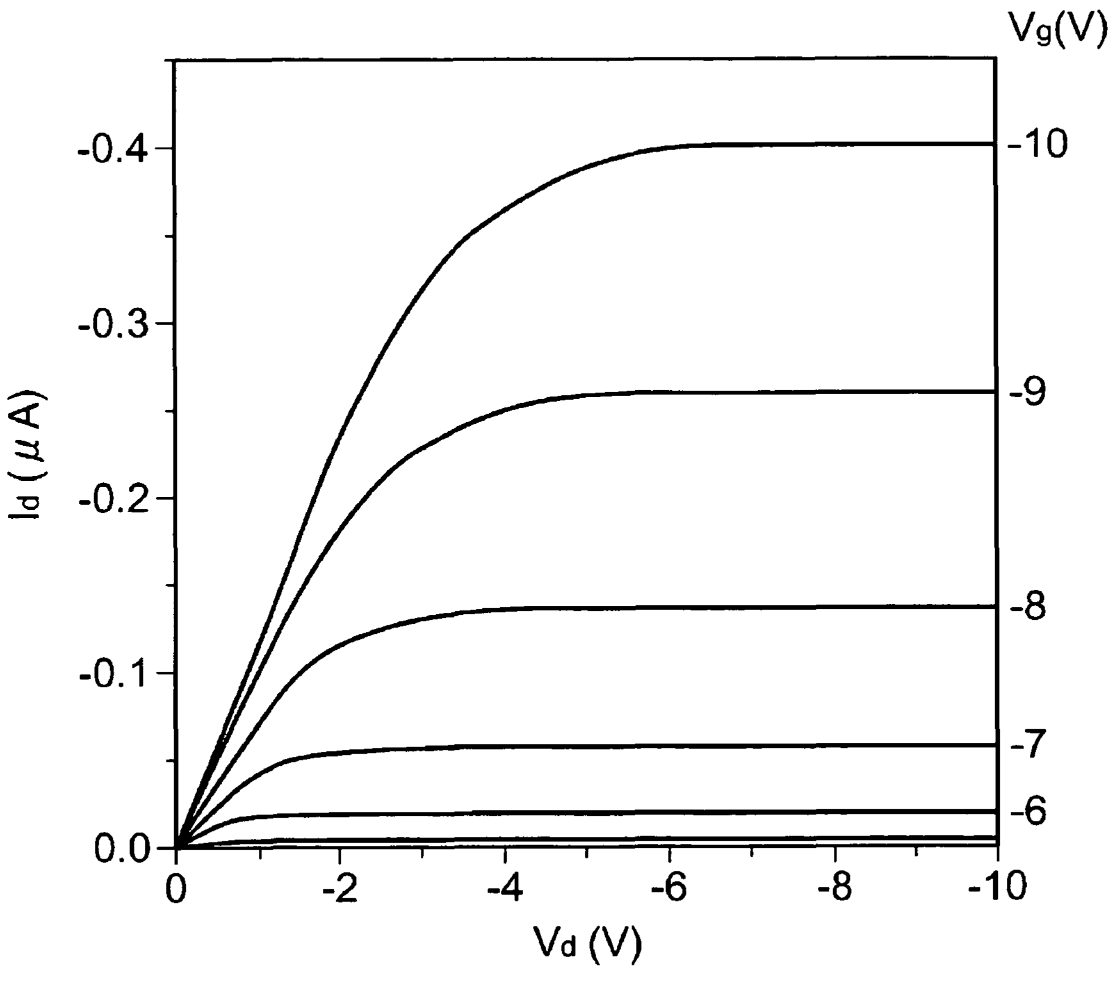

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Journal of Applied Physics,* 2002, vol. 92, 5259-5263
- *Journal of the American Chemical Society,* 2004, vol. 126, 13859-13874
- *Science,* 1998, vol. 280, 1741-1744
- *Journal of the American Chemical Society,* 2007, vol. 129, 2224-2225
- *Journal of the American Chemical Society,* 2005, vol. 127, 614-618
- *Organic Letters,* 2005, vol. 7 (5), 795-797
- *Journal of the American Chemical Society,* 2005, vol. 127, 11763-11768
- *Organic Letters,* 2001, vol. 3 (23), 3647-3650
- *Organic Letters,* 2002, vol. 4 (13), 2157-2159
- *Journal of the Organic Chemistry,* 2007, vol. 72 (17), 6364-6371
- *Macromolecules,* 1999, vol. 32, 2455
- *Org. Lett.,* 2006, vol. 8, 4071
- *Tetrahedron Lett.,* 2006, vol. 47, 8313
- *J. Am. Chem. Soc.,* 2001, vol. 123, 4763